(19) Europäisches Patentamt
European Patent Office
Office européen des brevets

(11) EP 0 949 243 A1

(12) **EUROPEAN PATENT APPLICATION**
published in accordance with Art. 158(3) EPC

(43) Date of publication:
**13.10.1999 Bulletin 1999/41**

(21) Application number: **97913440.0**

(22) Date of filing: **25.11.1997**

(51) Int. Cl.$^6$: **C07C 251/50**, C07C 249/04, C07C 249/08, C07C 239/20, C07C 259/04, C07C 271/06, C07C 309/63, C07C 33/22, C07C 29/136, C07C 45/42, C07C 49/78, C07B 53/00, C07B 57/00, C07D 333/28, C07D 317/18, C07D 319/06, C07D 317/54, C07D 319/18, A01N 35/10

(86) International application number:
**PCT/JP97/04291**

(87) International publication number:
**WO 98/23582 (04.06.1998 Gazette 1998/22)**

(84) Designated Contracting States:
**CH DE LI**

(30) Priority: **26.11.1996 JP 31527096**
**29.11.1996 JP 31986196**
**14.07.1997 JP 18815797**
**23.07.1997 JP 19721497**
**01.08.1997 JP 20770797**
**13.08.1997 JP 21854297**
**07.10.1997 JP 27428197**

(71) Applicant:
**MITSUBISHI CHEMICAL CORPORATION**
**Chiyoda-ku, Tokyo 100-0005 (JP)**

(72) Inventors:
- **ODA, Masatsugu, Mitsubishi Chemical Corporation Yokohama-shi, Kanagawa 227 (JP)**
- **KATSURADA, Manabu, Mitsubishi Chemical Corporation Yokohama-shi, Kanagawa 227 (JP)**
- **SHIGA, Yasushi, Mitsubishi Chemical Corporation Yokohama-shi, Kanagawa 227 (JP)**
- **OHNO, Fumihiko, Mitsubishi Chemical Corporation Yokohama-shi, Kanagawa 227 (JP)**
- **TANAKA, Ken, Mitsubishi Chemical Corporation Yokohama-shi, Kanagawa 227 (JP)**
- **TOMITA, Hirofumi, Mitsubishi Chemical Corporation Yokohama-shi, Kanagawa 227 (JP)**
- **FUKUCHI, Toshiki, Mitsubishi Chemical Corporation Yokohama-shi, Kanagawa 227 (JP)**
- **SUZUKI, Shigeru, Mitsubishi Chemical Corporation Yokohama-shi, Kanagawa 227 (JP)**
- **OKANO, Kazuya, Mitsubishi Chemical Corporation Inashiki-gun, Ibaraki 300-03 (JP)**
- **SHIRASAKI, Miwa, Mitsubishi Chemical Corporation Inashiki-gun, Ibaraki 300-03 (JP)**
- **TAKEHARA, Jun, Mitsubishi Chemical Corporation Inashiki-gun, Ibaraki 300-03 (JP)**
- **IWANE, Hiroshi, Mitsubishi Chemical Corporation Inashiki-gun, Ibaraki 300-03 (JP)**

(74) Representative:
**VOSSIUS & PARTNER**
**Postfach 86 07 67**
**81634 München (DE)**

EP 0 949 243 A1

(54) **OPTICALLY ACTIVE METHOXYIMINOACETAMIDE DERIVATIVES, PROCESS FOR THE PREPARATION OF THEM, INTERMEDIATES THEREFOR, AND PESTICIDES CONTAINING THEM AS THE ACTIVE INGREDIENT**

(57) An optically active methoxyiminoacetamide derivative represented by the following general formula (I)

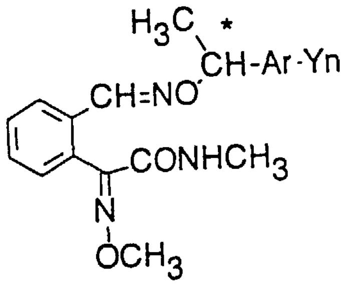

(I)

(in the above general formula, Ar represents an aryl group or a heteroaryl group, Y represents a hydrogen atom, a halogen atom, a $C_1$-$C_4$ alkyl group, a $C_1$-$C_4$ haloalkyl group, a $C_1$-$C_4$ alkoxy group, a $C_1$-$C_4$ haloalkoxy group, a $C_1$-$C_4$ alkylthio group, a $C_2$-$C_6$ alkynyloxy group, a cyano group, a nitro group or an aryl group and n is an integer of 1 to 3), wherein its angle of rotation shows a negative value in chloroform has markedly excellent activities as agricultural chemicals.

## Description

TECHNICAL FIELD

This invention relates to a novel optically active methoxyiminoacetamide derivative, to a method for the low cost and high efficiency production of said compound and to agricultural chemicals which use the compound as active ingredient.

BACKGROUND ART

It is known that certain methoxyiminoacetic acid derivatives have biological activities such as fungicidal effect and the like. For example, the specification of EP398692 discloses

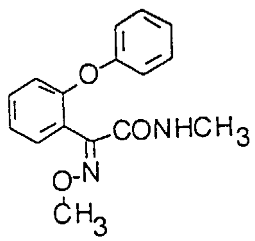

and the like.

The specification of EP499823 discloses

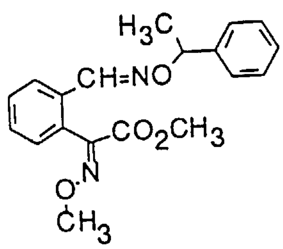

and the like.

The specification of EP596254 also discloses

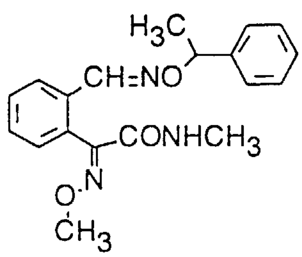

and the like.

However, as is evident from the results of test examples which will be described later, these compounds are not always satisfactory as agriculture and horticulture fungicides.

Certain types of such methoxyiminoacetic acid derivatives have asymmetric carbon atoms in their molecules, but their biological activities are known only for their racemates. It is known in general that as biological materials (enzymes and receptors) have a function to recognize these optical isomers in various manners, each enantiomer shows various activities, for example, an antipode shows no biological activities, similar but weak activities, competitive

activities or other activities {K. Mori, *Kagaku no Ryoiki Zokan*, 125, 155 (1980)}. In general, compounds having a similar skeleton have a tendency in that their toxicity increases in parallel with increase in their biological activities so that they cannot be put into practical use. In consequence, it became a first object also in the case of such methoxyiminoacetamide compounds to find a compound having conflicting effects, namely a compound which shows more superior effect as agricultural chemicals but having less influence upon organisms which are not to be controlled.

Also, it became a second object to find a method for the efficient production of such a compound. In general, when an optically active compound is synthesized for example by a method in which racemic forms are subjected to optical resolution, a method in which asymmetric synthesis is carried out or a method in which these techniques are combined, its production cost becomes higher than that of the synthesis of a racemic form, because the production steps are prolonged or expensive reagents are required. Particularly in the case of agricultural chemicals, their unit costs are low, which is different from the case of pharmaceutical preparations, so that the ratio of the cases using optically active forms is low due to the balance between increment of biological activity and increment of cost. Thus, in efficiently producing the optically active methoxyiminoacetamide compound of the present application, it became most important object to find an efficient production method of an optically active 1-arylethoxyamine derivative and an efficient condensation reaction using the derivative.

As a production method of an optically active 1-arylethoxyamine derivative, *Tetrahedron*, 51, 42, 11473 (1955) discloses that optically active N-(1-phenylethoxy)phthalimide having inverted configuration can be obtained with a yield of 49% and an optical purity of 95% by carrying out a so-called Mitsunobu method in which optically active 1-arylethylalcohol is allowed to react with N-hydroxyphthalimide, ethyl azodicarboxylic acid and triphenylphosphine. By allowing the thus isolated N-(1-phenylethoxy)phthalimide to react with hydrazine hydrate, an optically active 1-arylethoxyamine is obtained with a yield of 73%. Thus, N-hydroxyphthalimide is used in the prior art in order to obtain an optically active 1-arylethoxyamine, but such a method cannot be used for industrial purpose because of problems in that highly toxic and carcinogenic hydrazine must be used for the purpose of cutting off the phthalimide group, the reaction must be carried out at a high temperature and by-products due to the presence of the phthalimide group are generated in a large amount.

Examples of the known production method of racemic 1-arylethoxyamine derivatives include a method in which N-hydroxyurethane is allowed to react with a 1-arylethyl halide derivative, and the thus obtained carbethoxy hydroxamate derivative is hydrolyzed {*Helv. Chim. Acta*., 45, 1381 - 1395 (1962)}, a method in which the derivative of interest is directly produced by allowing a 1-arylethylalcohol derivative to react with chloramine {*J. Med. Chem*., 10, 556 - 564 (1967)} and a method in which acetone oxime is allowed to react with a 1-arylethyl halide derivative, and the thus obtained acetoxime derivative is hydrolyzed with an acid {*Tetrahedron*, 23, 4441 (1967), *Org. Synth. Coll* Vol. III, 172 (1955)}. However, these methods have low practical significance because, for example, each method has a low yield of 50% or less and chloramine is explosive.

With regard to a method for the production of an optically active 1-arylethylalcohol which can be used as the starting material for the optically active 1-arylethoxyamine derivative, known examples include a method in which aromatic ketones are subjected to asymmetric reduction using asymmetric oxaazaborolysine (*J. Am. Chem. Soc*., 112, 5741 (1990), a method in which asymmetric catalytic hydrogenation with high pressure hydrogen is carried out (*J. Am. Chem. Soc*., 2675 (1995)) and a method in which hydrogen transfer asymmetric reduction is carried out in the presence of a transition metal complex, a base, an optically active nitrogen-containing compound and a hydrogen donor (an unexamined published Japanese patent application (*kokai*) No. 9-157196). Also known are a method in which asymmetric hydrosilylation of styrenes is carried out in the presence of a palladium-asymmetric phosphine catalyst (*Chem. Pharm. Bull*., 43(6), 927 (1995)), a method in which phenylethylalcohols are converted into half esters with a dicarboxylic acid and then the resolution is effected with an optically active amine (*J. Am. Chem. Soc.*, 4513 (1985)) and a method in which asymmetric acylation is carried out using lipase to effect resolution (*Tetrahedron Assym*., 3285 (1996)).

Among these method, each of the asymmetric reduction methods has a low catalytic activity so that it requires a pressure apparatus and special materials thereof when high pressure hydrogen is used, thus entailing economic problem of high process cost. The method by asymmetric hydrosilylation has problems in that its asymmetric yield is low and it requires multiple steps. The resolution method has a limited yield by itself and the operation is complex, thus posing a difficulty in putting it into practical use.

With regard to a method for the production of an acetophenone derivative which can be used as the starting material for the 1-arylethylalcohol derivative, a method is known in which it is obtained by allowing an acyl chloride to react with Grignard reagent, arylborate anion or arylcadmium reagent of a bromobenzene derivative {*Res. Discl*., 386, 348 (1996), *J. Chem. Soc. Chem. Comm*., (5), 138 - 139 (1975), *J. Org. Chem*., 44(5), 696 - 699 (1979)}. Also known is a method in which it is obtained by allowing a benzonitrile derivative or a benzoyl chloride derivative to react with Grignard reagent of a methyl halide {*J. Am. Chem. Soc*., 70, 2837 - 2843 (1948), *J. Org. Chem*., 28(12), 3579 (1963)}.

However, the Grignard reaction method has a problem in that its workability is poor or it has a possibility of causing explosion, and the method in which borane or cadmium is used has a possibility of generating toxicity, so that they are not practical.

In consequence, a method in which an optically active 1-arylethoxyamine derivative is produced by a combination of the aforementioned techniques of known knowledge is impossible to realize, because a part or entire portion of the process will include certain steps which cannot be used industrially from the viewpoint of yield, safety or cost.

## DISCLOSURE OF THE INVENTION

A large number of agricultural chemicals have been developed and investigated without taking note of asymmetric carbon atoms of the compounds of interest, but, according to the studies conducted this time by the inventors of the present invention, it was found that an optically active methoxyiminoacetamide derivative which has a certain specific angle of rotation is possessed of a markedly excellent activity as agricultural chemicals, namely not only a superior fungicidal activity but also, to our surprise, an effect to exert little influences, such as toxicity, upon organisms that are not to be controlled, thus resulting in the accomplishment of the present invention.

Accordingly, the gist of the present invention resides in an optically active methoxyiminoacetamide derivative represented by the following general formula (I)

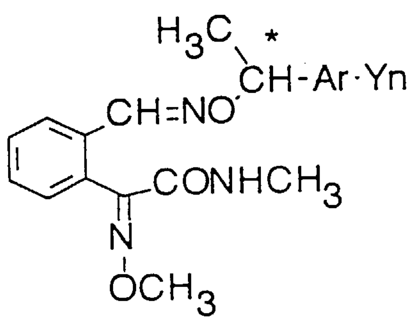

(I)

(in the above general formula, Ar represents an aryl group or a heteroaryl group, Y represents a hydrogen atom, a halogen atom, a $C_1$-$C_4$ alkyl group, a $C_1$-$C_4$ haloalkyl group, a $C_1$-$C_4$ alkoxy group, a $C_1$-$C_4$ haloalkoxy group, a $C_1$-$C_4$ alkylthio group, a $C_2$-$C_6$ alkynyloxy group, a cyano group, a nitro group or an aryl group and n is an integer of 1 to 3), wherein its angle of rotation shows a negative value in chloroform; in a production method and intermediate thereof, and in an agricultural chemical preparation comprising the same as the active ingredient as well as the use thereof.

## BRIEF DESCRIPTION OF DRAWINGS

Fig. 1 is a drawing showing changes in yield with time in Example 5-3 and Example 5-6. Fig. 2 is a drawing showing changes in optical purity of product with time in Example 5-3 and Example 5-6. Fig. 3 is a drawing showing changes in yield with time in Example 5-7 and Example 5-8. Fig. 4 is a drawing showing changes in optical purity of product with time in Example 5-7 and Example 5-8.

## BEST MODE OF CARRYING OUT THE INVENTION

The following describes the present invention in detail.

The optically active methoxyiminoacetamide derivative of the present invention is represented by the aforementioned general formula (I), and its angle of rotation shows a negative value in chloroform. It has an optical purity of preferably 50% ee or more, more preferably 70% ee or more. In addition, E/Z geometrical isomerism exist on its methoxyimine and 1-(hetero)arylethoxyimine moieties. Though E isomer is desirable for each moiety from the viewpoint of biological activity, all geometrical isomers on these moieties are included in the present invention.

In the aforementioned general formula (I), Ar is an aryl group such as phenyl, naphthyl or the like; or a heteroaryl group such as pyridyl, thiazolyl or the like. Among these groups, preferred is an aryl group, and more preferred is a phenyl group.

Y is a substituent linked to the ring of the aryl or heteroaryl group, and its examples include a hydrogen atom; halogen atoms such as fluorine, chlorine, bromine and the like; $C_1$-$C_4$ alkyl groups such as methyl, ethyl, n-propyl, isopropyl, n-butyl, sec-butyl and the like; $C_1$-$C_4$ haloalkyl groups such as trifluoromethyl, difluoromethyl, trichloromethyl, dichlorodifluoroethyl and the like; $C_1$-$C_4$ alkoxy groups such as methoxy, ethoxy, iso-propoxy, n-butoxy group and the

like; $C_1$-$C_4$ haloalkoxy groups such as trifluoromethoxy, difluoromethoxy, dichlorodifluoroethoxy and the like; $C_1$-$C_4$ alkylthio groups such as methylthio, ethylthio and the like; $C_2$-$C_6$ alkinyloxy groups such as propargyloxy and the like; a cyano group; a nitro group; or an aryl group such as phenyl and the like. Alternatively, adjacent two substituents of these substituents may together become a methylenedioxy group or an ethylenedioxy group and form a fused ring with the aryl or heteroaryl ring. Among these groups, preferred is a hydrogen atom, a halogen atom, a $C_1$-$C_4$ alkyl group, a $C_1$-$C_4$ haloalkyl group, a $C_1$-$C_4$ alkoxy group or a $C_1$-$C_4$ haloalkoxy group, more preferred is a hydrogen atom, a chlorine atom, a $C_1$-$C_2$ haloalkyl group or a $C_1$-$C_2$ haloalkoxy group, and most preferred is a trifluoromethyl group.

Also, n is an integer of 1 to 3. It is preferably 1 or 2.

All of the optically active methoxyiminoacetamide derivatives (I) of the present invention are novel compounds and can be produced for example in accordance with the following reaction schemes. Among these methods, the method shown by the scheme-1 is desirable from the viewpoint of reaction yield.

<Scheme-1>

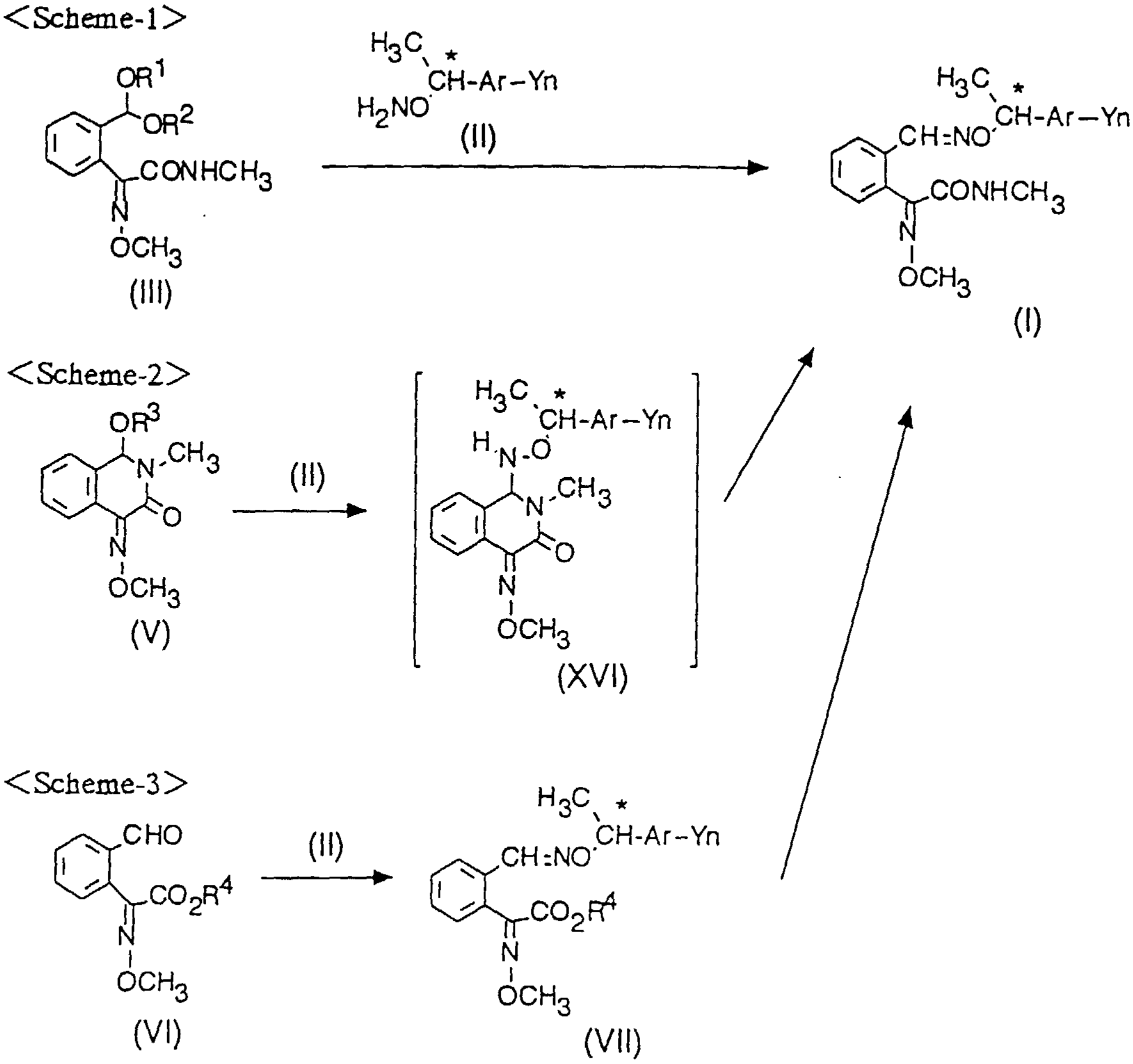


(III) (I)

<Scheme-2>

(V) (XVI)

<Scheme-3>

(VI) (VII)

In the above reaction scheme, Ar, Y and n are as defined in the aforementioned general formula (I). Each of $R^1$ and $R^2$ in the general formula (III) is independently a $C_1$-$C_4$ alkyl group such as methyl, ethyl, n-propyl, iso-propyl, n-butyl, sec-butyl or the like ; or $R^1$ and $R^2$ may together form a $C_2$-$C_4$ alkylene group such as ethylene, propylene or the like. Among these groups, preferred is a methyl group, an ethyl group, or an ethylene group or an propylene group formed by $R^1$ and $R^2$ together, and more preferred is an ethylene group formed by $R^1$ and $R^2$ together.

$R^3$ in the general formula (V) is a hydrogen atom or a $C_1$-$C_4$ alkyl group such as methyl, ethyl, n-propyl, iso-propyl, n-butyl, sec-butyl or the like. Preferred of these is a hydrogen atom, a methyl group or an ethyl group.

$R^4$ in the general formulae (VI) and (VII) is a $C_1$-$C_6$ alkyl group such as methyl, ethyl, n-propyl, iso-propyl, n-butyl, sec-butyl or the like. Preferred of these is methyl, ethyl or n-propyl.

In the reaction of the aforementioned scheme-1, the optically active methoxyiminoacetamide derivative (I) is produced by allowing an acetal derivative (III) to react with an optically active 1-arylethoxyamine derivative (II) or a salt thereof without solvent or in an inert solvent in the presence or absence of an appropriate acid.

The 1-arylethoxyamine derivative (II) may be used as it is or in the form of an inorganic or organic acid salt as occasion demands, in an amount of from 1 to 5 equivalents, preferably from 1 to 3 equivalents, more preferably from 1 to 1.5 equivalents, based on the compound (III).

Examples of the acid salt include inorganic acid salts such as with hydrochloric acid, sulfuric acid and the like; and organic acid salts such as with L-(+)-tartaric acid, D-(-)-tartaric acid, (-)-dibenzoyltartaric acid, (-)-malic acid, (+)-10-camphorsulfonic acid, (-)-pyroglutamic acid, (+)-aspartic acid, optically active $\alpha$-phenylethanesulfonic acid, optically active mandelic acid, optically active cis-2-benzamidocyclohexane carboxylic acid, formic acid, acetic acid, propionic acid, butyric acid and the like. Preferred are organic acid salts in view of the reactivity. When an optically resolved 1-arylethoxyamine derivative (II) is used, inexpensive tartaric acid salt is desirable in view of the process operation.

Examples of the acid to be used in this reaction include the just described inorganic and organic acids, as well as sulfonic acids such as methanesulfonic acid, p-toluenesulfonic acid and the like; acid addition salts of organic bases such as pyridine hydrochloride, pyridine sulfate and the like; or Lewis acids such as zinc chloride, iron chloride, aluminum chloride and the like. Among these acids, organic acids are preferred and acetic acid is more preferred. The acid is used in an amount of from 0.001 to 100 equivalents, preferably from 0.01 to 50 equivalents, more preferably from 0.1 to 10 equivalents, based on the compound (III).

Examples of the solvent include saturated hydrocarbons such as n-hexane, n-heptane, iso-octane and the like; aromatic hydrocarbons such as benzene, toluene, xylene and the like; halogenated hydrocarbons such as dichloromethane, chloroform, 1,2-dichloroethane and the like; ethers such as diethyl ether, tetrahydrofuran, dioxane and the like; esters such as ethyl acetate and the like; ketones such as acetone, methyl ethyl ketone and the like; alcohols such as methanol, ethanol, propanol, n-butanol and the like; nitriles such as acetonitrile and the like; and polar solvents such as N,N-dimethylformamide, N-methylpyrrolidone, dimethyl sulfoxide, acetic acid, water and the like, which may be used alone or as a mixed solvent. Among these solvents, ethers, alcohols, and polar solvents (e.g., N,N-dimethylformamide, acetic acid, water, and the like) are preferable. A mixed solvent containing water is particularly preferred, because the reaction rate and yield are improved and also chemical purity and optical purity of the methoxyiminoacetamide derivative (I) after crystallization can be increased. The ratio of water to be used as a solvent is within the range of from 0.5 to 100%, preferably from 10 to 70%, more preferably from 20 to 50%.

The amount of the solvent is within the range of from 0.5 to 1,000 equivalents, preferably from 1 to 50 equivalents based on the compound (III).

The reaction can be carried out at a temperature of from -20°C to boiling point of the solvent to be used, preferably from 0 to 100°C, more preferably from 10 to 60°C.

The reaction time may vary depending on the reaction temperature, but it is generally within 50 hours when the reaction is carried out at around room temperature. The reaction completes within 25 hours when the aforementioned water-containing mixed solvent is used, and the reaction can be completed within 10 hours when the 1-arylethoxyamine derivative (II) is used in the form of an organic acid salt.

After completion of the reaction, the product of interest may be purified in accordance with the usually used method in which the reaction mixture is extracted with an organic solvent, washed and concentrated and then the thus obtained residue is crystallized using an organic solvent, but it is desirable to use the aforementioned water-containing mixed solvent, because crystals are precipitated gradually during the reaction so that the product having high chemical purity and optical purity can be obtained easily by simply collecting the final crystals by filtration. On the occasion, it is necessary to pay attention to the ratio of organic solvent to water in the mixed solvent, because the isolation yield varies depending on solubility of products to the mixed solvent.

As an alternative production method of the optically active methoxyiminoacetamide derivative (I), it can be produced by allowing a 1-oxy-4-methoxyimino-2-methyl-3-oxo-1,2,3,4-tetrahydroisoquinoline derivative (V) to react with the optically active 1-arylethoxyamine derivative (II) in an inert solvent in the presence or absence of an acid, if necessary via a compound of formula (XVI) (scheme-2).

The optically active 1-arylethoxyamine derivative (II) may be used as it is or in the form of a salt as described in the aforementioned scheme-1, in an amount of from 1 to 5 equivalents, preferably from 1 to 2 equivalents, based on the 1-oxy-4-methoxyimino-2-methyl-3-oxo-1,2,3,4-tetrahydroisoquinoline derivative (V).

In this reaction, the methoxyiminoacetamide derivative of interest represented by the general formula (I) can be obtained stably with a high yield by the addition of an acid.

Examples of the acid to be used include those which are described in the aforementioned scheme-1, as well as hydrohalogenic acids such as hydrochloric acid and the like. Among these acids, sulfonic acids and hydrohalogenic acids are preferred.

The just described acid may be used in an amount of up to 5 equivalents, preferably from 0.01 to 2 equivalents,

based on the 1-oxy-4-methoxyimino-2-methyl-3-oxo-1,2,3,4-tetrahydroisoquinoline derivative (V).

As the solvent, similar solvents described in the aforementioned scheme-1 can be used. Among these solvents, ethers such as tetrahydrofuran, dioxane and the like; and polar solvents such as N,N-dimethylformamide, dimethyl sulfoxide, acetic acid and the like are desirable.

The solvent may be used in an amount of from 0.5 to 1,000 equivalents, preferably from 1 to 50 equivalents, based on the compound (III).

The reaction is carried out at a temperature of from 0°C to boiling point of the solvent to be used, preferably from 20 to 120°C.

The reaction completes generally within the range of from 1 to 24 hours.

When the aforementioned acid is not used in this reaction, a compound represented by the general formula (XVI) may be obtained as an intermediate product which, however, can be converted into the intended methoxyiminoacetamide derivative (I) by adding the aforementioned acid and continuing the reaction in the aforementioned manner.

As shown in the scheme-3, the optically active methoxyiminoacetamide derivative (I) can also be produced by allowing a benzaldehyde derivative (VI) to react with the optically active 1-arylethoxyamine derivative (II) or a salt thereof and then carrying out amidation of the thus obtained methoxyiminoacetic acid ester derivative (VII) by adding methylamine.

The optically active 1-arylethoxyamine derivative (II) or a salt thereof may be used in an amount of from 1 to 5 equivalents, preferably from 1 to 2 equivalents, based on the benzaldehyde derivative (VI). Methylamine may be used in an amount of from 1 equivalent to a large excess, preferably from 1 to 5 equivalents, based on the methoxyiminoacetic acid ester derivative (VII).

As the solvent, similar solvents described in the aforementioned scheme-1 can be used. Among these solvents, alcohols such as methanol, ethanol and the like are desirable. The solvent may be used in an amount of from 0.5 to 1,000 equivalents, preferably from 1 to 50 equivalents, based on the compound (III).

The reaction is carried out at a temperature of from 0°C to boiling point of the solvent to be used, preferably from 20 to 120°C. The reaction completes generally within the range of from 1 to 24 hours.

The optically active methoxyiminoacetic acid ester derivative represented by the general formula (VII) is a novel compound and can be used as an intermediate for the production of the methoxyiminoacetamide derivative (I) of the present invention.

In this production process, the methoxyiminoacetic acid ester derivative (VII) may be isolated and purified in the middle of the reaction and subjected to amidation, but it is more economical to carry out its amidation continuously by adding methylamine.

The acetal derivative (III), the 1-oxy-4-methoxyimino-2-methyl-3-oxo-1,2,3,4-tetrahydroisoquinoline derivative (V) and the benzaldehyde derivative (VI) as starting materials for the aforementioned reactions can be produced in accordance with the methods described in the specifications of PCT Application No. JP 96-01464 (WO 96-38408), PCT Application No. JP 96-01349 (WO 96-37463) and a national phase published Japanese patent application (*toppyo*) No. 8-508726 and EP393428, respectively, or by modified methods thereof.

In this connection, the acetal derivative (III) can be prepared in accordance with the following scheme-4.

<Scheme-4>

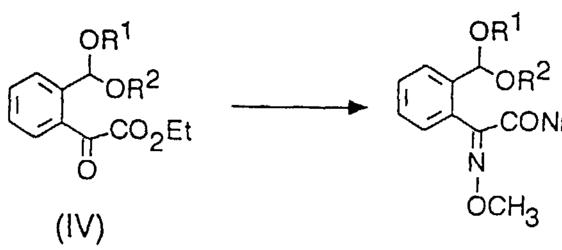

(IV) (III)

(In the above formula, $R^1$ and $R^2$ are as defined in the general formula (III). When a phenylglyoxylic acid ester derivative (IV) is allowed to react with methoxyamine or a salt thereof in an inert solvent while controlling the reaction pH at 4 to 6 and then to react with methylamine, E isomer (III) on oxime moiety can be obtained with a high yield of from 80 to 95%, which is desirable when a methoxyiminoacetamide derivative (I) is obtained as the final compound.

As the methoxyamine or a salt thereof (hydrochloride or sulfate for example), its anhydride may be used, but its use in a form of aqueous solution is economical, because the reaction progresses smoothly even in such a form. In that case, it is desirable to remove water layer by separating the reaction solution prior to the addition of methylamine,

because increase of temperature caused by heat of neutralization can be avoided.

Methoxyamine or a salt thereof may be used in an amount of from 1 to 5 equivalents, preferably from 1 to 2 equivalents, more preferably from 1 to 1.5 equivalents, based on the phenylglyoxylic acid ester derivative (IV).

In the case of reacting with methoxyamine in a solvent containing water, it is preferred to control the reaction pH in the range of from 4 to 6. Especially in the case of reacting with methoxyamine in the form of acid salt thereof, formation of by-products can be suppressed by allowing an appropriate base to coexist in order to control the reaction pH in aforementioned range, so that the derivative of interest can be produced stably with more higher yield.

Examples of the base to be used include inorganic bases such as alkali metal hydroxide (e.g., sodium hydroxide or the like); alkaline earth metal hydroxide (e.g., calcium hydroxide or the like); alkali metal bicarbonate (e.g., sodium bicarbonate or the like); alkali metal carbonate (e.g., sodium carbonate or the like); and alkaline earth metal carbonate (e.g., calcium carbonate or the like), or organic bases such as alkali metal carboxylate (e.g., sodium acetate or the like); alkaline earth metal carboxylate (e.g., calcium acetate or the like); tertiary amine (e.g., triethylamine, N-methylmorpholine, N,N-dimethylaniline or the like); pyridines (e.g., pyridine, alkyl group-substituted pyridines such as 4-picoline, 2,6-lutidine, or the like), or polymer bases such as polyvinyl pyrrolidone (cross-linked), polyvinyl pyridine or the like.

Among the above organic bases, preferred are organic bases having a conjugate acid pka value of from 5 to 8, and more preferred are organic bases having a conjugate acid pKa value of from 5.5 to 7, such as mono- or di-alkyl-substituted pyridines (e.g, 4-picoline, 2,6-lutidine and the like) and tertiary amines (e.g., N,N-dimethylaniline and the like).

These bases may be added gradually while controlling the reaction pH to from 4 to 6, but, when an organic base having a conjugate acid pKa value of from 5 to 8 (preferably, 5.5 to 7) is used, it becomes a buffer solution, so that said organic base can be charged in advance in one portion which is advantageous from the viewpoint of simplifying the operation.

The base may be used in an amount of from 1 to 5 equivalents, preferably from 1 to 2 equivalents, based on methoxyamine or a salt thereof.

As another preferable embodiment of this reaction, it is preferred to use organic bases having a conjugate acid pKa value of from 5.5 to 7 in the reaction with methoxyamine. Examples of the organic bases to be used include mono- or di-alkyl-substituted pyridines (e.g, 4-picoline, 2,6-lutidine and the like) and tertiary amines (e.g., N,N-dimethylaniline and the like).

With regard to methylamine to be used in this reaction, any of its forms including alcohol solution, aqueous solution and gas is effective in progressing the reaction, but the compound of interest can be produced economically advantageously when aqueous solution of methylamine is used, because it renders possible quick reaction and easy handling. It is used in an amount of generally from 1 to 15 equivalents, preferably from 1 to 5 equivalents, more preferably from 1 to 3 equivalents, based on the phenylglyoxylic acid ester derivative (IV).

Examples of the solvent to be used in this reaction include saturated hydrocarbons such as n-hexane, c-hexane, n-heptane, iso-octane and the like; aromatic hydrocarbons such as benzene, toluene, xylene and the like; halogenated hydrocarbons such as dichloromethane, chloroform, 1,2-dichloroethane and the like; ethers such as diethyl ether, tetrahydrofuran, dioxane, t-butyl methyl ether, diisopropyl ether, dibutyl ether and the like; alcohols such as methanol, ethanol, propanol, n-butanol, t-butanol, 2-ethylhexanol and the like; and polar solvents such as N,N-dimethylformamide, N-methylpyrrolidone, dimethyl sulfoxide, pyridine, water and the like, which may be used alone or as a mixed solvent. Preferred of these are alcohols and polar solvents (e.g., N,N-dimethylformamide and the like), more preferably n-butanol.

The solvent may be used in an amount of from 0.5 to 1,000 equivalents, preferably from 1 to 50 equivalents, based on the compound (IV).

The reaction is carried out at a temperature of from -10°C to boiling point of the solvent to be used, preferably from 0°C to 100°C, more preferably from 10°C to 70°C.

The reaction completes generally within 1 to 24 hours.

After completion of the reaction, the product of interest may be purified in accordance with the usually used method in which the reaction mixture is extracted with an organic solvent, washed and concentrated and then the thus obtained residue is crystallized using an organic solvent, but it is desirable to use the aforementioned n-butanol as a reaction solvent, because not only the reaction rate becomes quick but also the formed acetal derivative (III) precipitates as crystals during the reaction, so that the isolation and purification can be effected by simply subjecting the reaction mixture to filtration or centrifugation, which is industrially advantageous.

The phenylglyoxylic acid ester derivative (IV) to be used as the starting material for the scheme-4 can be produced in accordance with the method described in the specification of PCT Application No. JP 96-01464 (WO 96-38408) or by a modified method thereof.

The optically active 1-arylethoxyamine derivative (II) to be used as another starting material for the production of the methoxyiminoacetamide derivative (I) of the present invention is a novel compound. It may have an optical purity of preferably 50% ee or more, more preferably 70% ee or more.

Said compound can be produced by a method in which its racemates are optically resolved, a method in which asymmetric synthesis is employed or a combined method thereof, and each of such cases is described in the following.

When optical resolution is carried out, an acidic optical resolution agent is added to the racemic 1-arylethoxyamine derivative (II)' and dissolved under heating in an inert solvent and then half the amount of the salt is precipitated. Alternatively, the optically active 1-arylethoxyamine derivative (II) obtained by asymmetric synthesis may be subjected to optical resolution in order to further improve the optical purity.

Examples of the acidic optical resolution agent to be used include L-(+)-tartaric acid, D-(-)-tartaric acid, (-)-dibenzoyltartaric acid, (-)-malic acid, (+)-10-camphorsulfonic acid, (-)-pyroglutamic acid, (+)-aspartic acid, optically active $\alpha$-phenylethanesulfonic acid, optically active mandelic acid, optically active cis-2-benzamidocyclohexane carboxylic acid and the like. Preferred of these are optically active tartaric acids.

The acidic optical resolution agent may be used in an amount of from 0.3 to 2 equivalents, preferably from 0.5 to 1.5 equivalents, more preferably from 0.5 to 1.1 equivalents, based on the 1-arylethoxyamine derivative (II)'.

Examples of the solvent to be used include aromatic hydrocarbons such as benzene, toluene, xylene and the like; hydrocarbons such as n-hexane, n-heptane, iso-octane and the like; halogenated hydrocarbons such as dichloromethane, chloroform, 1,2-dichloroethane and the like; ethers such as diethyl ether, diisopropyl ether, t-butyl methyl ether, tetrahydrofuran, dioxane, dibutyl ether and the like; esters such as ethyl acetate and the like; alcohols such as methanol, ethanol, propanol, n-butanol, n-hexanol and the like; and polar solvents such as N,N-dimethylformamide, N-methylpyrrolidone, dimethyl sulfoxide, acetonitrile, water and the like, which may be used alone or as a mixed solvent.

Preferred of these are aromatic hydrocarbons, saturated hydrocarbons, alcohols or ethers, which may be used alone or as a mixed solvent with the above solvents. More preferred is methanol, ethanol, n-butanol, diisopropyl ether, dibutyl ether or tetrahydrofuran, alone or a mixed solvent with the aforementioned solvents. As the solvents suitable for use in the mixture, aromatic hydrocarbons (e.g., toluene, xylene and the like) and hydrocarbons (e.g., n-heptane, iso-octane and the like) are particularly preferred.

The solvent may be used in an amount of from 0.5 to 1,000 equivalents, preferably from 1 to 50 equivalents, based on the substrate.

The reaction is carried out by heating the reaction system up to boiling point of the solvent to be used, thereby dissolving the salt, and then gradually cooling the system to effect precipitation of crystals. The final temperature is generally within the range of from -20 to 50°C, though not particularly limited. Also, it is possible to effect precipitation of the crystals by evaporating a portion of the solvent after dissolution of the salt. This method is particularly effective in increasing optical purity of the 1-arylethoxyamine derivative (II) obtained by asymmetric synthesis.

The time for optical resolution varies depending on each method and is not particularly limited, but it is generally within 48 hours.

The optically active 1-arylethoxyamine derivative (II) can be isolated by neutralizing the salt of optically active compound obtained by optical resolution, generally with a base such as sodium hydroxide aqueous solution. In producing the methoxyiminoacetamide derivative (I), the thus isolated optically active 1-arylethoxyamine derivative (II) may be allowed to react with the acetal derivative (III) in accordance with the scheme-1, but it was found surprisingly that the amount of the acid to be added as a catalyst at the time of the final step condensation reaction can be reduced or eliminated when the thus precipitated optically active salt is allowed as it is to undergo the reaction in the same manner as shown in the aforementioned scheme-1, so that the methoxyiminoacetamide derivative (I) of interest can be produced further more efficiently, which is economically advantageous.

The racemic 1-arylethoxyamine derivative (II)' to be used for the optical resolution can be produced by the method shown in the following scheme-5.

<Scheme-5>

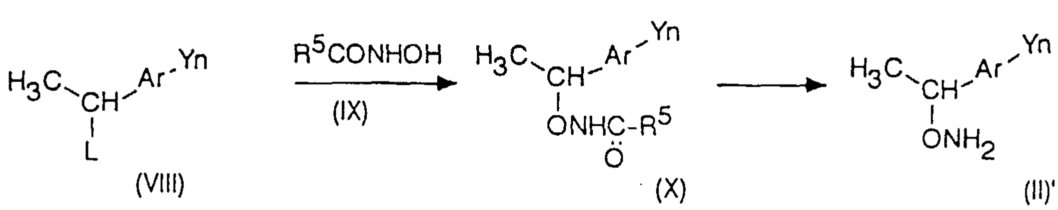


(VIII) (X) (II)'

In the above reaction scheme, Ar, Y and n are as defined in the foregoing. L is a halogen atom such as fluorine,

chlorine, bromine or the like, preferably chlorine. $R^5$ is a $C_1$-$C_6$ alkyl group such as methyl, ethyl, n-propyl, iso-propyl, n-butyl, sec-butyl or the like; a $C_1$-$C_6$ alkoxy group such as methoxy, ethoxy, iso-propoxy, t-butoxy or the like; a benzyloxy group; a phenoxy group; or a phenyl group. Preferred of these is a $C_1$-$C_6$ alkyl group, a $C_1$-$C_6$ alkoxy group or a phenyl group, and more preferred is a methyl group, a t-butoxy group or a phenyl group.

The 1-arylethoxyamine derivative represented by the general formula (II)' is produced by hydrolyzing a hydroxamic acid derivative represented by the general formula (X) in an inert solvent using an appropriate acid or base.

The acid or base may be used in an amount of from 1 to 20 equivalents, preferably from 1 to 10 equivalents, more preferably from 1 to 5 equivalents, based on the hydroxamic acid derivative (X).

It is desirable to use an acid in order to complete the reaction within a short period of time with a high yield.

Examples of the acid to be used include organic acids such as acetic acid, trifluoroacetic acid and the like; hydrohalogenic acids such as hydrochloric acid and the like; hydrogen halides such as hydrogen chloride and the like; sulfonic acids such as methanesulfonic acid, p-toluenesulfonic acid, sulfuric acid and the like; acid addition salts of organic bases such as pyridine hydrochloride, pyridine sulfate and the like; and Lewis acids such as zinc chloride, iron chloride, aluminum chloride and the like. Among these acids, hydrohalogenic acids or sulfonic acids are preferred.

Examples of the base to be used include alkali metal hydroxide such as sodium hydroxide, potassium hydroxide or the like; alkali metal or alkaline earth metal carbonate such as sodium carbonate, potassium carbonate, calcium carbonate or the like; alkali metal bicarbonate such as sodium bicarbonate, potassium bicarbonate or the like; and metal alcoholate such as sodium ethylate, t-butoxy potassium or the like. Preferred of these are alkali metal hydroxides.

Examples of the solvent to be used include aromatic hydrocarbons such as benzene, toluene, xylene and the like; hydrocarbons such as n-hexane, n-heptane, cyclohexane and the like; halogenated hydrocarbons such as dichloromethane, chloroform, 1,2-dichloroethane and the like; ethers such as diethyl ether, tetrahydrofuran, dioxane and the like; esters such as ethyl acetate and the like; ketones such as acetone, methyl ethyl ketone and the like; alcohols such as methanol, ethanol, propanol, n-butanol and the like; nitriles such as acetonitrile and the like; and polar solvents such as N,N-dimethylformamide, N-methylpyrrolidone, dimethyl sulfoxide, acetic acid, water and the like, which may be used alone or as a mixed solvent. Among these solvents, polar solvents such as ethers, alcohols and water and the like are preferred.

The reaction can be carried out at a temperature of from room temperature to boiling point of the solvent to be used, preferably from 20 to 120°C, more preferably from 30 to 70°C.

The hydroxamic acid derivative represented by the general formula (X) is produced by allowing the 1-arylethyl halide derivative represented by the general formula (VIII) to react with the hydroxamic acid (IX) in an inert solvent in the presence of an appropriate base.

The base to be used may be either an inorganic base or an organic base. Examples of the inorganic base include alkali metal hydroxide such as sodium hydroxide, potassium hydroxide or the like; alkali metal or alkaline earth metal carbonate such as sodium carbonate, potassium carbonate, calcium carbonate or the like; alkali metal bicarbonate such as sodium bicarbonate, potassium bicarbonate or the like; alkali metal hydride such as sodium hydride or the like; and alkali metal such as metallic sodium or the like. Examples of the inorganic base include tertiary amine such as triethylamine, 1,8-diazabicyclo[5.4.0]undec-7-ene (DBU), 1,5-diazabicyclo[4.3.0]non-5-ene (DBM), 1,4-diazabicyclo[2.2.2]octane (Dabco) or the like; aromatic base such as pyridine, picoline or the like; and alkali metal alcoholate such as sodium ethylate, t-butoxy potassium or the like. Preferred of these include an alkali metal carbonate, an alkali metal bicarbonate, an alkali metal hydroxide and an alkali metal alcoholate.

The just described base may be used in an amount of from 1 to 20 equivalent, preferably from 1 to 10 equivalent, more preferably from 1 to 5 equivalents, based on the 1-arylethyl halide derivative represented by the aforementioned general formula (VIII).

Optional use of a generally known phase-transfer catalyst (e.g., tetra-n-butylammonium bromide, tetra-n-butylammonium hydrogensulfate, trimethylstearylammonium chloride or the like), in order to proceed the reaction more smoothly, is economically advantageous, because the reaction time which is generally 7 to 20 hours can be shortened to 4 to 10 hours.

As the solvent, the same compounds described in the foregoing in relation to the hydrolysis reaction can be used. Its preferred examples include ethers, alcohols or polar solvents (e.g., N,N-dimethylformamide, dimethyl sulfoxide, water and the like).

The solvent may be used in an amount of from 0.5 to 1,000 equivalents, preferably from 1 to 50 equivalents, based on the substrate.

The reaction may be carried out at a temperature of from room temperature to boiling point of the solvent to be used, preferably from 20 to 150°C, more preferably from 30 to 90°C.

The 1-arylethoxyamine derivative represented by the general formula (II)' may be obtained by producing the hydroxamic acid derivative represented by the general formula (X) by the aforementioned method and then hydrolyzing the product after its isolation and purification, but the reaction progresses smoothly when the hydrolysis is carried out without isolating the hydroxamic acid derivative (X) in order to avoid complex operation, which is economically advantageous.

The hydroxamic acid derivative (X) to be used as an intermediate of the production method is a novel compound in which, preferably as the intermediate of the present invention, Ar is an aryl group, $R^5$ is a $C_1$-$C_6$ alkyl group, a $C_1$-$C_6$ alkoxy group or a phenyl group, Y is a $C_1$-$C_4$ haloalkyl group and n is 1. More preferably, Ar is an aryl group, $R^5$ is a methyl group, a t-butoxy group or a phenyl group, Y is a trifluoromethyl group and n is 1.

A method shown by the following scheme-6 can be exemplified as a method for the efficient production of the optically active 1-arylethoxyamine derivative (II) by asymmetric synthesis.

<Scheme-6>

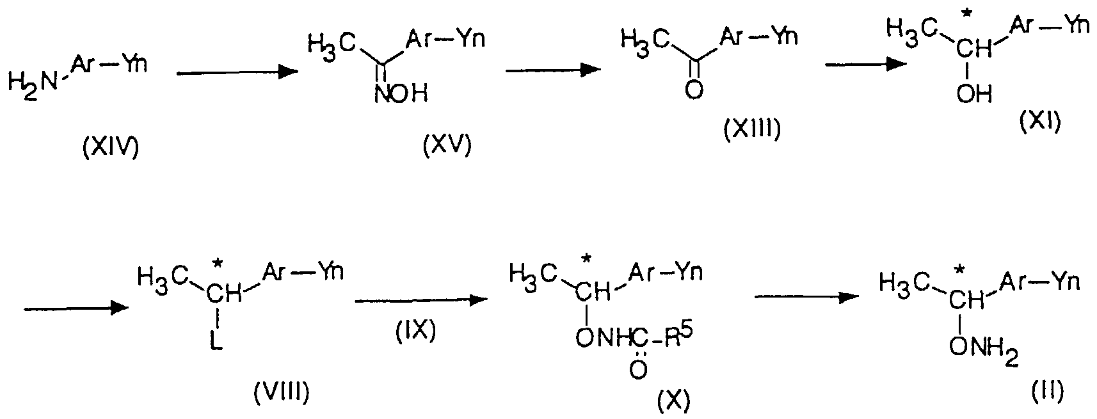


(XIV) (XV) (XIII) (XI) (VIII) (X) (II)

In the above reaction scheme, L, $R^5$, Ar, Y and n are as defined in the foregoing.

In the step for the production of the optically active 1-arylethoxyamine derivative (II) using the optically active 1-arylethyl halide derivative (VIII) as the starting material, it can be produced efficiently with extremely infrequent racemization by using the method described in the foregoing in relation to the production method of the racemic 1-arylethoxyamine derivative (II)'.

However, in order to increase its optical yield, it is desirable to carry out the reaction at a temperature of 100°C or less.

In said reaction, the optically active hydroxamic acid derivative (X) can be obtained efficiently with inversion of the configuration of asymmetric carbon atom, by allowing the optically active 1-arylethyl halide derivative (VIII) to react with the hydroxamic acid (IX) in an inert solvent in the presence of an appropriate base. By further hydrolyzing it in an inert solvent using an appropriate acid or base, the optically active 1-arylethoxyamine derivative (II) retaining the configuration of the optically active hydroxamic acid derivative (X) is obtained.

The optically active 1-arylethyl halide derivative (VIII) to be used as the starting material of the optically active 1-arylethoxyamine derivative (II) can be produced efficiently with inversion of configuration, by treating the optically active 1-arylethylalcohol derivative (XI) with a halogenating agent in the presence of a base.

Examples of the halogenating agent include substituted or unsubstituted alkylsulfonyl halide such as methanesulfonyl chloride, ethanesulfonyl chloride, methanesulfonyl bromide, benzylsulfonyl chloride or the like; substituted or unsubstituted arylsulfonyl halide such as benzenesulfonyl chloride, p-toluenesulfonyl chloride, naphthalenesulfonyl chloride, benzenesulfonyl bromide, p-toluenesulfonyl bromide, naphthalenesulfonyl bromide or the like; thionyl halide such as thionyl chloride, thionyl bromide or the like; phosphorous oxyhalide such as phosphorous oxychloride or the like; phosphorous halide such as phosphorous trichloride, phosphorous pentachloride, phosphorous tribromide or the like; phosgene; trichloromethyl chloroformate; and bistrichloromethyl carbonate.

Preferred of these include a $C_1$-$C_6$ alkylsulfonyl halide; or an arylsulfonyl halide which may be substituted with a substituent selected from $C_1$-$C_4$ alkyl groups and halogen atoms, and more preferred is methanesulfonyl chloride or p-toluenesulfonyl chloride.

Though not particularly limited, the halogenating agent may be used in an amount of from 1.0 to 10 equivalents, preferably from 1.0 to 5.0 equivalents, more preferably from 1.0 to 2.5 equivalents, based on the optically active 1-arylethylalcohol derivative (XI).

Examples of the base to be used in the reaction include inorganic bases such as alkali metal hydroxide (e.g,

sodium hydroxide, potassium hydroxide or the like); alkaline earth metal hydroxide (e.g., calcium hydroxide or the like); alkali metal bicarbonate (e.g., sodium bicarbonate or the like); alkali metal carbonate (e.g., lithium carbonate, potassium carbonate, sodium carbonate or the like); and alkaline earth metal carbonate (e.g., calcium carbonate or the like), or organic bases such as alkali metal carboxylate (e.g., sodium acetate, potassium acetate or the like); alkaline earth metal carboxylate (e.g., calcium acetate or the like); tertiary amine (e.g., triethylamine, N-methylmorpholine, tributylamine, N,N-dimethylaniline or the like); pyridines (e.g., pyridine, chloropyridine, 2-picoline, 3-picoline, 4-picoline, 2,6-lutidine, collidine or the like); and polymer bases (e.g., polyvinyl pyrrolidone (cross-linked), polyvinyl pyridine or the like).

Among these bases, carbonate such as potassium carbonate or the like is desirable as the inorganic base, but an organic base is more desirable than the inorganic base in view of reaction yield and reaction rate. As the organic base, pyridines (e.g., pyridine, chloropyridine, 2-picoline, 3-picoline, 4-picoline or the like) which may be substituted with a halogen atom or a $C_1$-$C_4$ alkyl group is more preferred, and pyridine is most preferred.

Though not particularly limited, the base may be used in an amount of from 1.0 to 10 equivalents, preferably from 1.0 to 5.0 equivalents, more preferably from 1.0 to 2.5 equivalents, based on the optically active 1-arylethylalcohol derivative (XI).

Though this reaction can be carried out without solvent, it can also be carried out by diluting the reaction mixture with an organic solvent as occasion demands. Examples of the solvent to be used include hydrocarbons such as n-hexane, n-heptane, cyclohexane and the like; halogenated hydrocarbons such as carbon tetrachloride, chloroform, dichloromethane, 1,2-dichloroethane and the like; ethers such as tetrahydrofuran, 1,4-dioxane, dimethoxyethane, diethoxyethane, diethyl ether, t-butyl methyl ether, dibutyl ether and the like; aromatic hydrocarbons such as benzene, toluene and the like; esters such as ethyl acetate, methyl propionate and the like; ketones such as acetone, methyl ethyl ketone, acetyl acetone, methyl isobutyl ketone and the like; and polar solvents such as N,N-dimethylformamide, N-methylpyrrolidone, N,N-dimethylimidazolinone, hexamethyl phosphoric triamide and the like, which may be used alone or as a mixed solvent. Preferred of these is a single solvent or a mixed solvent selected from hydrocarbons, ethers, esters, ketones, N,N-dimethylformamide, N-methylpyrrolidone and aprotic polar solvents (e.g., N,N-dimethylimidazolinone, hexamethyl phosphoric triamide and the like). More preferred is a single solvent or a mixed solvent of hydrocarbons, t-butyl methyl ether, N,N-dimethylformamide, N-methylpyrrolidone, N,N-dimethylimidazolinone, hexamethyl phosphoric triamide.

Among these solvents, N,N-dimethylformamide has a reaction acceleration effect as will be illustratively described later in Examples, and it can improve optical purity when it is made into a mixed solvent with hydrocarbons (e.g., n-heptane and the like) or ethers (e.g., t-butyl methyl ether and the like). In consequence, it is markedly efficient for the halogenation reaction to use a mixed solvent containing N,N-dimethylformamide.

The ratio of N,N-dimethylformamide to be used as the solvent, though it may vary depending on other solvent to be mixed therewith, may be within the range of from 0.5 to 99%, preferably from 1 to 60%, more preferably from 2 to 50%.

Though not particularly limited, the solvent may be used in an amount of from 0 to 100 times (by weight), preferably from 1 to 10 times, of the optically active 1-arylethylalcohol derivative (XI).

This reaction is carried out by charging the optically active 1-arylethylalcohol derivative (XI) and a base, as well as an organic solvent as occasion demands, and adding thereto a halogenating agent dropwise at a temperature of from -50°C to 50°C, preferably from -30°C to 30°C, more preferably from -30°C to 10°C. In this case, optical purity and yield of the formed optically active 1-arylethyl halide derivative (VIII) can be increased by carrying the dropwise addition slowly to prevent increase in the reaction temperature. Alternatively, the reaction may be carried out by charging the halogenating agent in advance and then adding dropwise the base slowly so that the reaction temperature does not increase. After addition of the halogenating agent or base, the reaction can be completed by carrying out the reaction for 0.5 to 50 hours while keeping the temperature or increasing it up to 100°C, preferably up to 50°C, more preferably up to 30°C.

After completion of the reaction, the optically active 1-arylethyl halide derivative (VIII) with inverted configuration can be isolated by adding water to the reaction mixture and extracting the product with an organic solvent, washing it optionally with an acid and a base and then concentrating the extract. In this connection, when the solvent to be used in this reaction is also used in the subsequent reaction, the extract may be subjected to the nest step without its concentration.

When a sulfonyl halide is used as a halogenating agent in this reaction, an optically active sulfonic acid ester derivative represented by the following general formula

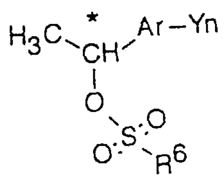

(XII)

(in the above formula, $R^6$ is a $C_1$-$C_4$ alkyl group, a phenyl group which may be substituted, a benzyl group or a naphthyl group) is formed. The optically active sulfonic acid ester derivative (XII) is a compound having the same configuration as the starting material, optically active 1-arylethylalcohol derivative (XI), and can produce the configuration-inverted 1-arylethyl halide derivative (VIII) by reacting with a hydrohalogenic acid salt after its isolation or without isolation. In consequence, the 1-arylethyl halide derivative (VIII) can also be obtained from the optically active sulfonic acid ester derivative (XII) which has the opposite configuration of interest, after synthesizing the latter from a starting material other than the 1-arylethylalcohol derivative (XI).

The term hydrohalogenic acid salt as used herein means an addition product of a hydrogen halide, formed when the aforementioned optically active 1-arylethylalcohol derivative (XI) is treated with a sulfonyl halide in the presence of a base, and said base, so that it includes a hydrohalogenate of an organic base and a halide of an alkali metal or alkaline earth metal as well.

The optically active sulfonic acid ester derivative of general formula (XII) is a novel compound in which Ar, y and n are as defined in the general formula (I), and $R^6$ is a $C_1$-$C_4$ alkyl group such as methyl, ethyl, n-propyl, n-butyl or the like; an optionally substituted phenyl group such as phenyl, p-tolyl, p-nitrophenyl or the like; a benzyl group; or an $\alpha$- or $\beta$-naphthyl group. As the intermediate of the present invention, preferred of these is a methyl group, an ethyl group or a p-tolyl group.

The optically active 1-arylethylalcohol derivative (XI) as the starting material of the optically active 1-arylethyl halide derivative (VIII) can be produced by the method represented by the following scheme-7.

<Scheme-7>

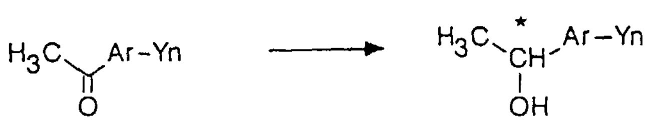

(XIII) (XI)

In the above reaction scheme, Ar, Y and n are as defined in the general formula (I), but, preferably, Ar is an aryl group, Y is a $C_1$-$C_4$ haloalkyl group such as triflurometbyl, difluoromethyl, trichloromethyl, dichlorodifluoroethyl or the like, and n is 1.

The optically active 1-arylethylalcohol derivative (XI) can be produced efficiently by subjecting the acetophenone derivative (XIII) to hydrogen transfer type asymmetric reduction in the presence of a transition metal complex, a base, an optically active nitrogen-containing compound and a hydrogen donor.

This reaction is carried out in accordance with the method described in an unexamined published Japanese patent application (*kokai*) No. 9-157196, but, when Ar is an aryl group, Y is a $C_1$-$C_4$ haloalkyl group and n is 1 in the acetophenone derivative of general formula (XIII), the asymmetric reduction can be carried out with good efficiency even if the acetophenone derivative as the substrate is used in a high concentration.

As the transition metal complex to be used in the present invention, various types of transition metal complex can be used, but it is preferably a complex compound represented by the following general formula (XVI).

$$[RuCl_2(arene)] \qquad (XVI)$$

In the above formula, arene is a benzene derivative which may be substituted with a $C_1$-$C_6$ alkyl group such as methyl, ethyl, n-propyl, iso-propyl or the like. Cymene can be exemplified as a preferred arene.

The transition metal complex may be used in an amount of from about 0.00001 to 0.01 mol, preferably from 0.0002 to 0.002 mol, based on 1 mol of the carbonyl compound as the reaction substrate, though it may vary depending on the size and system of the reaction vessel and economic situation.

Examples of the base to be used in this method include alkali metal hydroxide such as sodium hydroxide, potassium hydroxide, lithium hydroxide or the like; alkaline earth metal hydroxide such as calcium hydroxide, barium hydroxide or the like; alkali metal bicarbonate such as sodium bicarbonate or the like; alkali metal carbonate such as lithium carbonate, potassium carbonate, sodium carbonate or the like; alkaline earth metal carbonate such as calcium carbonate or the like; alkali metal carboxylate such as sodium acetate, potassium acetate or the like; alkaline earth metal carboxylate such as calcium acetate or the like; and alcoholate such as potassium methoxide, potassium iso-propoxide, potassium t-butoxide, lithium methoxide, lithium ethoxide, lithium t-butoxide, sodium t-butoxide or the like. Preferred of these is an alkali metal hydroxide.

These bases may be used in an amount of from about 0.5 to 50 equivalents, preferably from 2 to 10 equivalents, based on the transition metal complex.

Preferred example of the optically active nitrogen-containing compound according to the present invention is a 1,2-diphenylethylenediamine monosulfonamide derivative having an aromatic sulfonyl group such as benzenesulfonyl, p-toluenesulfonyl or the like.

The optically active nitrogen-containing compound may be used in an amount of from about 0.5 to 20 equivalents, preferably from 1 to 4 equivalents, based on the transition metal complex.

The hydrogen donor to be used in the present invention is a compound which can donate hydrogen by a thermal action or a catalytic action, and, though not particularly limited, preferred examples of such a hydrogen donating compound include alcohols such as methanol, ethanol, n-propanol, isopropanol, n-butanol, benzylalcohol and the like; formic acid and its metal salts or ammonium salts; azeotropic mixture of formic acid and an amine; unsaturated hydrocarbon or heterocyclic compound having partial saturated carbon bonding such as tetralin, dacalin and the like; hydroquinone; phosphorous acid; and hydrazine. Among these hydrogen donors, alcohols and formic acid are preferred and isopropanol and formic acid are particularly preferred.

It is desirable to use the hydrogen donor in an excess amount based on the acetophenone derivative (XIII), and is generally from 2 to 1,000 equivalents, preferably from 10 to 100 equivalents.

Examples of the solvent to be used include aromatic hydrocarbons such as toluene, xylene and the like; halogenated hydrocarbons such as dichloromethane and the like; and polar solvents such as acetonitrile, dimethyl sulfoxide, N,N-dimethylformamide and the like. However, these organic solvents may not be used when the hydrogen donor is a liquid, because the liquid itself is used as the reaction solvent.

The substrate concentration is generally from 0.01 to 2 mol/L, and the acetophenone derivative (XIII) when Ar is an aryl group, Y is a $C_1$-$C_4$ haloalkyl group and n is 1 is desirable from the industrial point of view, because the reaction progresses smoothly even at a high concentration of 0.2 mol/L or more.

This method of the present invention is carried out by allowing the acetophenone derivative represented by the general formula (XIII) to contact with a transition metal complex, a base, an optically active nitrogen-containing compound and a hydrogen donor. Consequently, an asymmetric complex catalyst is prepared prior to the commencement of the reaction using the transition metal complex and the optically active nitrogen-containing compound in the following manner.

That is, a transition metal complex, an optically active nitrogen-containing compound and a base are added to a solvent such as an alcohol or the like, and the mixture is stirred under heating in an atmosphere of an inert gas. The reaction solution may be used as it is, but a solid asymmetric complex catalyst may be obtained as occasion demands by cooling the solution, evaporating the solvent under a reduced pressure and then subjecting the residue to recrystallization.

By taking economic situation into consideration, the reaction is carried out at a temperature of from -20 to 100°C, preferably from 0 to 50°C, more preferably from 25 to 40°C. The reaction time varies depending on the reaction substrate, and the conditions such as the concentration, temperature, pressure and the like, but the reaction completes within the range of from several minutes to 100 hours.

According to this method of the present invention, when alcohols are used as the hydrogen donor, it is desirable to carry out the reaction simultaneously removing a carbonyl compound formed by dehydrogenation of the alcohols, because the amount of hydrogen donor to be added to the reaction system can be reduced and optical purity of the thus obtained 1-arylethylalcohol derivative (XI) can be increased. Examples of the method for removing the aforementioned carbonyl compound include a method in which it is evaporated under ordinary pressure or a reduced pressure; a method in which it is removed together with a bubbling inert gas; and a method in which an adsorbent such as a porous substance is allowed to coexist. Preferably, it may be evaporated under a reduced pressure.

It is desirable to carry out the removal under such a pressure that the reaction temperature can be kept within the preferred range, illustratively under a pressure of from 10 to 760 mmHg, preferably from 30 to 200 mmHg.

As the removing apparatus, a reactor equipped with a heat exchanger and a solvent collector can be exemplified. Also, it is desirable to equip the reactor further with a distillation column having necessary number of theoretical plates. When the number of theoretical plates is small, the alcohols may also be removed together, but the reaction progresses smoothly when the amount of removed alcohols is supplemented as occasion demands.

Removal of the carbonyl compound can be carried out any time during the reaction, but it is desirable to carry out it intermittently plural times or continuously, because the reaction yield and optical yield can be improved so that the effect of the present invention can be improved.

After completion of the reaction, the reaction is terminated by adding water or an acid as occasion demands. Examples of the acid to be added include mineral acids such as hydrochloric acid, sulfuric acid, nitric acid and the like; and organic acids such as acetic acid, citric acid and the like.

The thus obtained compound of the present invention can be isolated and purified in the usual way by extraction, distillation, recrystallization, column chromatography and the like.

The acetophenone derivative (XIII) which is the starting material of the optically active 1-arylethylalcohol derivative (XI) shown in the scheme-7 can be produced by the method represented by the following scheme-8.

<Scheme-8>

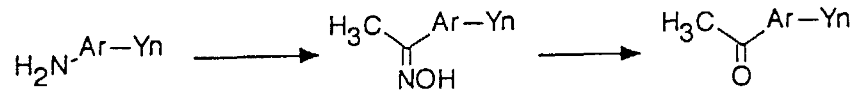

(XIV) (XV) (XIII)

In the above reaction scheme, Ar, Y and n are as defined in the general formula (I). Preferably, Ar is an aryl group, Y is a $C_1$-$C_4$ haloalkyl group such as trifluoromethyl, difluoromethyl, trichloromethyl, dichlorodifluoroethyl or the like, and n is 1.

The hydroxime derivative represented by the general formula (XV) is produced by allowing a diazonium salt obtained from an aniline derivative represented by the general formula (XIV) to react with acetaldoxime in an inert solvent in the presence of appropriate base and catalyst.

The diazonium salt of aniline derivative (XIV) can be obtained by a known method (*Org. Syn. Col.*, vol.III, p.296) or a modified method thereof. In general, it can be obtained by allowing aqueous solution of the aniline derivative (XIV) in its hydrochloride, sulfate, hydroborofluoride or the like to react with sodium nitrite at a temperature of 15°C or less. Hydrochloride or sulfate can be exemplified as a preferred salt, and preferred temperature is -10 to 10°C. It is necessary to allow the thus obtained diazonium salt aqueous solution to react with acetaldoxime immediately without its isolation and purification.

The hydroxime derivative (XV) can be obtained by adding the diazonium salt aqueous solution dropwise to an aqueous solution consisting of acetaldoxime, a base and a catalyst at a temperature of from -20 to 20°C, preferably from -10 to 10°C, and, after the dropwise addition, carrying out the reaction for a period of from 0.5 to 10 hours at a temperature of from -10 to 50°C, preferably from -5 to 30°C. When the dropwise addition is carried out by keeping the temperature at preferably from -10 to 10°C, more preferably from -10 to 5°C, the amount of by-products is reduced and the yield is increased as the result, so that it is economically advantageous.

Acetaldoxime may be used in an amount of from 1 to 10 equivalents, preferably from 1 to 5 equivalents, based on the aniline derivative represented by the aforementioned general formula (XIV).

The base to be used may be either an inorganic base or an organic base. Examples of the inorganic base include alkali metal hydroxide such as sodium hydroxide, potassium hydroxide or the like; alkali metal acetate such as sodium acetate, potassium acetate or the like; alkali metal or alkaline earth metal carbonate such as sodium carbonate, potassium carbonate, calcium carbonate or the like; alkali metal bicarbonate such as sodium bicarbonate, potassium bicarbonate or the like; alkali metal hydride such as sodium hydride or the like; and alkali metal such as metallic sodium or the like. Examples of the organic base include tertiary amine such as triethylamine or the like; aromatic base such as pyridine, picoline or the like; and alkali metal alcoholate such as sodium ethylate, t-butoxy potassium or the like. An alkali metal acetate can be exemplified as a preferred base.

The just described base may be used in an amount of from 1 to 50 equivalents, preferably from 1 to 10 equivalents, based on the aniline derivative represented by the aforementioned general formula (XIV).

In order to advance the reaction smoothly, sulfate (e.g., copper sulfate, sodium sulfate or the like) and sodium sulfite are used as the catalyst. Copper sulfate may be used as a preferred sulfate in an amount of from 0.0001 to 5

equivalents, preferably from 0.05 to 1 equivalent, more preferably from 0.1 to 0.35 equivalent, based on the aniline derivative represented by the general formula (XIV). When copper sulfate in an amount of 0.1 to 0.35 equivalent is used, the amount of by-products is reduced and the yield is increased as the result, so that it is economically advantageous. Sodium sulfite may be used in an amount of up to 1 equivalent, preferably up to 0.1 equivalent.

Examples of the solvent to be used include aromatic hydrocarbons such as benzene, toluene, xylene and the like; halogenated hydrocarbons such as dichloromethane, chloroform, 1,2-dichloroethane and the like; ethers such as diethyl ether, tetrahydrofuran, dioxane and the like; esters such as ethyl acetate and the like; ketones such as acetone, methyl ethyl ketone and the like; alcohols such as methanol, ethanol, propanol, butanol and the like; nitriles such as acetonitrile and the like; and polar solvents such as N,N-dimethylformamide, N-methylpyrrolidone, dimethyl sulfoxide, propionic acid, acetic acid, water and the like, which may be used alone or as a mixed solvent. Polar solvents such as acetic acid and water are preferable.

Though not particularly limited, the solvent may be used in an amount of from 1 to 100 times (by weight), preferably from 1 to 30 times, based on the substrate.

The acetophenone derivative represented by the general formula (XIII) is efficiently produced by hydrolyzing the hydroxime derivative (XV) obtained by the aforementioned hydroxime formation reaction, after its isolation or without isolation in an inert solvent using an appropriate acid or base.

When it is necessary to isolate the derivative after the hydroxime formation reaction, its isolation and purification can be effected in the usual way by extracting it with an organic solvent, concentrating the extract after its washing with optional acid and base and then subjecting the resulting residue for example to recrystallization or a column chromatography.

When the hydrolysis is carried out without isolation, it can be effected by adding an appropriate acid or base and further carrying out the reaction. This case is simple in handling and economically advantageous. It is possible also to subject a solution extracted with an organic solvent after completion of the hydroxime formation reaction to hydrolysis with an acid or base without concentrating the extract. In that case, it sometimes becomes a two-phase reaction, but the reaction progresses smoothly, so that the handling is simple and easy in comparison with the case in which the hydrolysis is carried out after isolation and purification, and the substrate concentration can be set at a higher level than the case of the method in which the hydrolysis is carried out without extraction, which is economically advantageous.

In carrying out the hydrolysis, the reaction may be carried out by adding an acid or base to the hydroxime derivative (XV) or a mixed solution containing the same, but, in order to complete the reaction within a short period of time with a high yield, it is desirable to add the hydroxime derivative (XV) or a mixed solution containing the same to a solution of an acid or base.

The reaction can be carried out at a temperature of from room temperature to boiling point of the solvent to be used, preferably from 20 to 120°C, and is completed generally within a period of from 0.5 to 5 hours.

The acid or base to be used in the hydrolysis may be used in an amount of from 1 to 10 equivalents, preferably from 1 to 5 equivalents, based on the hydroxime derivative (XV).

It is desirable to use an acid, in order to complete the reaction within a short period of time with a high yield.

Examples of the acid to be used include organic acids such as acetic acid, trifluoroacetic acid and the like; hydrohalogenic acids such as hydrochloric acid and the like; hydrogen halides such as hydrogen chloride and the like; sulfonic acids such as methanesulfonic acid, p-toluenesulfonic acid, sulfuric acid and the like; acid addition salts of organic bases such as pyridine hydrochloride, pyridine sulfate and the like; and Lewis acids such as zinc chloride, iron chloride, aluminum chloride and the like. Preferred of these are hydrohalogenic acids or sulfonic acids.

Examples of the base to be used include alkali metal hydroxide such as sodium hydroxide, potassium hydroxide or the like; alkali metal or alkaline earth metal carbonate such as sodium carbonate, potassium carbonate, calcium carbonate or the like; sodium bicarbonate, potassium bicarbonate or the like alkali metal bicarbonate; and alcoholate such as sodium ethylate, t-butoxy potassium or the like alkali metal. Preferred of these is an alkali metal hydroxide.

As the solvent to be used, the same solvents exemplified in the aforementioned hydroxime formation reaction can be used. Preferred of these solvents are hydrocarbons, aromatic hydrocarbons, ethers, alcohols, polar solvents such as water and mixed solvents thereof.

Though not particularly limited, the solvent may be used in an amount of from 1 to 100 times (by weight), preferably from 1 to 30 times, based on the substrate.

Each member of the thus obtained optically active methoxyiminoacetamide derivative (I) of the present invention is a novel compound and possessed of a markedly excellent activity as agricultural chemicals, namely not only superior fungicidal and insecticidal activities but also, to our surprise, an effect to exert little influences upon organisms that are not to be controlled.

For example, it shows exceedingly higher fungicidal activities than racemates upon plant diseases such as rice blast (*Pyricularia oryzae*); rice sheath blight (*Rhizoctonia solani*); wheat and barley powdery mildew (*Erysiphe graminis* f. sp. *tritici, E. graminis* f. sp. *hodei*); various rusts of wheat and barley (for example, *Puccinia recondita*); gray mold of vegetables and fruits (*Botrytis cinerea*); and late blight of various crops (*Phytophthora infestance*), while it also has a

characteristic feature in that it shows rather little influence upon living things, including mammals, which are not subjects of the control, so that it is useful as agricultural chemicals.

In using the compound (I) of the present invention as an agricultural chemical, the compound may be used as it is, but it is desirable to use it as a composition to which an agricultural auxiliary substance generally used in a formulation field is added. The formulation type is not particularly limited, but it is desirable to use it, for example, as emulsifiable concentrate, wettable powders, dust, floables, granules, tablets, oil solutions, propellents, aerosols and the like.

The agricultural auxiliary substance is used for the purpose, for example, of improving effect, stability and dispersibility of the composition, and is generally made up of a carrier (diluent) such as a liquid carrier or a solid carrier; and a surfactant, which varies depending on formulation type.

Examples of the liquid carrier include water; aromatic hydrocarbons such as alkylbenzenes (e.g., toluene, xylene and the like), alkylnaphthalenes (e.g., methylnaphthalene, dimethylnaphthalene and the like), chlorobenzenes and the like; alcohols such as methyl alcohol, ethyl alcohol, isopropyl alcohol, n-butyl alcohol, benzyl alcohol and the like; halogenated hydrocarbons such as ethylene chloride, methylene chloride, chloroform, tetrachloromethane and the like; ketones such as acetone, methylethylketone, cyclohexanone, methylbutylketone and the like; ethers such as ethyl ether, ethylene oxide, dioxane, and the like; esters such as ethyl acetate, amyl acetate, γ-butyrolactone, ethylene glycol acetate, and the like; nitriles such as acetonitrile, acrylonitrile and the like; amides such as N,N-dimethylformamide, N,N-dimethylacetamide, N-methylpyrrolidone and the like; sulfoxides such as dimethyl sulfoxide and the like; alcohol ethers such as ethylene glycol monomethyl ether; aliphatic or alicyclic hydrocarbons such as n-hexane, cyclohexane and the like; industrial gasolines such as petroleum ether, solvent naphtha and the like; petroleum distillates such as paraffins, kerosene, gas oil and the like; animal and plant oils; and fatty acids.

As the solid carrier, hard mineral powders such as clay, kaolin, talc, diatomaceous earth, silica, calcium carbonate, montmorillonite, bentonite, feldspar, quartz and the like; plant origin powders such as starch, crystalline cellulose, wheat flour and the like; silicates, saccharide polymers, alumina, highly dispersed silicic acid, waxes, gum arabic and the like can be used.

For the formulation of emulsifiable concentrate, wettable powders and floables, surfactants (or emulsifiers) are used for purpose of emulsifying, dispersing, solubilizing, wetting, foaming, lubricating, spreading, etc. Such emulsifiers include, for example, nonionic surfactants such as polyoxyethylene alkyl ethers, polyoxyethylene alkyl aryl ethers, polyoxyethylene alkyl esters, polyoxyethylene caster oil ether, polyoxyethylene sorbitan alkyl esters, sorbitan alkyl esters, carboxymethylcellulose, polyvinylalcohol, organic silicons (e.g., trisiloxane alcoxylate), and the like; anionic surfactants such as alkylbenzenesulfonates, alkylsulfosuccinates, alkylsulfates, polyoxyethylene alkylsulfates, arylsulfonates, sodium lignosulfonate, sodium lauryl sulfate, and the like; and cationic surfactants such as alkyl ammonium salts (e.g., cationic surfactant alkyldimethylbenzyl ammonium chloride), and the like may be used alone or as a mixture of plural surfactants depending on the purpose.

Use of surfactants has various effects such as sticking effect of carboxymethylcellulose, polyvinyl alcohol and the like; disintegration effect of sodium lignosulfonate, sodium lauryl sulfate and the like; spreading effect of sodium higher alcohol sulfate, stearyltrimethylammonium chloride, polyoxyethylene laurylphenyl ether and the like; wet-spreading effect of dialkyl sulfosuccinate and the like; and sticking effect of carboxymethylcellulose, polyvinyl alcohol and the like.

In the case of using optically active methoxyiminoaceamide derivative (I) of the present invention for agricultural chemicals, when an organosilicon surfactant such as trisiloxane alcoxylate (e.g., "Silwet"-77) is mixed in a formulation or added by means of tankmix, a spreading property of an active ingredient should be improved so that a biological activity can markedly go up. This is economically advantageous. These organosilicon surfactant mixed in a formulation may be used in an amount of 0.1 times by weight or more based on active ingredient, practically preferred from 0.1 to 10 times, more preferably from 0.5 to 2.5 times by weight.

The amount of active ingredient to be contained in the agricultural chemical preparation of the present invention is selected within the range of from 0.1 to 99.5% and optionally decided depending on various conditions such as preparation forms, application methods and the like, which, for example, is about 0.5 to 20% by weight, preferably 1 to 10% by weight, in the case of dust, about 1 to 90% by weight, preferably 10 to 80% by weight, in the case of wettable powders, and about 1 to 90% by weight, preferably 10 to 40% by weight, in the case of emulsifiable concentrate.

For example, in the case of an emulsifiable concentrate in the use of the formulated agricultural chemical of the present invention, it is prepared by mixing the compound of the present invention to be used as the active ingredient with a solvent and a surfactant and the like to prepare a stock solution, and is generally applied by diluting the stock solution with water to a predetermined concentration when used. In the case of a wettable powder, it is prepared by mixing the active ingredient with a solid carrier and a surfactant and the like, and is generally applied by diluting the stock solution with water to a predetermined concentration when used. In the case of a dust, the active ingredient is mixed with a solid carrier and the like and generally applied as such when used. In the case of a granule preparation, a mixture consisting of the active ingredient, a solid carrier and a surface active agent and the like is made into granules and generally applied as such when used. As a matter of course, the formulation methods of the aforementioned respective formulation types are not limited thereto, and can be selected at will by those skilled in the art depending on the types of the active ingredient, application purposes and the like.

Application method of the agricultural chemical of the present invention is not particularly limited, and it can be applied by any one of the foliar application, submerged application, soil application, seed treatment and the like. For example, in the case of foliar application, an aqueous solution within the concentration range of from 5 to 1,000 ppm, preferably from 10 to 500 ppm, may be applied in an amount of from 50 to 500 liters per 10 ares. In the case of submerged application, the application amount is generally from 1 to 10 kg per 10 ares, as granules containing 5 to 15% of the active ingredient. In the case of soil application, an aqueous solution within the concentration range of from 5 to 1,000 ppm, preferably from 10 to 500 ppm, may be applied in an amount of from 1 to 10 liters per 1 $m^2$. In the case of seed treatment, an aqueous solution within the concentration range of from 10 to 1,000 ppm may be applied in an amount of from 10 to 100 liters per 1 kg of seed weight.

The agricultural chemical of the present invention can be used by mixing it with other active components such as a fungicide, an insecticide, an acaricide and the like, provided that they do not spoil the fungicidal and insecticidal effects of the active ingredient of the present invention. When the derivative of the present invention is combined with an appropriate known fungicidal, insecticidal or acaricidal agent, mutual complement of the controlling spectrum becomes possible, so that the number of total application times can be reduced and, as the result, considerable effect can be exerted in terms of the reduction of total amount to be used. In addition, combination of the invention derivative with known fungicidal, insecticidal or acaricidal agent having different actions is effective in inhibiting or delaying development of resistance which is probable when each component is used singly. Also, in some cases, improvement of the activity due to synergistic effect can be obtained as will be described later in Examples, so that such a combined use is economically advantageous.

When the aforementioned mixture preparation is produced, an active ingredient in which the compound (I) of the present invention is blended with at least one of known fungicides, insecticides or acaricides is mixed with an appropriate carrier and auxiliary agent such as an emulsifying agent, a dispersing agent, a stabilizing agent, a suspending agent, a penetrating agent or the like, and the mixture is made into wettable powders, water-soluble powders, emulsifiable concentrate, liquid formulations, sols (floables), oil solutions, dust, granules, aerosols and the like. The carrier to be used may be either solid or liquid with no particular limitation, provided that it is usually used in agricultural chemicals. Also, though not particularly limited, a surfactant (or emulsifying agent) may be used in preparing formulations such as emulsifiable concentrate, wettable powders, sols and the like, in order to obtain the effects such as emulsification, dispersion, solubilization, wetting, foaming, lubrication, spreading and the like. In addition to these components, various auxiliaries and, as occasion demands, stabilizing agents (e.g., antioxidants, ultraviolet ray absorbents and the like) and coloring agents may be used.

The percentage content (%) of the active ingredient of the present invention in these preparations may be within the range of from 1 to 90% (% by weight, the same shall apply hereinafter) in the case of wettable powders, water-soluble powders, emulsifiable concentrate, liquid formulations and sols, within the range of from 0.5 to 10% in the case of oil solutions, dust and granules, or within the range of from 0.01 to 2% in the case of aerosols.

With regard to the mixing ratio of the compound (I) of the present invention and other fungicidal, insecticidal or acaricidal component, the latter may be blended in an amount of generally from 0.01 to 99% by weight, preferably from 0.1 to 20% by weight, based on 1% by weight of the former. These preparations can be used in various purposes by diluting them to an appropriate concentration and subjecting them to foliar application, seed treatment, soli application, submerged application or direct application.

Though not particularly limited, illustrative examples of the fungicide which can be used by mixing it with the compound of the present invention include (2RS,3SR)-1-[3-(2-chlorophenyl)-2,3-epoxy-2-(4-fluorophenyl)propyl]-1H-1,2,4-triazole, 1-(biphenyl-4-yloxy)-3,3-dimethyl-1-(1H-1,2,4-triazol-1-yl)butan-2-ol, 1-[(2RS,4RS:2RS,4SR)-4-bromo-2-(2,4-dichlorophenyl)tetrahydrofurfuryl]-1H-1,2,4-triazole, bis(4-fluorophenyl)(methyl)(1H-1,2,4-triazol-1-ylmethyl)silane, (2RS,3RS;2RS,3SR)-2-(4-chlorophenyl)-3-cyclopropyl-1-(1H-1,2,4-triazol-1-yl)butan-2-ol, cis,trans-3-chloro-4-[4-methyl-2-(1H-1,2,4-triazol-1-ylmethyl)-1,3-dioxiran-2-yl)phenyl 4-chlorophenyl ether, 4-(4-chlorophenyl)-2-phenyl-2-(1H-1,2,4-triazol-1-ylmethyl)butyronitrile, 3-(2,4-dichlorophenyl)-6-fluoro-2-(1H-1,2,4-triazol-1-yl)quinazolin-4(3H)-one, (RS)-2-(2,4-dichlorophenyl)-1-(1H-1,2,4-triazol-1-yl)hexan-2-ol, (1RS,5RS;1RS,5SR)-5-(4-chlorobenzyl)-2,2-dimethyl-1-(1H-1,2,4-triazol-1-ylmethyl)cyclopentanol, 2-p-chlorophenyl-2-(1H-1,2,4-triazol-1-ylmethyl)hexanenitrile, (±)-1-[2-(2,4-dichlorophenyl)-4-propyl-1,3-dioxiran-2-ylmethyl]-1H-1,2,4-triazole, (RS)-1-p-chlorophenyl-4,4-dimethyl-3-(1H)-1,2,4-triazol-1-ylmethyl)pentan-3-ol, (RS)-2-(2,4-dichlorophenyl)-3-(1H-1,2,4-triazol-1-yl)propyl 1,1,2,2-tetrafluoroethyl ether, 1-(4-chlorophenoxy)-3,3-dimethyl-1-(1H-1,2,4-triazol-1-yl)bunan-2-one, (1RS,2RS;1RS,2SR)-1-(4-chlorophenoxy)-3,3-dimethyl-1-(1H-1,2,4-triazol-1-yl)butan-2-ol and such triazole compounds; (E)-4-chloro-α,α,α-trifluoro-N-(1-imidazol-1-yl-2-propoxyethylidene)-o-toluidine, N-propyl-N-[2-(2,4,6-trichlorophenoxy)ethyl]imidazole-1-carboxamide, (±)-1-(β-allyloxy-2,4-dichlorophenylethyl)imidazole and such imidazole compounds; (±)-2,4-dichloro-α-(pyrimidin-5-yl)benzhydrylalcohol, (±)-2-chloro-4'-fluoro-α-(pyrimidin-5-yl)benzhydrylalcohol and such pyrimidine compounds; (±)-cis-4-[3-(4-tert-butylphenyl)-2-methylpropyl]-2,6-dimethylmorpholine, 2,6-dimethyl-4-tridecylmorpholine,

(RS)-1-[3-(4-tert-butylphenyl)-2-methylpropyl]piperidine and such morpholine compounds and morpholine derivatives; methyl 1-(butylcarbamoyl)benzimidazol-2-ylcarbamate, dimethyl 4,4'-(o-phenylene)bis(3-thioallophanate), methyl benzimidazol-2-ylcarbamate, 2-(thiazol-4-yl)benzimidazole and such benzimidazole compounds; N-(3,5-dichlorophenyl)-1,2-dimethylcyclopropane-1,2-dicarboxyimide, 3-(3,5-dichlorophenyl)-N-isopropyl-2,4-dioxoimidazolidine-1-carboxamide, (RS)-3-(3,5-dichlorophenyl)-5-methyl-5-vinyl-1,3-dioxazolidine-2,4-dione and such dicarboxyimide compounds; methyl N-(2-methoxyacetyl)-N-(2,6-xylyl)-DL-alaninate, 2-methoxy-N-(2-oxo-1,3-oxazolidin-3-yl)-aceto-2',6'-xylidate and such acylalanine compounds; O-ethyl S,S-diphenyl phosphorodithioate, S-benzyl O,O-diisopropylphosphorothioate and such organic phosphorus compounds; 3'-isopropoxy-o-tolanilide, α,α,α-trifluoro-3'-isopropoxy-o-tolanilide, 5,6-dihydro-2-methyl-1,4-oxath-ine-3-carboxanilide 4,4-dioxide and such phenylamide compounds; zinc ion coordination manganese ethylenebisdithiocarbamate, manganese ethylenebisdithiocarbamate, zinc ion coordination ethylenebisdithiocarbamate, zinc ion coordination bisdimethyldithiocarbamate and such dithiocarbamate compounds; N-(4-methyl-6-propyn-1-ylpyrimidin-2-yl)aniline, N-(4,6-dimethylpyrimidin-2-yl)aniline, 4-cyclopropyl-6-methyl-N-phenylpyrimidine-2-amine and such anilinopyrimidine compounds; and methylmethoxyimino-α-(o-tolyloxy)-o-tolylacetate, methyl(E)-{2-[6-(2-cyanophenoxy)pyrimidin-4-yloxy]phenyl}-3-methoxyacrylate and such strobilurin derivatives; as well as S,S-(6-methylquinoxaline-2,3-diyl)dithiocarbonate, 3-chloro-N-(3-chloro-5-trifluoromethyl-2-pyridyl)-α,α,α-trifluoro-2,6-dinitro-p-toluidine, tetrachloroisophthalonitrile, N-dichlorofluoromethylthio-N,N'-dimethyl-N-phenylsulfamide, 1-(2-cyano-2-methoxyiminoacetyl)-3-ethylurea, aluminumtris(ethyl phosphonate), 2,3-dichloro-N-fluorophenylmaleimide, 5,10-dihydro-5,10-dioxonaphtho[2.3-b]-1,4-dith-ine-2,3-dicarbonitrile, (E,Z)-4-[3-(4-chlorophenyl)-3-(3,4-dimethoxyphenyl)acryloyl]morpholine, 4-(2,2-difluoro-1,3-benzodioxol-4-yl)-1H-pyrrole-3-carbonitrile, 1,1'-iminodi(octamethylene)diguanidine, 4,5,6,7-tetrachlorofusaride, 3-allyloxy-1,2-benz[d]isothiazole 1,1-dioxide, 5-methyl-1,2,4-triazolo[3,4-b][1,3]benzothiazole, 1,2,5,6-tetrahydropyrrolo[3,2,1-ij]quinolin-4-one, di-isopropyl 1,3-dithiolan-2-ylidenemalonate, isopropyl 3,4-diethoxycarbanylate, various antibiotics and the like.

Illustrative examples of other insecticide components include, though not particularly limited, dimethyl-2,2,2-trichloro-1-hydroxyethyl phosphonate, O,O-diethyl O-2-isopropyl-6-methylpyrimidin-4-yl phosphorothioate, 2,2-dichlorovinyldimethyl phosphate, dimethyl-2,2,2-trichloro-1-hydroxyethyl phosphonate and such organic phosphorus insecticides; 2-sec-butylphenylmethylcarbamate, 1-naphthylmethylcarbamate, 2-dimethylamino-5,6-dimethylpyrimidin-4-yldimethylcarbamate and such carbamate insecticides; (RS)-α-cyano-3-phenoxybenzyl-N-(2-chrolo-α,α,α-trifluoro-p-tolyl)-D-valinate, 2-(4-ethoxyphenyl)-2-methylpropyl 3-phenoxybenzyl ester, (RS)-α-cyano-3-phenoxybenzyl(1RS,3RS;1RS,3SR)-3-(2,2-dichlorovinyl)-2,2-dimethylcyclopropane carboxylate and such pyrethroid insecticides; and 1-(3,5-dichloro-2,4-difluorophenyl)-3-(2,6-difluorobenzoyl)urea, 1-(4-chlorophenyl)-3-(2,6-difluorobenzoyl)urea, 1-[3,5-dichloro-4-(3-chloro-5-trifluoromethyl)-2-pyridyloxy)phenyl]-3-(2,6-difluorobenzoyl)urea and such benzoylurea insecticides; as well as 4-bromo-2-(4-chlorophenyl)-1-ethoxymethyl-5-trifluoromethylpyrrole-3-carbonitrile, 1-(6-chloro-3-pyridylmethyl)-N-nitroimidazolidin-2-ylideneamine, N-tert-butyl-N'-(4-ethylbenzoyl)-3,5-dimethylbenzohydrazide, 1-tert-butyl-3-(2,6-di-isopropyl-4-phenoxyphenyl)thiourea, S,S'-(2-dimethylaminotrimethylene)bis(thiocarbamate), various antibiotics and the like. Illustrative examples of known acaricide components include a great variety of compounds such as, though not particularly limited, N-(4-tert-butylbenzyl)-4-chloro-3-ethyl-1-methylpyrrazole-5-carboxamide, 2-tert-butyl-5-(4-tert-butylbenzylthio)-4-chloropyridazin-3(2H)-one, tert-butyl (E)-α-(1,3-dimethyl-5-phenoxypyrazol-4-ylmethylene aminooxy)-p-toluate, 2,2,2-trichloro-1,1-bis(4-chlorophenyl)ethanol, 2-(4-tert-butylphenoxy)cyclohexylprop-2-ynylsulfite, N-methylbis(2,4-xylyliminomethyl)amine, (4RS,5RS)-5-(4-chlorophenyl)-N-cyclohexyl-4-methyl-2-oxo-1,3-thiazolidine-3-carboxamide, 3,6-bis(2-chlorophenyl)-1,2,4,5-tetrazine and the like.

The thus mixed and obtained agricultural chemical components can be used broadly and effectively as a fungicidal agent, a fungicidal and insecticidal agent or a fungicidal and acaricidal agent. For example, the fungicidal agent is effective in controlling a broad range of diseases caused by various phytopathogenic fungi belonging to Ascomycetes, Basidiomycete, Oomycete and inperfent fungi which generate on a variety of crops, vegetables, fruit trees and flowers and ornamental plants, examples of such diseases including wheat powdery mildew (*Erysiphe graminis* f. sp. *tritici*), brown rust (*Puccinia recondita*), leaf blotch (*Septoria tritici*), glume blotch (*Septoria nodorum*), eye spot (*Pseudocercosporella herpotrichoides*), fusarium blight (*Gibberella zeae*), snow mold (*Fusarium nivale*) and the like; barley powderly mildew (*Erysiphe graminis* f. sp. *hordei*), brown rust (*Puccinia hordei*), scald (*Rhynchosporium secalis*), net blotch (*Pyrenophora teres*) and the like; rice blast (*Pyricularia oryzae*), sheath blight (*Rhizoctonia solani*), helminthosporium leaf spot (*Cochliobolus miyabeanus*), bakanae disease (*Gibberella fujikuroi*) and the like; kidney bean gray mold (*Botrytis cinerea*), stem rot (*Sclerotinia sclerotiorum*) and the like; potato late blight (*Phytophthora infestans*), early blight (*Alternaria solani*) and the like, sugar beet cercospora leaf spot (*Cercospora viticola*) and the like; cucumber powdery mildew (*Sphaerotheca fuliginea*), anthracnose (*Colletotrichum lagenarium*), scab (*Cladosporium cucumerinum*), gray mold (*Botrytis cinerea*), downy mildew (*Pseudoperonospora cubensis*) and the like; apple scab (*Venturia inaequalis*), alternaria leaf spot (*Alternaria mali*), blossom blight (*Monilinia mali*) and the like; pear scab (*Venturia nashicola*), black spot (*Alternaria kikuchiana*) and the like; peach brown spot (*Monilinia fructicola*), scab (*Cladosporium carpophilum*) and the like; grapes downy mildew (*Plasmopara viticola*), leaf spot (*Pseudocercospora vitis*), gray mold (*Botrytis cinerea*) and the like; persimons angular leaf spot and circular leaf spot (*Cercospora kaki,*

*Mycosphaerella nawae*) and the like; and tea anthracnose (*Colletotrichum theae-sinensis*), gray blight (*Pestalotia theae, Pestalotia longiseta*), blister blight (*Exobasidium vexans*) and the like. Also, in addition to the control of just described various plant diseases, the fungicidal and insecticidal agent and the fungicidal and acaricidal agent can be used in controlling a wide variety of insect pests and plant-parasitic acarids, such as diamond back moth (*Plutella xylostella*), common cabbage worm (*Pieris rapae crucuvora*), apple leafminer (*Phyllonorycter ringoniella*), peach fruit moth (*Carposina niponensis*), smaller tea tortrix (*Adoxophes* sp.), tea tussock moth (*Euproctis pseudoconspersa*), common cutworm (*Spodoptera litura*), a leafminer (*Lyonetia prunifoliella malinella*), persimon fruit moth (*Stathmopoda masinissa*) and such insects belonging to Lepidoptera; green peach aphid (*Myzus persicae*), cotton aphid (*Aphis gossypii*), greenhouse whitefly (*Trialeurodes vaporariorum*), a stink bug (*Coptosoma punctatissimun*), green stink bug and bean bug (*Nezara* spp., *Riptortus clavatus* and the like), comstock mealy bug (*Pseudococcus comstocki*), foxglove aphid (*Aulacorthum solani*), grape leafhopper (*Arboridia apicalis*), tea green leafhopper (*Empoasca onukii*), brown rice planthopper (*Nilaparvata lugens*) and such insects belonging to Hemiptera; Japanese beetle (*Popillia japonia*), cupreous chafer (*Anomala cuprea*), twenty-eight-spotted ladybird (*Henosepilachna* spp.), cucurbit leaf beetle (*Aulacophora femoralis*), adzuki bean weevil (*Callosobruchus chinensis*) and such insects belonging to Coleoptera; stone leek leafminer (*Liriomyza chinensis*), rice leafminer (*Agromyza oryzae*) and such insects belonging to Diptera; and onion thrips (*Thrips tabaci*), yellow tea thrips (*Scirtothrips dorsalis*), a thrips (*Thrips palmi*) and such insects belonging to Thysanoptera, as well as two-spotted spider mite (*Tetranychus urticae*), carmine spider mite (*Tetranychus cinnabarinus*), kanzawa spider mite (*Tetranychus kanzawai*), European red mite (*Panonychus ulmi*), citrus red mite (*Panonychus citri*), broad mite (*Polyphagotarsonemus latus*) and such acarids belonging to Acarina.

Next, the present invention is described more illustratively with reference to the following example, but the present invention should not be construed as being limited to these examples.

In this connection, the optical purity of compounds used in the following examples and compounds obtained was determined by a high performance liquid chromatography {column: CHIRALCEL OJ manufactured by Daicel Chemical Industries, eluate: hexane-isopropyl alcohol (4:1 - 100:0), flow rate: 1.0 ml/min, detection: 220 nm}. However, optical purity of the 1-arylethoxyamine derivative (II) was determined under the above conditions after converting said compound into its cyclohexanoneoxime form by the reaction with cyclohexanone.

As references, Production Example 1 shows preparation of the optically active methoxyiminoacetamide (I), and Production Examples 2 to 4 shows preparation method of the phenylglyoxylic acid ester derivative (IV) which is the production raw material of the acetal derivative (III).

Production Example 1

Separation of optically active N-methyl-2-[2-{1-(3-trifluoromethylphenyl)ethoxyiminomethyl}phenyl]-2-methoxyiminoacetamide by a high performance liquid chromatography using an optical isomer separation column (compound No. 1 and No. 2)

Using a CHIRALCEL OJ column, N-methyl-2-[2-{1-(3-trifluoromethylphenyl)ethoxyiminomethyl}phenyl]-2-methoxyiminoacetamide (600 g) was separated into both enantiomers under a condition of hexane:isopropyl alcohol = 8:2 as the mobile phase. The first elution isomer (to be referred to as "first peak" hereinafter) was obtained in an amount of 234 g, and the second elution isomer (to be referred to as "second peak" hereinafter) in 242 g.

Results of the analysis of respective isomers are shown in the following table.

Analytical conditions;

| | |
|---|---|
| Column | CHIRALCEL OJ |
| Mobile phase | hexane:isopropyl alcohol = 8:2 |
| Flow rate | 1.0 ml/min |
| Temperature | 40°C |
| Wave length | 254 nm |

Results of analysis;

| Peak | Retention time (min) | Optical purity (% ee) | Specific angle of rotation $[\alpha]_D^{24.0}$ | Melting point (%) |
|---|---|---|---|---|
| First peak | 7.92 | >99.7 | -40.1° (C =1.0, $CHCl_3$) | 87.9 - 88.8 |
| Second peak | 11.62 | 99.7 | +42.2° (C =1.0, $CHCl_3$) | 87.8 - 89.6 |

Production Example 2

Synthesis of ethyl 2-(1,3-dioxan-2-yl)phenylglyoxylate

A 36.5 g (150 mmol) portion of 2-(1,3-dioxan-2-yl)bromobenzene was added dropwise to a mixture of 3.72 g (153 mmol) of magnesium, 0.03 g of iodine and 30 ml of tetrahydrofuran, while heating under reflux. After additional 2 hours of heating under reflux, this was cooled to room temperature, and the resulting brown solution was added dropwise to 90 ml of toluene solution containing 43.8 g (300 mmol) of diethyl oxalate, while keeping the inner temperature to -3 to 0°C. After completion of the dropwise addition, this was returned to room temperature, poured into 300 ml of saturated ammonium chloride aqueous solution and extracted with 200 ml of ethyl acetate. The organic layer was washed twice with 100 ml of saturated brine and dried over anhydrous sodium sulfate. The solvent was evaporated, 100 ml of hexane was added to thus precipitated crystals, and the mixture was stirred under ice-cooling and then filtered. The thus collected crystals were washed with 30 ml of cold hexane and air-dried to give 29.8 g of the title compound (III) (GC purity 93%). The yield was 70%.

Melting point; 104.9°C - 108.3°C
$^1$H-NMR δ ($CDCl_3$); 1.41 (3H, t), 1.3 - 1.6 (1H, m), 2.0 - 2.2 (1H, m), 3.91 (2H, t), 4.2 (2H, m), 4.39 (2H, q), 5.75 (1H, s), 7.43 (1H, dd), 7.5 - 7.6 (2H, m), 7.65 (1H, d)

Production Example 3

Synthesis of ethyl 2-(4,4,6-trimethyl-1,3-dioxan-2-yl)phenylglyoxylate

A 17.1 g (60 mmol) portion of 2-(4,4,6-trimethyl-1,3-dioxan-2-yl)bromobenzene was added dropwise to a mixture of 1.49 g (61.3 mmol) of magnesium, 0.02 g of iodine and 30 ml of tetrahydrofuran, while heating under reflux. After additional 1 hour of heating under reflux, this was cooled to room temperature, and the resulting brown solution was added dropwise to 30 ml of toluene solution containing 17.5 g (120 mmol) of diethyl oxalate, while keeping the inner temperature to -8 to 0°C. After completion of the dropwise addition, this was returned to room temperature, poured into 100 ml of saturated ammonium chloride aqueous solution and extracted with ethyl acetate. The organic layer was washed with saturated brine and dried over anhydrous sodium sulfate. After evaporation of the solvent, excess diethyl oxalate was evaporated under a reduced pressure to give 18.3 g of the title compound (GC purity 70%). The yield was 70%.

$^1$H-NMR δ ($CDCl_3$); 1.24 (3H, d), 1.30 (3H, s), 1.39 (3H, s), 1.40 (3H, t), 1.47 - 1.54 (2H, m), 1.48 (1H, d), 1.52 (1H, d), 4.0 (1H, m), 4.37 (2H, q), 6.01 (1H, s), 7.40 (1H, dd), 7.47 - 7.56 (2H, m), 7.71 (1H, d)

Production Example 4

Synthesis of ethyl 2-(1,3-dioxolan-2-yl)phenylglyoxylate (referred to as EGA)

A 100 g (0.540 mol) portion of o-bromobenzaldehyde, 40.2 g (0.648 mol) of ethylene glycol, 200 ml of toluene, and 1.0 g (0.0054 mol) of p-toluenesulfonic acid monohydrate were heated under reflux for 3 hours while effecting azeotropic dehydration by the Dean and Stark method. After cooling to 40°C, this was washed with 100 ml aqueous solution containing 0.343 g of sodium carbonate and 20 g of sodium chloride. This was again washed with 100 ml of aqueous solution containing 20 g of sodium chloride, and concentrated the toluene layer to give 123.19 g of 2-(1,3-dioxolan-2-yl)bromobenzene. The yield was 99% or more based on o-bromobenzaldehyde.

A few drops part of 100 g (0.436 mol) of 2-(1,3-dioxolan-2-yl)bromobenzene was added dropwise to 10.4 g (0.429 mol) of magnesium, 200 ml of tetrahydrofuran and 10 mg of iodine under nitrogen atmosphere, and the mixture was heated. The reflux was started, the oil bath was removed, and remaining 2-(1,3-dioxolan-2-yl)bromobenzene was added dropwise while refluxing gently. After completion of the dropwise addition, this was heated under reflux for 1 hour and then cooled to room temperature. The resulting Grignard reagent was added dropwise to 127.7 g (0.874 mol) of diethyl oxalate and 400 ml of toluene under an atmosphere of nitrogen spending 1 hour, while keeping the inner temperature at -5 to 5°C. After 1 hour of reaction at room temperature, this was added dropwise into a flask which has been charged with 50 g of concentrated hydrochloric acid and 420 g of water and cooled to 0°C, while keeping the inner temperature at 10°C or less. This was separated, and the organic layer was washed with 5% sodium bicarbonate aqueous solution and 20% brine, adjusted to pH 7 and then concentrated to give 173.80 g of an oily material.

Then, excess diethyl oxalate was recovered from 171.70 g of the oily material by distillation under a reduced pressure to give 108.53 g of crude title compound as a yellow oily residue. This was analyzed by an internal standard method and, as a result, it was found that 86.9 g (0.3473 mol) of the title compound was formed. The yield was 80% based on 2-(1,3-dioxolan-2-yl)bromobenzene.

$^1$H-NMR $\delta$ ($CDCl_3$); 1.39 (3H, t), 3.8 - 3.9 (2H, m), 3.9 - 4.0 (2H, m), 4.38 (2H, q), 6.25 (1H, s), 7.3 - 7.7 (4H, m)

Example 1-1

Synthesis of (R)-N-methyl-2-[2-{1-(3-trifluoromethylphenyl)ethoxyiminomethyl}phenyl]-2-methoxyiminoacetamide (compound No. 1)

A 52 g portion of lipase (Toyozyme • LIP, fixed on celite, manufactured by Toyobo) was added to 150 ml of diisopropyl ether mixed solution containing 50 g (0.263 mol) of 1-(3-trifluoromethylphenyl)ethanol and 113 g (1.31 mol) of vinyl acetate, and the mixture was stirred at room temperature for 12 hours. The reaction mixture was filtered, and the filtrate was concentrated under a reduced pressure. A 2 g portion of the resulting residue was purified by a silica gel column chromatography (silica gel 50 g, hexane:ethyl acetate = 10:1) to give 0.6 g (3.2 mmol) of optically active alcohol compound and 1.2 g (5.2 mmol) of acetyl compound.

Optical purity of the alcohol compound was 99.5% ee or more, and its specific angle of rotation was
$[\alpha]_D^{24.0}$ = -27.9° (C = 1.6396, MeOH)
$[\alpha]_D^{24.0}$ = -33.6° (C = 1.6696, $CHCl_3$). This result revealed that the resulting optically active alcohol compound has an absolute configuration of s {*Tetrahedron: Asymmetry*, Vol. 6, N0. 9, pp. 2385 - 2394, (1995) and *J. Am. Chem. Soc.*, 112, 5741 - 5747 (1990)}.

A 0.49 g (3.2 mmol) portion of N-hydroxylphthalimide and 0.79 g (3.2 mmol) of triphenylphosphine were added to 5 ml of ethyl acetate solution containing 0.6 g (3.2 mmol) of (S)-1-(3-trifluoromethylphenyl)ethanol. Under ice-cooling, 0.52 g (3.2 mmol) of diethyl azodicarboxylic acid was added dropwise to the mixture, and was stirred at room temperature for 1 hour.

The solvent was evaporated under a reduced pressure, diethyl ether was added to the residue to remove insoluble matter by filtration, and then the resulting filtrate was concentrated under a reduced pressure to give 0.92 g (2.7 mmol) of a crude phthalimide. To 0.92 g (2.7 mmol) of the crude phthalimide in 10 ml of ethanol solution was added 0.5 ml (10 mmol) of hydrazine monohydrate, followed by heating under reflux for 1 hour. After cooling, water was added, and the mixture was extracted with hexane. The resulting organic layer was washed with water and saturated brine and then dried over anhydrous sodium sulfate. After evaporation of the solvent under a reduced pressure, the resulting residue was purified by a silica gel column chromatography (silica gel 20 g, developing solvent = dichloromethane) to give 0.42 g (2.0 mmol) of (R)-1-(3-trifluoromethylphenyl)ethoxyamine.

Optical purity of this compound was 99.5% ee or more, and its specific angle of rotation was $[\alpha]_D^{22.0}$ = +60.8° (C = 0.98, $CHCl_3$).

A mixture of 0.59 g (2.0 mmol) of N-methyl-2-(2-diethoxymethylphenyl)-2-methoxyiminoacetamide, 0.42 g (2.0 mmol) of (R)-1-(3-trifluoromethylphenyl)ethoxyamine, 0.1 ml of water and 2 ml of acetic acid was stirred for 4 hours while heating at 60°C. The solvent was evaporated from the reaction mixture, water was added to the residue, and the mixture was extracted with ethyl acetate. The resulting organic layer was washed with water, saturated sodium bicarbonate aqueous solution and saturated brine in that order and then dried over anhydrous sodium sulfate. After evaporation of the solvent, the resulting residue was purified by a silica gel column chromatography (ethyl acetate/hexane = 1:1) to give 0.52 g of the title compound as white crystals (yield 85%). The optical purity was 99.5% ee.

This compound coincided with the second peak shown in Production Example 1 when analyzed by HPLC using an optically active column CHIRALCEL OJ.

Example 1-2

Synthesis of (S)-N-methyl-2-[2-{1-(3-trifluoromethylphenyl)ethoxyiminomethyl}phenyl]-2-methoxyiminoacetamide (compound No. 2)

A 15.39 g (75 mmol) portion of 1-(3-trifluoromethylphenyl)ethoxyamine and 11.26 g (75 mmol) of D-(-)-tartaric acid were dissolved under heating with a mixed solution of methanol/water = 2:1 (50 ml), and the resulting solution was gradually cooled to effect precipitation of crystals. The crystals were collected by filtration and again dissolved under heating with a mixed solution of methanol/water = 2:1 (25 ml), and the resulting solution was gradually cooled to effect precipitation of crystals. To 13.0 g (36.6 mmol) of the crystals collected by filtration was added 80 ml of 1 N sodium hydroxide aqueous solution, followed by extraction with 40 ml of hexane. The resulting organic layer was washed with saturated brine and dried over anhydrous sodium sulfate. By evaporating the solvent, 6.63 g (32.3 mmol) of (S)-1-(3-trifluoromethylphenyl)ethoxyamine was obtained. Optical purity of the compound was 96.9% ee. Its specific angle of rotation was $[\alpha]_D^{22.0}$ = -58.9° (C = 0.986, $CHCl_3$).

A 15 ml portion of acetic acid solution containing 7.5 g (32.3 mmol) of 1-methoxy-4-methoxyimino-2-methyl-3-oxo-1,2,3,4-tetrahydroisoquinoline, 6.63 g (32.3 mmol) of (S)-1-(3-trifluoromethylphenyl)ethoxyamine (to be referred to as SBOA hereinafter) and 0.25 g of methanesulfonic acid was stirred for 4 hours while heating at 80°C. The reaction mixture was poured into water and extracted with ethyl acetate. The resulting organic layer was washed with water and saturated brine in that order and then dried over anhydrous sodium sulfate. After evaporation of the solvent, the resulting residue was purified by a silica gel column chromatography (ethyl acetate/hexane = 1:2) to give 9.8 g of the title compound as white crystals (yield 74%). The optical purity was 96.0% ee.

This compound coincided with the first peak shown in Production Example 1 when analyzed by HPLC using an optically active column CHIRALCEL OJ.

Example 1-3

Synthesis of (S)-N-methyl-2-[2-{1-(3-trifluoromethylphenyl)ethoxyiminomethyl}phenyl]-2-methoxyiminoacetamide (compound No. 2)

A 2.05 g (10 mmol) portion of 1-(3-trifluoromethylphenyl)ethoxyamine and 1.50 g (10 mmol) of L-(+)-tartaric acid were dissolved under heating with a mixed solution of methanol/water = 2:1 (10 ml), and the resulting solution was gradually cooled to effect precipitation of crystals. The crystals were removed by filtration, the resulting filtrate was concentrated under a reduced pressure, and the resulting residue was dissolved under heating with a mixed solution of 6 ml of tetrahydrofuran and 2 ml of diisopropyl ether. This was gradually cooled to effect precipitation of crystals which were subsequently collected by filtration. To 1.43 g of the resulting crystals was added 20 ml of 1 N sodium hydroxide aqueous solution, followed by extraction with 20 ml of hexane. The resulting organic layer was washed with saturated brine and dried over anhydrous sodium sulfate. By evaporating the solvent, 0.73 g (3.56 mmol) of SBOA was obtained. The optical purity was 87.4% ee.

A 7 ml portion of methanol solution containing 0.82 g (3.47 mmol) of ethyl 2-(2-formylphenyl)-2-methoxyiminoacetate and 0.73 g (3.56 mmol) of SBOA was stirred for 2 hours at 40°C. Subsequently, 7 ml of 40% methylamine methanol solution was added and the mixture was stirred at room temperature for 3 hours. The solvent was evaporated under a reduced pressure, water was added to the residue, followed by extraction with ethyl acetate. The resulting organic layer was washed with water and saturated brine in that order and then dried over anhydrous sodium sulfate. After evaporation of the solvent, the resulting residue was purified by a silica gel column chromatography (ethyl acetate/hexane = 1:1) to give 1.2 g of the title compound as white crystals (yield 83%). The optical purity was 87.5% ee.

This compound coincided with the first peak shown in Production Example 1 when analyzed by HPLC using an optically active column CHIRALCEL OJ.

Compound Nos. 3 to 7 shown in table 1 were synthesized in the same manner as described in Examples 1 to 3, except that the starting materials were changed.

Table 1

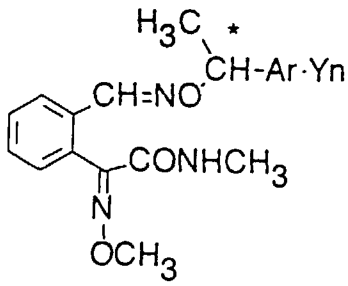

| Compound No. | Ar | Yn | Melting point (°C) |
|---|---|---|---|
| 1 | Phenyl | 3-$CH_3$ | 87.8 - 89.6 |
| 2 | Phenyl | 3-$CH_3$ | 87.9 - 88.8 |
| 3 | Phenyl | H | 105 - 108 |
| 4 | Phenyl | 4-$CF_3$ | 70 - 71 |
| 5 | Phenyl | 3-Cl | 81 - 82 |
| 6 | Phenyl | 4-Cl | 85 - 87 |
| 7 | Phenyl | 3-$OCH_3$, 4-$OCHF_2$ | viscous |

Though each of the compounds in the above table contains two oxime moieties and exists in respective geometrical isomers (E/Z), physical properties of E form compounds are shown in the table. Also, the compound No. 1 showed a positive value of specific angle of rotation in chloroform solvent and was assumed to have an absolute configuration of R based on the result of Example 1. Each of the compound Nos. 2 to 7 showed a negative value of specific angle of rotation in chloroform solvent and was assumed to have an absolute configuration of S.

Next, the condensation reaction shown in the reaction scheme-1 is described further in detail with reference to the synthesis of compound No. 2, (S)-N-methyl-2-[2-{1-(3-trifluoromethylphenyl)ethoxyiminomethyl}phenyl]-2-methoxyiminoacetamide (to be referred to as SMA hereinafter).

Example 1-4

A mixture of 2.56 g (12.49 mmol, 91.4% ee) of SBOA, 3.00 g (11.35 mmol) of N-methyl-2-{2-(1,3-dioxolan-2-yl)phenyl}-2-methoxyiminoacetamide (to be referred to as EGAA hereinafter), 6 ml of acetic acid and 6 ml of water was stirred at room temperature for 24 hours. Then, 6 ml of water and 30 ml of toluene were added to effect separation of layers, the resulting organic layer was washed with 10 ml of water, 10 ml of 5% sodium carbonate aqueous solution and

10 ml of water in that order, and then concentrated under a reduced pressure. Then, 15 ml of n-heptane was added to effect crystallization, thereby obtaining 4.27 g of SMA crystals. The yield was 92.3%, chemical purity was 94.6% and optical purity was 92.2% ee.

Melting point; 88.5 - 89.0°C
$^1$H-NMR δ ($CDCl_3$); 1.58 (3H, d), 2.88 (3H, d), 3.86 (3H, s), 5.30 (1H, q), 6.67 (1H, br), 7.15 (1H, dd), 7.35 - 7.55 (5H, m), 7.60 (1H, s), 7.68 (1H, dd), 7.96 (1H, s)

Example 1-5

A mixture of 2.56 g (12.49 mmol, 91.4% ee) of SBOA, 3.00 g (11.35 mmol) of EGAA, 6 ml of acetic acid and 6 ml of water was stirred at room temperature for 24 hours. After 8 hours in the course the reaction, seed crystal was added to effect gradual precipitation of crystals. The reaction mixture was filtered, and the resulting crystals were washed with 10 ml of acetic acid/water (1/2) and 10 ml of n-heptane, and then dried to give 4.24 g of crystals of SMA. The yield was 91.7%, chemical purity was 95.6%, and optical purity was 94.2% ee.

Example 1-6

The reaction and after-treatment of Example 1-4 were repeated except that amounts of acetic acid and water were changed. The results are shown in the following Table 2.

Example 1-7

The reaction and after-treatment of Example 1-5 were repeated except that amounts of acetic acid and water were changed. The results are shown in the following Table 2.

Table 2

| Example | EGAA (g) | SBOA (g) | SBOA (% ee) | Solvent (ml) AcOH | Solvent (ml) $H_2O$ | Purification method | SMA (g) | Optical purity (% ee) | Chemical purity (%) |
|---|---|---|---|---|---|---|---|---|---|
| 1-4 | 3.00 | 2.56 | 91.4 | 6 | 6 | extraction | 4.24 | 92.2 | 94.6 |
| 1-5 | 3.00 | 2.56 | 91.4 | 6 | 6 | crystallization | 4.24 | 94.2 | 95.6 |
| 1-6 | 3.00 | 2.56 | 91.4 | 8 | 4 | extraction | 4.24 | 93.4 | 88.7 |
| 1-7 | 3.00 | 2.56 | 91.4 | 8 | 4 | crystallization | 2.40 | 98.3 | 95.1 |
| AcOH = acetic acid, $H_2O$ = water | | | | | | | | | |

According to the above results, it was found that SMA having excellent chemical purity and optical purity can be obtained with a high yield by the method in which a water-containing mixed solvent is used, but the isolation yield of SMA is reduced when the ratio of acetic acid is increased.

Next, the effect of solvent ratio is shown in Examples 1-8 to 1-13, and the effect of solvent quantity in Examples 1-14 to 1-17. Also, the condensation reaction when an organic acid salt of SBOA is used is shown in Examples 1-19 to 1-25.

Examples 1-8 to 1-13

A 0.85 g (4.16 mmol) portion of SBOA, 1.00 g (3.78 mmol) of EGAA and 5 ml of respective acetic acid/water mixed solvent having varied mixing ratio were prepared, and the reaction yield after 24 hours of the reaction was measured by HPLC. The results are shown in the following Table 3.

Examples 1-14 to 1-17

A 2.56 g (12.49 mmol) portion of SBOA, 3.00 g (11.35 mmol) of EDAA and a mixed solvent of acetic acid/water = 2:1 with varied amount were prepared, and the reaction yield after 24 hours of the reaction was measured by HPLC. The results are shown in the following Table 3.

Example 1-18

A mixture of 2.69 g (7.57 mmol, 98.0% ee) of (S)-1-(3-trifluoromethylphenyl)ethoxiamine • L-tartarate (to be referred to as SBOA • LTA hereinafter), 2.00 g (7.57 mmol) of EGAA, 6 ml of acetic acid and 6 ml of water was stirred at room temperature for 24 hours. The reaction mixture was filtered, and the resulting crystals were washed with 20 ml of water at 50°C and then dried to give 2.82 g of SMA 20565 in crystals. The yield was 91.5%, chemical purity was 97.8%, and optical purity was 98.8% ee. In this connection, the reaction yield measured by HPLC is shown in the following Table 3.

Examples 1-19 to 1-25

The effect of solvent was examined by repeating the reaction of Example 1-18, except that amount of the reaction solvent was changed. Results of the measurement of reaction yield by HPLC are shown in the following Table 3.

Table 3

| Example | Mode of SBOA | Solvent ratio | Solvent (ml) | SMA (%) 6 h | SMA (%) 24 h |
|---|---|---|---|---|---|
| 1-8 | SBOA (0.85 g) | $AcOH/H_2O$ = 20/1 | 5 | 42.1 | 80.7 |
| 1-9 | SBOA (0.85 g) | $AcOH/H_2O$ = 4/1 | 5 | 72.7 | 90.4 |
| 1-10 | SBOA (0.85 g) | $AcOH/H_2O$ = 3/1 | 5 | 72.7 | 89.1 |
| 1-11 | SBOA (0.85 g) | $AcOH/H_2O$ = 2/1 | 5 | 75.1 | 94.1 |
| 1-12 | SBOA (0.85 g) | $AcOH/H_2O$ = 1/1 | 5 | 60.2 | 89.2 |
| 1-13 | SBOA (0.85 g) | $AcOH/H_2O$ = 1/2 | 5 | 46.3 | 88.4 |
| 1-14 | SBOA (2.56 g) | $AcOH/H_2O$ = 2/1 | 6 | 37.9 | 78.4 |
| 1-15 | SBOA (2.56 g) | $AcOH/H_2O$ = 2/1 | 9 | 56.0 | 89.9 |
| 1-16 | SBOA (2.56 g) | $AcOH/H_2O$ = 2/1 | 12 | 68.4 | 92.6 |
| 1-17 | SBOA (2.56 g) | $AcOH/H_2O$ = 2/1 | 15 | 74.9 | 91.2 |
| 1-18 | SBOA • LTA | $AcOH/H_2O$ = 1/1 | 12 | 96.0 | 96.4 |
| 1-19 | SBOA • LTA | $AcOH/H_2O$ = 1/2 | 12 | 96.1 | 96.9 |
| 1-20 | SBOA • LTA | $AcOH/H_2O$ = 1/5 | 12 | 86.9 | 92.7 |
| 1-21 | SBOA • LTA | $AcOH/H_2O$ = 0/1 | 12 | 50.0 | 95.2 |
| 1-22 | SBOA • LTA | $AcOEt/H_2O$ = 1/1 | 12 | 87.0 | 88.6 |
| 1-23 | SBOA • LTA | n-Heptane/$H_2O$ = 1/1 | 12 | 49.5 | 94.3 |
| 1-24 | SBOA • LTA | n-BuOH/$H_2O$ = 1/1 | 12 | 44.6 | 95.4 |
| 1-25 | SBOA • LTA | n-BuOH/AcOH/$H_2O$ = 1/1 | 12 | 47.3 | 93.5 |
| EtOAc = ethyl acetate, n-BuOH = n-butanol | | | | | |

The above results suggested that there are optimum values in the mixing ratio of acetic acid/water mixed solvent and in the amount of solvent. It was found also that the reaction completes for the most part after 6 hours when the reaction is carried out with the tartaric acid salt in an acetic acid/water. In this connection, it is evident that the reaction also progresses sufficiently even in other solvent systems when the reaction is carried out for 24 hours.

Next, the following exemplifies condensation reaction of SBOA with a compound in which the acetal moiety of EGAA is modified.

Example 1-26

A mixture of 2.56 g (12.5 mmol) of SBOA, 3.16 g (11.4 mmol) of N-methyl-2-[2-(1,3-dixan-2-yl)phenyl]-2-methoxyiminoacetamide, 6 ml of acetic acid and 6 ml of water was subjected to the same reaction and after-treatment described in Example 1-4, thereby obtaining 3.93 g of SMA in the form of crystals. The yield was 84.3%.

Example 1-27

A mixture of 2.56 g (12.5 mmol) of SBOA, 3.64 g (11.4 mmol) of N-methyl-2-[2-(4,4,6-trimethyl-1,3-dioxan-2-yl)phenyl]-2-methoxyiminoacetamide, 6 ml of acetic acid and 6 ml of water was subjected to the same reaction and after-treatment described in Example 1-4, thereby obtaining 4.05 g of SMA in the form of crystals. The yield was 87.5%.

Next, methods for the synthesis of starting materials to be used for the production of optically active methoxyiminoacetamide derivatives are described in detail with reference to the following examples.

First, the production method for the acetal derivative (III) is described in order to reveal effects of bases and solvents. The effect of bases in a non-aqueous system is revealed in Examples 2-1 to 2-3, and the effect of solvents in an aqueous solution system in Examples 2-4 and 2-5.

Example 2-1

Synthesis of N-methyl-2-[2-(1,3-dioxan-2-yl)phenyl]-2-methoxyiminoacetamide

A mixture of 5.41 g (64.8 mmol: 1.15 eq) of methoxyamine hydrochloride, 6.57 g (70.5 mmol: 1.25 eq) of 4-picoline and 45 ml of ethanol was stirred at room temperature for 20 minutes. Then, 14.9 g (56.4 mmol) of ethyl 2-(1,3-dioxan-2-yl)phenylglyoxylate obtained by the method of Production Example 2 was added, and the mixture was stirred at room temperature for 24 hours. Then, 15 ml of N,N-dimethylformamide (DMF) was added, and the mixture was stirred for 8 hours. The reaction mixture was concentrated under a reduced pressure, and 100 ml of toluene and 100 ml of water were added to effect separation of layers. The resulting organic layer was washed with 25 ml of water and 25 ml of saturated brine and then dried over anhydrous sodium sulfate. By evaporating the solvent, 16.24 g of ethyl 2-[2-(1,3-dioxan-2-yl)phenyl)-2-methoxyiminoacetate was obtained. A ratio of E/Z = 77/23 (NMR integral ratio) was obtained. $^1$H-NMR $\delta$ ($CDCl_3$); 1.30 (3H, t), 2.1 - 2.3 (2H, m), 3.88 (2H, dd), 4.02 (3H, s), 4.18 (2H, dd), 4.31 (2H, q), 5.36 (1H, s), 7.16 (1H, d), 7.35 - 7.45 (2H, m), 7.63 (1H, d)

A mixture of 16.24 g of ethyl 2-[2-(1,3-dioxan-2-yl)phenyl]-2-methoxyiminoacetate, 4 ml of methanol and 16 ml of 40% methylamine methanol solution was stirred at room temperature for 3 hours. The solvent was evaporated, and 7 ml of ethyl acetate and 21 ml of hexane were added to the 15.63 g of residue to effect precipitation of crystals, which were subsequently collected by filtration and washed with an ethyl acetate/hexane mixed solvent (1/3, 40 ml), thereby obtaining 11.67 g of the title compound. A ratio of E/Z = 90/10 (NMR integral ratio) was obtained. The yield was 74%.

Melting point; 125.0 - 136.0°C
$^1$H-NMR $\delta$ ($CDCl_3$); 2.1 - 2.3 (2H, m), 2.91 (3H, d), 3.88 (2H, dd), 3.95 (3H, s), 4.19 (2H, dd), 5.41 (1H, s), 6.55 (1H, br), 7.17 (1H, d), 7.35 - 7.45 (2H, m), 7.68 (1H, d)

Example 2-2

Synthesis of N-methyl-2-[2-(4,4,6-trimethyl-1,3-dioxan-2-yl)phenyl]-2-methoxyiminoacetamide

A mixture of 4.07 g (48.8 mmol) of methoxyamine hydrochloride, 4.05 g (51.3 mmol) of 4-picoline and 40 ml of ethanol was stirred at room temperature for 20 minutes, 13.0 g (42.4 mmol) of ethyl 2-(4,4,4-trimethyl-1,3-dioxan-2-yl)phenylglyoxylate obtained by the method of Production Example 3 was added, and the mixture was stirred at room temperature for 24 hours. Then, 100 ml of toluene and 100 ml of water were added to the reaction mixture to effect separation of layers, and the resulting organic layer was washed with 25 ml of 1 N hydrochloric acid, 25 ml of water and 25 ml of saturated brine in that order and then dried over anhydrous sodium sulfate. By evaporating the solvent, 13.34 g of ethyl 2-[2-(4,4,6-trimethyl-1,3-dioxan-2-yl)phenyl]-2-methoxyiminoacetate was obtained. A ratio of E/Z = 79/21 (NMR integral ratio) was obtained.

A mixture of 13.34 g of ethyl 2-[2-(4,4,6-trimethyl-1,3-dioxan-2-yl)phenyl]-2-methoxyiminoacetate and 13 ml of 40% methylamine methanol solution was stirred at room temperature for 4 hours. The solvent was evaporated, 1 ml of ethyl acetate and 30 ml of hexane were added to the residue to effect precipitation of crystals, which were subsequently collected by filtration and washed with 30 ml of hexane, thereby obtaining 9.52 g of the title compound. A ratio of E/Z = 95/5 (NMR integral ratio) was obtained. The yield was 70%.

Melting point; 112.4 - 116.1°C
$^1$H-NMR δ ($CDCl_3$); 1.24 (3H, d), 1.25 (3H, s), 1.35 (3H, s), 1.46 (2H, dd), 2.97 (3H, d), 3.95 (3H, s), 3.95 - 4.05 (1H, m), 5.68 (1H, s), 6.47 (1H, br), 7.12 (1H, d), 7.32 - 7.43 (2H, m), 7.72 (1H, d)

Example 2-3

Synthesis of N-methyl-2-[2-(1,3-dioxolan-2-yl)phenyl]-2-methoxyiminoacetamide (EGAA)

To a mixture of 19.21 g (230 mmol: 1.15 eq) of methoxyamine hydrochloride and 25 ml of ethanol was added 23.28 g (250 mmol: 1.25 eq) of 4-picoline, followed by stirring at room temperature for 1 hour. Then, 50.05 g (200 mmol) of ethyl 2-(1,3-dioxolan-2-yl)phenylglyoxylate (to be referred to as "EGA" hereinafter) obtained by the method of Production Example 4 was added, and the mixture was stirred at room temperature for 24 hours. A 100 ml portion of 40% methylamine methanol solution was added dropwise, spending 15 minutes, to the reaction mixture under ice-cooling, and the mixture was stirred under ice-cooling for 30 minutes and at room temperature for 2 hours. After evaporation of the solvent from the reaction mixture, 250 ml of ethyl acetate was added to the residue, and the mixture was washed with saturated brine (50 ml x 3) and then dried over anhydrous sodium sulfate. After evaporation of the solvent, the resulting residue was crystallized by adding 25 ml of ethyl acetate and 75 ml of hexane, filtered and then washed with an ethyl acetate/hexane mixed solvent (1/3, 100 ml) to give 43.65 g of the title compound. A ratio of E/Z = 94/6 (NMR integral ratio) was obtained. The yield from EGA was 83%.

Thereafter, the E/Z mixture was separated and purified by a silica gel column chromatography and their physical properties were measured.

(E)-N-methyl-2-[2-(1,3-dioxolan-2-yl)phenyl]-2-methoxyiminoacetamide

Melting point; 106.1 - 110.8°C
$^1$H-NMR δ ($CDCl_3$); 2.93 (3H, d), 3.95 (7H, s), 5.88 (1H, s), 6.72 (1H, br), 7.12 - 7.18 (1H, m), 7.37 - 7.45 (2H, m), 7.58 - 7.54 (1H, m)

(Z)-N-methyl-2-[2-(1,3-dioxolan-2-yl)phenyl]-2-methoxyiminoacetamide

Melting point; 71.4 - 73.7°C
$^1$H-NMR δ ($CDCl_3$); 2.90 (3H, d), 4.01 (3H, s), 4.03 (4H, s), 6.25 (1H, s), 6.93 (1H, br), 7.35 - 7.5 (3H, m), 7.63 (1H, d)

Example 2-4

Synthesis of EGAA

A reaction vessel was charged with 130.5 g (0.521 mol) of 33% methoxyamine hydrochloride aqueous solution, 87 g of water and 60.7 g (0.651 mol) of 4-picoline and then charged with 106.10 g of crude EGA obtained by the method of Production Example 4 (83.9 g, 0.335 mol as its pure form) and 106 ml of n-butanol, and the mixture was stirred at room temperature for 35 hours. To the reaction mixture was added 12.1 g of sodium chloride, separated and washed with brine (12.1 g of sodium chloride and 87 g of water). Next, 78 g of 40% methylamine aqueous solution was added to the resulting organic layer spending 15 minutes at room temperature, and the mixture was stirred for 3 hours as such. When this was separated by adding 10 g of sodium chloride and washed with brine (26 g of sodium chloride and 87 g of water) and then n-butanol was evaporated under a reduced pressure, crystals of EDAA were precipitated. The crystals were washed with a mixed solvent of 144 ml of n-hexane and 36 ml of ethyl acetate, again washed with the mixed solvent of 144 ml of n-hexane and 36 ml of ethyl acetate, collected by filtration and then dried to give 74.73 g of light yellow crystals of EGAA (0.283 mol, purity 96.7%, E/Z = 93/7). The yield from EGA was 84%.

Example 2-5

Synthesis of EGAA

A reaction vessel was charged with 22.02 g (87.9 mmol) of 33% methoxyamine hydrochloride aqueous solution, 14.68 g of water and 8.93 g (95.9 mmol) of 4-picoline and then charged with 25.2 g of crude EGA obtained by the method of Production Example 4 (20.0 g, 79.9 mmol as its pure form) and 20 ml of n-butanol, and the mixture was stirred at 50°C for 6 hours. After cooling to room temperature, this was separated by adding 3.0 g of sodium chloride to remove the water layer, 18.6 g of 40% methylamine aqueous solution was added to the resulting organic layer spending 5 minutes at room temperature, and the mixture was stirred for 2 hours as such, resulting in precipitation of white crystals of EGAA. In this case, all of the EGAA crystals were floated over the n-butanol layer. This was separated by adding 3.0 g of NaCl to remove the water layer, and the product was collected by filtration and dried to give 16.80 g (63.6 mmol, E/Z = 98/2) of EGGA in the form of white crystals. In this case, impurities other than its Z isomer were not found in the crystals of EGAA. The yield from EGA was 80%.

Comparative Example 2-1

Synthesis of EGAA using pyridine

A 19.75 g (250 mmol: 1.25 eq) portion of pyridine was added to a mixture of 19.21 g (230 mmol: 1.15 eq) of methoxyamine hydrochloride and 25 ml of ethanol, and the resulting mixture was stirred at room temperature for 1 hour. The, 50.05 g (200 mmol) of EGA was added and the mixture was again stirred at room temperature for 23 hours. A 100 ml portion of 40% methylamine methanol solution was added dropwise, spending 10 minutes, to the reaction mixture under ice-cooling, and the resulting mixture was stirred for 30 minutes under ice-cooling and then for 2 hours at room temperature. The solvent was evaporated from the reaction mixture, 250 ml of ethyl acetate was added to the residue, and the mixture was washed with saturated brine (50 ml x 3) and then dried over anhydrous sodium sulfate. After evaporation of the solvent, the resulting residue was crystallized by adding 25 ml of ethyl acetate and 75 ml of hexane, filtered and then washed with an ethyl acetate/hexane mixed solvent (1/3, 100 ml) to give 38.58 g of the title compound. E/Z = 94/6 (NMR integral ratio). The yield from EGA was 73%.

Comparative Example 2-2

Synthesis of EGAA using pyridine

A mixture of 3.34 g (40 mmol) of methoxyamine hydrochloride, 3.80 g (48 mmol) of pyridine and 25 ml of ethanol was stirred at room temperature for 20 minutes, the mixture was cooled to 5°C, 10.0 g (40 mmol) of EGA was added, and then the mixture was stirred for 65 hours at a temperature of from 5°C to room temperature. Then, 75 ml of toluene and 100 ml of water were added to the reaction mixture to effect separation of layers, and the resulting organic layer was washed with 25 ml of 1 N hydrochloric acid, 25 ml of water, 25 ml of saturated sodium bicarbonate aqueous solution and 25 ml of saturated brine in that order and then dried over anhydrous sodium sulfate. By evaporating the solvent, 10.18 g of ethyl 2-[2-(1,3-dioxolan-2-yl)phenyl]-2-methoxyiminoacetate was obtained. E/Z = 85/15 (GC area ratio).

Ethyl (E)-2-[2-(1,3-dioxolan-2-yl)phenyl]-2-methoxyiminoacetate

$^1$H-NMR δ ($CDCl_3$); 1.31 (3H, t), 3.9 - 4.0 (4H, m), 4.02 (3H, s), 4.33 (2H, q), 5.79 (1H, s), 7.12 - 7.19 (1H, m), 7.38 - 7.45 (2H, m), 7.53 - 7.60 (1H, m)

Ethyl (Z)-2-[2-(1,3-dioxolan-2-yl)phenyl]-2-methoxyiminoacetate

$^1$H-NMR δ ($CDCl_3$); 1.31 (3H, t), 3.98 - 4.15 (4H, m), 4.03 (3H, s), 4.32 (2H, q), 6.13 (1H, s), 7.35 - 7.48 (3H, m), 7.68 (1H, d)

A mixture of 10.18 g of ethyl 2-[2-(1,3-dioxolan-2-yl)phenyl]-2-methoxyiminoacetate (E/Z = 85/15) and 10 ml

of 40% methylamine methanol solution was stirred at room temperature for 3 hours. After evaporation of the solvent, the resulting residue was crystallized by adding 10 ml of ethyl acetate and 30 ml of hexane, filtered and then washed with an ethyl acetate/hexane mixed solvent (1/3, 40 ml; 1/1, 10 ml) to give 5.33 g of the title compound. E/Z = 95/5 (NMR integral ratio). The yield from EGA was 50%.

Based on the results of Examples 2-1 to 2-5 and Comparative Examples 2-1 and 2-2, it was revealed that the acetal derivative (III) can be obtained with a high yield when 4-picoline is used as a base. It was found also that EGAA having most higher purity is obtained with a high yield when n-butanol is used as a solvent and the oxime formation and amidation are carried out by one pot reaction.

Next, in order to further clarify these points, the effect of base in a non-aqueous system was examined in Examples 2-6 to 2-8, the effect of solvent in a non-aqueous system was examined in Examples 2-9 to 2-12, and the effect of pH in an aqueous solution system in Examples 2-13 to 2-16, with regard to the following oxime formation reaction as an example.

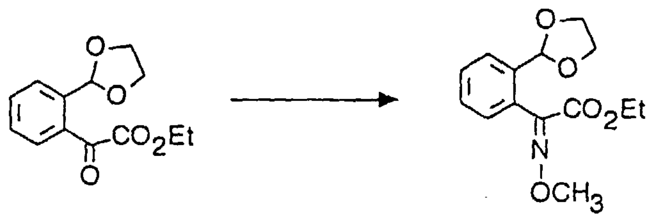

Oxime

Examples 2-6 to 2-8

To a mixture of 0.77 g (9.20 mmol, 1.25 eq) of methoxyamine hydrochloride and a base (1.25 eq) was added 2.0 g (7.99 mmol) of EGA and 4 ml of ethanol, followed by 24 to 48 hours of the reaction at room temperature. The results obtained by changing bases are shown in Table 4. The data indicate the total amount of formed oxime in E and Z forms calculated by analyzing each reaction solution with a gas chromatography.

Table 4

| Example | $MeONH_2 \cdot HCl$ (eq.) | Base (eq.) | Solvent | Reaction temp (°C) | Reaction time (h) | Oxime (%) |
|---|---|---|---|---|---|---|
| Example 2-6 | anhydrous 1.15 | 4-picoline 1.25 | EtOH | room temp. | 24 | 91 |
| Example 2-7 | anhydrous 1.15 | N,N-dimethylaniline 1.25 | EtOH | room temp. | 24 | 90 |
| Example 2-8 | anhydrous 1.15 | 2,6-lutidine 1.25 | EtOH | room temp. | 48 | 91 |
| Comparative Example 2-3 | anhydrous 1.15 | pyridine 1.25 | EtOH | room temp. | 24 | 80 |
| Comparative Example 2-4 | anhydrous 1.15 | $Et_3N$ 1.25 | ETOH | room temp. | 24 | 2.3 |
| $Et_3N$ = triethylamine, EtOH = ethanol | | | | | | |

The above results suggested that 4-picoline, N,N-dimethylaniline or 2,6-lutidine is an excellent base in the oxime formation in the non-aqueous system. Examples 2-9 to 2-12

The reaction of Example 2-6 was repeated with 4-picoline as the base in the same way, except that the reaction temperature was fixed to 50°C and the solvent was changed. The results are shown in the following Table 5.

Table 5

| Example | $MeONH_2$ • HC 1 (eq.) | Base (eq.) | Solvent | Reaction temp. (°C) | Reaction time (h) | Oxime (%) |
|---|---|---|---|---|---|---|
| 2-9 | anhydrous 1.15 | 4-picoline 1.25 | EtOH | 50 | 4.0 | 76 |
| 2-10 | anhydrous 1.15 | 4-picoline 1.25 | n-BuOH | 50 | 1.0 | 89 |
| 2-11 | anhydrous 1.15 | 4-picoline 1.25 | Toluene | 50 | 6.0 | 57 |
| 2-12 | anhydrous 1.15 | 4-picoline 1.25 | $i-Pr_2O$ | 50 | 5.0 | 69 |
| $i-Pr_2O$ = diisopropyl ether | | | | | | |

As shown in the above, it was found that excellent results can be obtained when n-butanol is used as the solvent.

Example 2-13

The reaction of Example 2-10 was repeated, except that methoxyamine hydrochloride was used in the form of aqueous solution. The results are shown in the following Table 6. In this case, pH of the reaction solution was maintained at 4 to 6.

Examples 2-14 to 2-16

The procedure of Example 2-13 was repeated, except that the reaction was carried out at room temperature by changing bases. The results are shown in the following Table 6. In this case, pH values of the reaction solutions of Examples 2-14 to 2-16 were maintained at 4 to 6, but pH of the reaction solution of Comparative Example 2-5 fluctuated between 6 and 1 and finally reached 1 or less. Table 6

| Example | $MeONH_2$ • HCl (eq.) | Base (eq.) | Solvent | Reaction temp (°C) | Reaction time (h) | Oxime (%) |
|---|---|---|---|---|---|---|
| Example 2-13 | 40% aqueous solution 1.15 | 4-picoline 1.25 | n-BuOH | 50 | 6.0 | 90 |
| Example 2-14 | 40% aqueous solution 1.15 | 4-picoline 1.25 | n-BuOH | room temp. | 35 | 92 |
| Example 2-15 | 40% aqueous solution 1.15 | N,N-dimethylaniline 1.25 | n-BuOH | room temp. | 16 | 81 |
| Example 2-16 | 40% aqueous solution 1.15 | pH 5 with 48% NaOH aqueous solution | n-BuOH | room temp. | 35 | 92 |
| Comparative Example 2-5 | 40% aqueous solution 1.15 | NaOH 0.87 | n-BuOH | room temp. | 6.0 | 0 |

Next, as the production method for the optically active 1-arylethoxyamine derivative (II), production of racemic compound, asymmetric synthesis and a production method in which optical resolution is employed to make the optically active compound into more higher optical purity are described in succession.

Example 3-1

Synthesis of *O*-{1-(3-trifluoromethylphenyl)ethyl}-acetohydroxamate

A 20 mg portion of DMF was added to 5 ml of toluene solution containing 3 g (15.8 mmol) of 1-(3-trifluoromethylphenyl)ethylalcohol. Then, 2.25 g (18.9 mmol) of thionyl chloride was added under ice-cooling. After 2 hours of stirring at 10°C or less, this was concentrated under a reduced pressure on a water bath at temperature of 50 to 60°C. After cooling to room temperature, 8 ml of DMF, 3.3 g (23.9 mmol) of anhydrous potassium carbonate and 2.15 g (20.5 mmol) of acetohydroxamic acid were added, and the mixture was stirred for 6 hours while heating at 120 to 130°C. After cooling, ethyl acetate was added to the reaction mixture, and the mixture was washed with water and saturated brine in that order and then dried over anhydrous sodium sulfate. This was concentrated under a reduced pressure, and the resulting residue was purified by a silica gel column chromatography (n-Hex:AcOEt = 1:1 → 1:2) to give 2.32 g of the title compound (yield = 59.5%).

Melting point; 75.3 - 76.5°C
$^1$H-NMR δ ($CDCl_3$); 1.57 (3H, d), 1.8 - 2.2 (3H, br), 4.8 - 5.15 (1H, br), 7.45 - 7.55 (4H, m), 7.8 - 8.0 (1H, br)

Example 3-2

Synthesis of *O*-{1-(3-trifluoromethylphenyl)ethyl}-acetohydroxamate

At 70°C and spending 30 minutes, 300 g (1.578 mol) of 1-(3-trifluoromethylphenyl)ethylalcohol was added dropwise to 500 g (6 mol) of concentrated hydrochloric acid. This was stirred at 85°C for 1 hour. After cooling, this was extracted with 400 ml of n-heptane, and the resulting organic layer was washed with water and saturated brine in that order. After drying over anhydrous sodium sulfate, this was concentrated under a reduced pressure to give 315 g of 1-(3-trifluoromethylphenyl)ethyl chloride (yield = 95.7%).

$^1$H-NMR δ ($CDCl_3$); 1.86 (3H, d), 5.12 (1H, q), 7.49 (1H, t), 7.57 (1H, d), 7.62 (1H, d), 7.67 (1H, s)

A 1.5 g (20 mmol) portion of acetohydroxamic acid, 10 mg of tetra-n-butylammonium bromide and 2.0 g (9.6 mmol) of the just obtained 1-(3-trifluoromethylphenyl)ethyl chloride were added to a mixed solution of 0.8 g (20 mmol) of sodium hydroxide, 6 ml of ethanol and 2 ml of water, and the mixture was stirred for 3 hours while heating at 70°C. After cooling to room temperature, this was extracted with 20 ml of toluene, and the resulting organic layer was washed with water and saturated brine in that order. After drying over anhydrous sodium sulfate, this was concentrated under a reduced pressure, and 10 ml of n-heptane was added to the resulting residue. By collecting the thus precipitated crystals by filtration, 1.85 g of the title compound was obtained (yield = 78.1%).

By repeating the same procedure, except for changing the starting materials, the following compounds were synthesized.

*O*-(1-phenylethyl)-acetohydroxamate;
Melting point; 78.0 - 78.7°C
Yield; 70.1%
*O*-{1-(4-trifluoromethylphenyl)ethyl}-acetohydroxamate;
Melting point; 80 - 83°C
Yield; 81.5%
*O*-{1-(3-chlorophenyl)ethyl}-acetohydroxamate;
Melting point; 69 - 71°C
Yield; 75%
*O*-{1-(4-chlorophenyl)ethyl}-acetohydroxamate;
Melting point; 71 - 74°C
Yield; 79%

Example 3-3

Synthesis of 1-(3-trifluoromethylphenyl)ethoxyamine

A 1 ml portion of concentrated hydrochloric acid was added to 3 ml of ethanol solution containing 1.0 g (0.4 mmol) of *O*-{1-(3-trifluoromethylphenyl)ethyl}-acetohydroxamate, and the mixture was stirred at 50°C for 2 hours. Then, n-hexane was added to the reaction mixture, and the mixture was washed with water and saturated brine in that order and then dried over anhydrous sodium sulfate. This was concentrated under a reduced pressure to give 0.82 g of the title compound (yield = 99%).

Example 3-4

Synthesis of 1-(3-trifluoromethylphenyl)ethoxyamine

A 80.4 g (1.157 mol) portion of hydroxylamine hydrochloride was added to a mixed solution of 138 g (1.302 mol) of sodium carbonate, 75 ml of ethanol and 75 ml of water, and the mixture was stirred at 35°C for 20 minutes. Then, 132 g (1.293 mol) of acetic anhydride was slowly added dropwise by spending 3 hours. Then, 100 ml of ethanol was added and the mixture was stirred at 40°C for 2 hours. After cooling to 15°C, 84.9 g (1.44 mol) of 95% potassium hydrochloride and 1.5 g (4.65 mmol) of tetra-n-butylammonium bromide were added under ice-cooling. At 45°C, 120.1 g (0.575 mol) of 1-(3-trifluoromethylphenyl)ethyl chloride which has been dissolved in 50 ml of ethanol was added dropwise. After 6 hours of stirring at 70°C and subsequent cooling to 20°C, 350 ml (4.03 mol) of concentrated hydrochloric acid was added dropwise to the reaction mixture under ice-cooling. After 2 hours of stirring at 50°C for 2 hours and subsequent cooling, 120 g of ice was added thereto. Then, 100 ml of n-hexane was added to the reaction mixture to effect separation, and the resulting water layer was neutralized by adding 110 g (2.75 mol) of 99% sodium hydroxide at 30°C or less. A 180 ml portion of n-hexane was added to effect separation of layers, and the resulting organic layer was washed with water and saturated brine in that order. After drying over anhydrous sodium sulfate, this was concentrated under a reduced pressure, and the resulting residue was purified by distillation under a reduced pressure to give 93 g of the title compound (yield = 74.3%).

Boiling point; 59.5 - 61°C/2 mmHg

Examples 3-5 to 3-24

Examples of the 1-arylethoxyamine derivative (II)' obtained in the same manner as described in Examples 3-2 to 3-4, except for changing the starting materials, are shown in Table 7.

Table 7

| $H_2NOCH(CH_3)$-Ar-Yn | | |
|---|---|---|
| Example | Ar-Yn | $^1$H-NMR data ($CDCl_3$) |
| 3-5 | Phenyl | 1.43 (3H, d), 4.65 (1H, q), 5.21 (2H, bs), 7.3 - 7.4 (5H, m) |
| 3-6 | 4-Me-Phenyl | 1.41 (3H, d), 2.35 (3H, s), 4.61 (1H, q), 5.17 (2H, bs), 7.17 (2H, d), 7.23 (2H, d) |
| 3-7 | 4-t-Bu-Phenyl | 1.32 (9H, s), 1.43 (3H, d), 4.62 (1H, q), 5.19 (2H, bs), 7.27 (2H, d), 7.39 (2H, d) |
| 3-8 | 4-Cl-Phenyl | 1.40 (3H, d), 4.64 (1H, q), 5.23 (2H, br), 7.27 (2H, d), 7.33 (2H, d) |
| 3-9 | 3-$CF_3$-Phenyl | 1.43 (3H, d), 4.72 (1H, q), 5.30 (2H, br), 7.4 - 7.6 (3H, m), 7.60 (1H, s) |
| 3-10 | 4-$CF_3$-Phenyl | 1.42 (3H, d), 4.73 (1H, q), 5.30 (2H, br), 7.45 (2H, d), 7.62 (2H, d) |
| 3-11 | 3-Cl-Phenyl | 1.40 (3H, d), 4.62 (1H, q), 5.27 (2H, br), 7.2 - 7.4 (4H, m) |
| 3-12 | 4-CN-Phenyl | 1.40 (3H, d), 4.72 (1H, q), 5.35 (2H, bs), 7.45 (2H, d), 7.66 (2H, d) |
| 3-13 | 3-$OCF_3$-Phenyl | 1.41 (3H, d), 4.68 (1H, q), 5.29 (2H, br), 7.14 (1H, d), 7.20 (1H, s), 7.26 (1H, d), 7.38 (1H, dd) |
| 3-14 | 4-$OCF_3$-Phenyl | 1.41 (3H, d), 4.67 (1H, q), 5.26 (2H, bs), 7.21 (2H, d), 7.36 (2H, d) |
| 3-15 | 4-$OCH_2$-$CF_3$-Phenyl | 1.41 (3H, d), 4.35 (2H, m), 4.62 (1H, q), 5.19 (2H, bs), 6.93 (2H, d), 7.30 (2H, d) |
| 3-16 | 4-SMe-Phenyl | 1.41 (3H, d), 2.48 (3H, s), 4.61 (1H, q), 5.20 (2H, br), 7.26 (4H, s) |
| 3-17 | 3-OMe-4-$OCHF_2$-Phenyl | 1.42 (3H, d), 3.90 (3H, s), 4.63 (1H, q), 5.26 (2H, bs), 6.54 (1H, t), 6.91 (1H, dd), 6.96 (1H, d), 7.15 (1H, d) |
| 3-18 | 4-O-Propargyl-Phenyl | 1.41 (3H, d), 2.52 (1H, t), 4.61 (1H, q), 4.69 (2H, d), 5.18 (2H, bs), 6.95 (2H, d), 7.30 (2H, d) |

Table 7 (continued)

| $H_2NOCH(CH_3)$-Ar-Yn | | |
|---|---|---|
| Example | Ar-Yn | $^1$H-NMR data ($CDCl_3$) |
| 3-19 | 4-$NO_2$-Phenyl | 1.42 (3H, d), 4.78 (1H, q), 5.37 (2H, bs), 7.50 (2H, d), 8.23 (2H, d) |
| 3-20 | 4-Phenyl-Phenyl | 1.53 (3H, d), 4.95 (1H, q), 6.50 (2H, br), 7.3 - 7.7 (9H, m) |
| 3-21 | 2-Naphthyl | 1.51 (3H, d), 4.81 (1H, q), 5.25 (2H, bs), 7.4 - 7.5 (3H, m), 7.7 -7.9 (4H, m) |
| 3-22 | 1,3-Benzodioxol -5-yl | 1.39 (3H, d), 4.56 (1H, q), 5.19 (2H, bs), 6.79 (1H, s), 6.83 (2H, d) |
| 3-23 | 1,4-Benzodioxan -6-yl | 1.39 (3H, d),4.25 (4H, m), 4.53 (1H, q), 5.18 (2H, bs), 6.8 - 7.0 (3H, m) |
| 3-24 | 2-Chlorothiophen-5-yl | 1.50 (3H, d), 4.78 (1H, q), 5.33 (2H, bs), 6.78 (2H, s) |

The following describes a method in which the optically active 1-arylethoxyamine derivative (II) is obtained by subjecting the optically active 1-arylethyl halide derivative (VIII) to etherification and hydrolysis.

Example 3-25

Synthesis of (S)-*O*-{1-(3-trifluoromethylphenyl)ethyl}-acetohydroxamate

A 3.3 g (23.9 mmol) portion of anhydrous potassium carbonate and 2.15 g (20.5 mmol) of acetohydroxamic acid were added to 8 ml of DMF solution containing 3.3 g (15.8 mmol, optical purity: 93% ee) of (R)-1-(3-trifluoromethylphenyl)-1-ethyl chloride, and the mixture was stirred at 120 to 130°C for 6 hours. Ethyl acetate was added to the reaction mixture, and the mixture was washed with water and saturated brine in that order and then dried over anhydrous sodium sulfate. This was concentrated under a reduced pressure, and the resulting residue was purified by a silica gel column chromatography (n-Hex:AcOEt = 1:1 → 1:2) to give 2.55 g of the title compound (yield = 65.3%, optical purity: 82% ee). The optical yield was 88.2%.

$^1$H-NMR δ ($CDCl_3$); 1.57 (3H, d), 1.8 - 2.2 (3H, br), 4.8 - 5.15 (1H, br), 7.45 - 7.55 (4H, m), 7.8 - 8.0 (1H, br)

Example 3-26

Synthesis of (S)-*O*-{1-(3-trifluoromethylphenyl)ethyl}-acetohydroxamate

A 1.5 g (20 mmol) portion of acetohydroxamic acid, 10 mg of tetra-n-butylammonium bromide and 2.0 g (9.6 mmol, optical purity: 93% ee) of (R)-1-(3-trifluoromethylphenyl)-1-ethyl chloride were added to a mixed solution of 0.8 g (20 mmol) of sodium hydroxide, 6 ml of ethanol and 2 ml of water, and the mixture was stirred at 70°C for 3 hours. After cooling to room temperature, this was extracted with 20 ml of toluene, and the resulting organic layer was washed with water and saturated brine in that order. After drying over anhydrous sodium sulfate, this was concentrated under a reduced pressure, and the resulting residue was purified by a silica gel column chromatography (n-Hex:AcOEt = 1:1 → 1:2) to give 1.85 g of the title compound (yield = 77.9%, optical purity: 90% ee). The optical yield was 96.8%.

Example 3-27

Synthesis of (S)-*O*-(1-phenyl-ethyl)-acetohydroxamate

The title compound (yield = 80.5%, optical yield: 93%) was obtained by the same method of Example 3-26, except that the starting material was changed.

Example 3-28

Synthesis of (S)-*O*-{1-(4-trifluoromethylphenyl)ethyl}-acetohydroxamate

The title compound (yield = 82.0%, optical yield: 92%) was obtained by the same method of Example 3-26, except that the starting material was changed.

Example 3-29

Synthesis of (S)-*O*-{1-(3-chlorophenyl)ethyl}-acetohydroxamate

The title compound (yield = 75.3%, optical yield: 96%) was obtained by the same method of Example 3-26, except that the starting material was changed.

Example 3-30

Synthesis of (S)-*O*-{1-(4-chlorophenyl)ethyl}-acetohydroxamate

The title compound (yield = 86.0%, optical yield: 90%) was obtained by the same method of Example 3-26, except that the starting material was changed.

Example 3-31

Synthesis of (S)-1-(3-trifluoromethylphenyl)ethoxyamine (SBOA)

A 1 ml portion of concentrated hydrochloric acid was added to 3 ml of ethanol solution containing 1.0 g (0.4 mmol, optical purity: 90% ee) of (S)-*O*-{1-(3-trifluoromethylphenyl)ethyl}-acetohydroxamate, and the mixture was stirred at 50°C for 1.5 hours. Then, n-hexane was added to the reaction mixture, and the mixture was washed with water and saturated brine in that order and then dried over anhydrous sodium sulfate. This was concentrated under a reduced pressure to give 0.82 g of the title compound (yield = 99%, optical purity: 90% ee). The optical yield was 100%.

The resulting title compound showed a specific angle of rotation of $[\alpha]_D^{22.0}$ = -54.7° (C = 0.980, $CHCl_3$).

Example 3-32

Synthesis of SBOA

A 26.8 g (0.386 mol) portion of hydroxylamine hydrochloride was added to a mixed solution of 46 g (0.434 mol) of sodium carbonate, 25 ml of ethanol and 25 ml of water, and the mixture was stirred at 35°C for 20 minutes. Then, 44 g (0.431 mol) of acetic anhydride was slowly added dropwise, spending 3 hours. Then, 35 ml of ethanol was added, and the mixture was stirred for 2 hours at 40°C and then for 1 hour at 50°C. After cooling to 15°C, 28.3 g (0.48 mol) of 95% potassium hydroxide and 0.5 g (1.55 mmol) of tetra-n-butylammonium bromide were added under ice-cooling. At 45°C, 41.7 g (0.192 mol, optical purity: 90% ee) of (R)-1-(3-trifluoromethylphenyl)-1-ethyl chloride which has been dissolved in 15 ml of ethanol was added dropwise. After 6 hours of stirring at 70°C and subsequent cooling to 20°C, 115 ml (1.32 mol) of concentrated hydrochloric acid was added dropwise to the reaction mixture under ice-cooling, followed by 2 hours of stirring at 50°C. After cooling and subsequent addition of 50 g of ice, this was neutralized by adding 36 g (0.891 mol) of 99% sodium hydroxide at 30°C or less. A 100 ml portion of n-hexane was added to effect separation, and the resulting organic layer was washed with water and saturated brine in that order. After drying over anhydrous sodium sulfate, this was concentrated under a reduced pressure, and the resulting residue was purified by distillation under a reduced pressure (boiling point; 71.5 - 73°C/4 mmHg) to give 30.7 g of the title compound (yield = 74.1%, optical purity: 86% ee). The optical yield was 95.6%.

The resulting title compound showed a specific angle of rotation of $[\alpha]_D^{22.0}$ = -52.3° (C = 0.870, $CHCl_3$).

Example 3-33

Synthesis of SBOA

A 1.1 g (7.2 mmol) portion of 1,8-diazabicyclo[5,4,0]undec-ene (DBU) was added to a toluene solution containing 1.3 g (6.2 mmol, optical purity: 78.2% ee) of (R)-1-(3-trifluoromethylphenyl)-1-ethyl chloride and 0.9 g (12.9 mmol) of acetohydroxamic acid, and the mixture was stirred at 60°C for 6 hours. After cooling, 3 ml (34.4 mmol) of concentrated hydrochloric acid was added dropwise to the reaction mixture under ice-cooling, and the mixture was heated to 50°C and stirred for 2 hours. After cooling, this was neutralized with sodium hydroxide and 30 ml of n-hexane was added to effect separation. The resulting organic layer was washed with water and saturated brine in that order. After drying over anhydrous sodium sulfate, this was concentrated under a reduced pressure, and the resulting residue was purified by a silica gel column chromatography (n-Hex:AcOEt = 4:1) to give 1.0 g of the title compound (yield = 80%, optical purity: 72.2% ee). The optical yield was 92.3%.

Example 3-34

Synthesis of SBOA

A 1.1 g (7.2 mmol) portion of DBU was added to a mixed solution of 6 ml of ethanol and 2 ml of water, containing 1.3 g (6.2 mmol, optical purity: 75.5% ee) of (R)-1-(3-trifluoromethylphenyl)-1-ethyl chloride and 0.9 g (12.9 mmol) of acetohydroxamic acid, and the mixture was stirred at 60°C for 4 hours. After cooling, 3 ml (34.4 mmol) of concentrated hydrochloric acid was added dropwise to the reaction mixture under ice-cooling, and the mixture was heated to 50°C and stirred for 2 hours. After cooling, this was neutralized with sodium hydroxide and n-hexane (30 ml) was added to effect separation. The resulting organic layer was washed with water and saturated brine in that order. After drying over anhydrous sodium sulfate, this was concentrated under a reduced pressure, and the resulting residue was purified by a silica gel column chromatography (n-Hex:AcOEt = 4:1) to give 1.1 g of the title compound (yield = 88%, optical purity: 71.7% ee). The optical yield was 94.9%.

Example 3-35

Synthesis of SBOA

The procedure of Example 3-34 was repeated, except that DBU was changed to triethylamine, thereby obtaining 0.72 g of the title compound (yield = 56.4%, optical purity: 68.8% ee). The optical yield was 91.1%.

Example 3-36

Synthesis of SBOA

Potassium carbonate was added to a mixed solution of 1.55 ml (15.9 mmol) of ethyl acetate, 4 ml of methanol and 2 ml of water, containing 867 mg (10.56 mmol) of hydroxylamine sulfate, under ice-cooling, and the resulting mixture was stirred at room temperature for 12 hours. A 1.11 g (5.30 mmol, optical purity: 87% ee) portion of (R)-1-(3-trifluoromethylphenyl)-1-ethyl chloride and 10 mg (0.031 mmol) of tetra-n-butylammonium bromide were added to the reaction mixture under ice-cooling, and the mixture was stirred at 70°C for 6 hours. After cooling to 20°C, 3.0 ml (36.5 mmol) of concentrated hydrochloric acid was added dropwise to the reaction mixture, and the mixture was heated to 50°C and stirred for 1 hour. After cooling, this was neutralized by adding 1.4 g (35 mmol) of 99% sodium hydroxide at 30°C or less. Then, 20 ml of n-hexane was added to effect separation, and the resulting organic layer was washed with water and saturated brine in that order. After drying over anhydrous sodium sulfate, this was concentrated under a reduced pressure, and the resulting residue was purified by a kugelrohr to give 0.89 g of the title compound (yield = 82%, optical purity: 84% ee). The optical yield was 97%.

Example 3-37

Synthesis of (S)-t-butyl-N-{1-(3-trifluoromethylphenyl)ethoxy}carbamate

A 1 ml portion of ethanol solution containing 217 mg (3.6 mmol) of potassium hydroxide, 97 mg (0.3 mmol) tetra-n-butylammonium bromide and 843 mg (3.0 mmol, optical purity: 87% ee) of (R)-1-(3-trifluoromethylphenyl)-1-ethyl chloride was added at 0°C to 5 ml of ethanol solution containing 799 mg (6.0 mmol) of t-butyl-N-hydroxycarbamate, and the mixture was stirred at 70°C for 5 hours. Diisopropyl ether was added to the reaction mixture, and the mixture was washed with water and saturated brine in that order, dried over anhydrous sodium sulfate and then concentrated under a reduced pressure. The resulting residue was purified by a silica gel column chromatography (n-hexane:ethyl acetate = 20:1 → 0:1) to give 708 mg of (S)-t-butyl-N-{1-(3-trifluoromethylphenyl)ethoxy}carbamate (yield = 77%, optical purity: 84% ee). The optical yield was 97%. Analytical data of the product were as follows.

$^1$H-NMR δ ($CDCl_3$); 1.46 (9H, s), 1.54 (3H, d), 4.97 (1H, q), 6.96 (1H, brs), 7.48 - 7.62 (4H, m)

Example 3-38

Synthesis of SBOA

A 1 ml portion of ethanol solution containing 217 mg (3.6 mmol) of potassium hydroxide, 97 mg (0.3 mmol) tetra-n-butylammonium bromide and 843 mg (3.0 mmol, optical purity: 87% ee) of (R)-1-(3-trifluoromethylphenyl)-1-ethyl chloride was added at 0°C to 5 ml of ethanol solution containing 799 mg (6.0 mmol) of t-butyl-N-hydroxycarbamate, and the mixture was stirred at 70°C for 5 hours. After allowing to stand overnight at room temperature, 0.87 ml of concentrated hydrochloric acid was added at 0°C to the reaction mixture, and the resulting mixture was heated to 50°C and stirred for 2.5 hours. Then, n-hexane was added to the reaction solution, and the mixture was separated with water. Then, 400 mg of sodium hydroxide was added to the water layer, and the mixture was extracted again with n-hexane. The organic layers were combined, washed with water and saturated brine in that order, dried over anhydrous sodium sulfate and then concentrated under a reduced pressure to give the title compound. The yield was 77% when analyzed by a gas chromatography, and the optical purity was 84% ee and the optical yield was 97% when analyzed by a high performance liquid chromatography.

Examples 3-39 to 3-53

Examples of optically active 1-(hetero)arylethoxyamine derivatives obtained in the same manner as described in Example 3-32, except for changing the starting materials, are shown in Table 8.

Table 8

| $H_2NOCH^*(CH_3)$-Ar-Yn | | |
|---|---|---|
| Exam ple | Ar-Yn | Yield (%), optical purity (% ee) and optical yield (%) from 1-(hetero)arylethyl chloride derivative |
| 3-39 | Phenyl | 63%, 85% ee, 97% |
| 3-40 | 4-t-Bu-Phenyl | 77%, 89% ee, 97% |
| 3-41 | 4-Cl-Phenyl | 70%, 87% ee, 98% |
| 3-42 | 4-$CF_3$-Phenyl | 62%, 90% ee, 99% |
| 3-43 | 3-Cl-Phenyl | 60%, 80% ee, 95% |
| 3-44 | 4-CH-Phenyl | 49%, 82% ee, 90% |
| 3-45 | 3-$OCF_3$-Phenyl | 79%, 93% ee, 96% |
| 3-46 | 4-SMe-Phenyl | 46%, 60% ee, 92% |
| 3-47 | 4-$OCF_3$-Phenyl | 63%, 86% ee, 98% |
| 3-48 | 4-O-Propargyl-Phenyl | 65%, 81% ee, 94% |
| 3-49 | 3-OMe-4-$OCHF_2$-Phenyl | 68%, 85% ee, 96% |
| 3-50 | 4-$NO_2$-Phenyl | 55%, 77% ee, 94% |
| 3-51 | 2-Naphthyl | 61%, 76% ee, 92% |
| 3-52 | 1-Benzodioxol-5-yl | 65%, 73% ee, 91% |
| 3-53 | 2-Chloro-thiophen-5-yl | 60%, 84% ee, 95% |

The optical purity of the optically active 1-arylethoxyamine derivative (II) obtained in the aforementioned manner can be improved further in combination with optical resolution. Such a method is described with reference to a compound in which Ar-Yn of the general formula (II) is 3-trifluoromethylphenyl.

Example 3-54

Synthesis of (S)-1-(3-trifluoromethylphenyl)ethoxyamine • L-tartarate (to be referred to as SBOA • LTA hereinafter)

A 4.10 g (SBOA; 20 mmol) portion of (S)-1-(3-trifluoromethylphenyl)ethoxyamine having an optical purity of 91.4% ee, 1.44 g (9.6 mmol) of L-tartaric acid, 24 ml of tetrahydrofuran (THF) and 4 ml of diisopropyl ether (i-$Pr_2O$) were charged in a flask and, using an oil bath, heated under reflux until the starting materials were completely dissolved. Thereafter, the oil bath was removed, and the resulting solution was cooled to room temperature while stirring. The thus precipitated crystals were collected by filtration and dried, thereby obtaining 4.88 g of the title compound (optical purity:

99% ee or more).

Example 3-55

Synthesis of SBOA • LTA

A 2.00 g (9.7 mmol) portion of SBOA having an optical purity of 77.3% ee, 1.44 g (9.6 mmol) of L-tartaric acid, 10 ml of tetrahydrofuran and 4 ml of di-n-butyl ether (n-$Bu_2O$) were charged in a flask and, using an oil bath, heated under reflux until the materials were completely dissolved. Thereafter, the oil bath was removed, and the resulting solution was cooled to room temperature while stirring. The thus precipitated crystals were collected by filtration and dried, thereby obtaining 2.50 g of the title compound (optical purity: 97.8% ee).

Example 3-56

Synthesis of SBOA • LTA

The procedure of Example 3-55 was repeated, except that the solution was cooled to room temperature and then further ice-cooled while stirring, thereby obtaining 2.80 g of the title compound (optical purity: 91.1% ee).

Examples 3-57 to 3-62

Synthesis of SBOA • LTA

The title compound was obtained by repeating the procedure of Example 3-55, except that kinds and amounts of the solvent to be used were changed. The conditions and results are shown in Table 9.

Table 9

| Example | SBOA (g) | SBOA (ee%) | Solvent | Cooling temp. (°C) | SBOA • LTA (g) | SBOA • LTA (ee%) |
|---|---|---|---|---|---|---|
| 3-54 | 4.10 | 91.4 | THF 24 ml<br>i-$Pr_2O$ 4 ml | room temp. | 4.88 | 99< |
| 3-55 | 2.00 | 77.3 | THF 10 ml<br>n-$Bu_2O$ 2 ml | room temp. | 2.50 | 97.8 |
| 3-56 | 2.00 | 77.3 | THF 10 ml<br>n-$Bu_2O$ 2 ml | 0 | 2.80 | 91.1 |
| 3-57 | 2.00 | 77.3 | THF 10 ml<br>Xylene 2 ml | 0 | 2.80 | 96.7 |
| 3-58 | 2.00 | 77.3 | n-BuOH 6 ml | room temp. | 2.70 | 96.3 |
| 3-59 | 2.00 | 77.3 | n-BuOH 5 ml | 0 | 2.98 | 91.6 |
| 3-60 | 2.00 | 79.5 | n-BuOH 4.5 m<br>i-Octane 2 ml | 0 | 2.82 | 94.4 |
| 3-61 | 2.00 | 79.5 | n-Pentanol 5 ml<br>i-Octane 2 ml | 0 | 1.24 | 99< |
| 3-62 | 2.00 | 79.5 | n-Hexanol 5 ml<br>i-Octane 1 ml | 0 | 1.56 | 99< |

Example 3-63

Synthesis of SBOA • LTA

A 10.0 g portion of SBOA (optical purity; 79.5% ee), 7.20 g of L-tartaric acid, 50 ml of THF and 30 ml of xylene were charged in a flask and, using an oil bath, heated under reflux until the materials were completely dissolved. Thereafter, THF was completely evaporated under ordinary pressure. The oil bath was removed, and the resulting solution was spontaneously cooled to room temperature while stirring. The thus precipitated crystals were collected by filtration and dried, thereby obtaining 15.26 g of the title compound (optical purity: 98% ee).

A method in which the optically active 1-arylethyl halide derivative (VIII) is obtained by halogenating the optically active 1-arylethylalcohol derivative (XI) is described with reference to a compound of the general formula (XI) and (VIII) in which Ar is phenyl and Yn is 3-trifluoromethyl.

Example 4-1

A 0.96 g portion of (R)-1-(3-trifluoromethylphenyl)ethanol (100% ee) was dissolved in 5.0 ml of pyridine to which, ice-cooled, was subsequently added dropwise 0.8 ml of methanesulfonyl chloride. After completion of the dropwise addition, 24 hours of reaction was carried out at room temperature. Then, 10 ml of 1 N hydrochloric acid aqueous solution was added to the reaction mixture, and the mixture was extracted with 10 ml of diethyl ether. The resulting organic layer was washed with 10 ml of saturated sodium bicarbonate aqueous solution and 10 ml of water. The organic layer was dried over anhydrous sodium sulfate, and the solvent was evaporated under a reduced pressure to give 0.77 g of (S)-1-(3-trifluoromethylphenyl)ethyl chloride. Physiological properties of this compound were as follows.

$[\alpha]_D^{24}$; +53.7° (c = 0.93, $CHCl_3$)
$^1$H-NMR (300 MHz, $CDCl_3$, TMS) $\delta$; 1.86 (d, 3 H), 5.12 (q, 1 H), 7.44 - 7.65 (m, 3 H), 7.67 (brs, 1 H)

The yield was 73% when analyzed by a gas chromatography, and the optical purity was 93% ee when measured by a high performance liquid chromatography.

Example 4-2

The reaction of Example 4-1 was repeated, except that the reaction temperature was changed from room temperature to 45°C. The yield of (S)-1-(3-trifluoromethylphenyl)ethyl chloride was 78% when analyzed by a gas chromatography, and the optical purity was 79% ee when measured by a high performance liquid chromatography.

Example 4-3

The reaction of Example 4-1 was repeated, except that the reaction was carried out at 60°C for 20 hours. The yield of (S)-1-(3-trifluoromethylphenyl)ethyl chloride was 75% when analyzed by a gas chromatography, and the optical purity was 73% ee when measured by a high performance liquid chromatography.

Example 4-4

A 0.96 g portion of (R)-1-(3-trifluoromethylphenyl)ethanol (100% ee), 0.45 ml of pyridine and 0.24 g of lithium chloride were dissolved in 4.5 ml of N,N-dimethylformamide to which, ice-cooled, was subsequently added dropwise 0.43 ml of methanesulfonyl chloride. After completion of the dropwise addition, 24 hours of reaction was carried out at room temperature. Then, 10 ml of 1 N hydrochloric acid aqueous solution was added to the reaction mixture, and the mixture was extracted with 10 ml of diethyl ether. The resulting organic layer was washed with 10 ml of saturated sodium bicarbonate aqueous solution and 10 ml of water. The organic layer was dried over anhydrous sodium sulfate, and the solvent was evaporated under a reduced pressure to give 0.88 g of (S)-1-(3-trifluoromethylphenyl)-1-ethyl chloride. The yield of (S)-1-(3-trifluoromethylphenyl)ethyl chloride was 75% when analyzed by a gas chromatography, and the optical purity was 88% ee when measured by a high performance liquid chromatography.

Example 4-5

The reaction of Example 4-4 was repeated, except that the reaction was carried out at room temperature for 3 hours without adding lithium chloride. The yield of (S)-1-(3-trifluoromethylphenyl)ethyl chloride was 79% when analyzed by a gas chromatography, and the optical purity was 94% ee when measured by a high performance liquid chromatography.

Example 4-6

The reaction of Example 4-4 was repeated, except that 1.07 g of p-toluenesulfonyl chloride was added instead of methanesulfonyl chloride. The yield of (S)-1-(3-trifluoromethylphenyl)ethyl chloride was 56% when analyzed by a gas chromatography, and the optical purity was 85% ee when measured by a high performance liquid chromatography.

Example 4-7

A 0.97 g portion of (R)-1-(3-trifluoromethylphenyl)ethanol (100% ee) and 1.4 ml of triethylamine were dissolved in 5.0 ml of dichloromethane to which, ice-cooled, was subsequently added dropwise 0.58 ml of methanesulfonyl chloride. After completion of the dropwise addition, 5 hours of reaction was carried out at room temperature. Then, 10 ml of water was added to the reaction solution, and the mixture was extracted with 10 ml of dichloromethane. The resulting organic layer was washed with 10 ml of saturated brine. The organic layer was dried over anhydrous sodium sulfate, and the solvent was evaporated under a reduced pressure to give 1.34 g of (R)-α-methanesulfonyloxy-3-trifluoromethylethylbenzene. Physiological properties of this compound were as follows.

$[\alpha]_D^{24}$; -62.5° (c = 1.02, $CHCl_3$)
$^1$H-NMR (300 MHz, $CDCl_3$, TMS) δ; 1.75 (d, 3 H), 2.85 (s, 3 H), 5.80 (q, 1 H), 7.50 - 7.67 (m, 4 H)

Next, 0.94 g of (R)-α-methanesulfonyloxy-3-trifluoromethylethylbenzene was dissolved in 1.5 ml of N,N-dimethylformamide to which, under ice-cooling, was subsequently added dropwise 3 ml of N,N-dimethylformamide solution containing 195 mg of lithium chloride. After completion of the dropwise addition, the reaction was carried out at room temperature for 3 hours. The yield of (S)-1-(3-trifluoromethylphenyl)ethyl chloride was 84% when analyzed by a gas chromatography, and the optical purity was 95% ee when measured by a high performance liquid chromatography.

Example 4-8

The reaction of Example 4-4 was repeated, except that 0.77 ml of triethylamine was added instead of pyridine, and the reaction was carried out at room temperature for 3 hours. The yield of (S)-1-(3-trifluoromethylphenyl)ethyl chloride was 87% when analyzed by a gas chromatography, and the optical purity was 88% ee when measured by a high performance liquid chromatography.

Example 4-9

The reaction of Example 4-4 was repeated, except that 0.41 g of potassium chloride was added instead of 1.0 g of lithium chloride. The yield of (S)-1-(3-trifluoromethylphenyl)ethyl chloride was 80% when analyzed by a gas chromatography, and the optical purity was 94% ee when measured by a high performance liquid chromatography.

Example 4-10

The reaction of Example 4-4 was repeated, except that 4.5 ml of tetrahydrofuran was added instead of 4.5 ml of N,N-dimethylformamide. The yield of (S)-1-(3-trifluoromethylphenyl)ethyl chloride was 22% when analyzed by a gas chromatography, and the optical purity was 93% ee when measured by a high performance liquid chromatography.

Example 4-11

The reaction of Example 4-4 was repeated, except that 4.5 ml of chloroform was added instead of 4.5 ml of N,N-dimethylformamide. The yield of (S)-1-(3-trifluoromethylphenyl)ethyl chloride was 35% when analyzed by a gas chromatography, and the optical purity was 90% ee when measured by a high performance liquid chromatography.

Example 4-12

The reaction of Example 4-4 was repeated, except that 0.39 g of potassium carbonate was added instead of 0.45 ml of pyridine. The yield of (S)-1-(3-trifluoromethylphenyl)ethyl chloride was 44% when analyzed by a gas chromatography, and the optical purity was 87% ee when measured by a high performance liquid chromatography.

Example 4-13

The reaction of Example 4-10 was repeated, except that 0.41 ml of thionyl chloride was added instead of 0.43 ml of methanesulfonyl chloride. The yield of (S)-1-(3-trifluoromethylphenyl)ethyl chloride was 46% when analyzed by a gas chromatography, and the optical purity was 41% ee when measured by a high performance liquid chromatography.

Example 4-14

A 1.0 g portion of (S)-1-(3-trifluoromethylphenyl)ethanol (100% ee), 0.563 g of 4-picoline and 4.5 ml of N,N-dimethylformamide were put into a flask and cooled to 0°C. Then, 0.663 g of methanesulfonyl chloride was added dropwise. After 30 minutes of stirring at 0°C, 24 hours of reaction was carried out at room temperature. When the reaction solution was analyzed by a gas chromatography, it was found that (R)-1-(3-trifluoromethylphenyl)-1-ethyl chloride was formed with a yield of 85%. The optical purity was 80.0% ee when measured by a high performance liquid chromatography.

Example 4-15

A 1.0 g portion of (S)-1-(3-trifluoromethylphenyl)ethanol (100% ee), 0.478 g of pyridine, 0.1 ml of N,N-dimethylformamide and 4.9 ml of t-butyl methyl ether were put into a flask and cooled to 0°C. Then, 0.688 g of thionyl chloride was added dropwise. After 30 minutes of stirring at 0°C, 8 hours of reaction was carried out at room temperature. When the reaction solution was analyzed by a gas chromatography, it was found that (R)-1-(3-trifluoromethylphenyl)-1-ethyl chloride was formed with a yield of 87%. The optical purity was 74% ee when measured by a high performance liquid chromatography.

Example 4-16

A 1.0 g portion of (S)-1-(3-trifluoromethylphenyl)ethanol (100% ee), 0.563 g of 4-picoline, 0.1 ml of N,N-dimethylformamide and 4.9 ml of n-heptane were put into a flask and cooled to 0°C, to which was subsequently added dropwise 0.688 g of thionyl chloride. Thereafter, 3 hours of reaction was carried out at 0°C. When the reaction solution was analyzed by a gas chromatography, it was found that (R)-1-(3-trifluoromethylphenyl)-1-ethyl chloride was formed with a yield of 83%. The optical purity was 77.0% ee when measured by a high performance liquid chromatography.

The results of the above experiments are shown in the following Table 10.

Table 10

| Example | Halogenating agent (eq. *1) | Base (eq. *1) | Hydrohalogenate (eq. *1) | Solvent (ml) | Reaction temp. (°C) | Reaction time (hr) | Yield (%) | Optical purity (%ee) |
|---|---|---|---|---|---|---|---|---|
| 4-1 | MsCl 2.0 | Py 12.0 | - | - | room temp. | 24 | 73 | 93 |
| 4-2 | MsCl 2.0 | Py 12.0 | - | - | 45 | 24 | 78 | 79 |
| 4-3 | MsCl 2.0 | Py 12.0 | - | - | 60 | 1.5 | 75 | 73 |
| 4-4 | MsCl 1.1 | Py 1.1 | LiCl 1.1 | DMF 4.5 | room temp. | 24 | 75 | 88 |
| 4-5 | MsCl 1.1 | Py 1.1 | - | DMF 4.5 | room temp. | 3 | 79 | 94 |
| 4-6 | TsCl 1.1 | Py 1.1 | LiCl 1.1 | DMF 4.5 | room temp. | 24 | 56 | 85 |
| 4-8 | MsCl 1.1 | $Et_3N$ 1.1 | LiCl 1.1 | DMF 4.5 | room temp. | 3 | 87 | 88 |
| 4-9 | MsCl 1.1 | Py 1.1 | LiCl 1.1 | DMF 4.5 | room temp. | 5 | 80 | 94 |
| 4-10 | MsCl 1.1 | Py 1.1 | LiCl 1.1 | THF 4.5 | room temp. | 24 | 22 | 93 |
| 4-11 | MsCl 1.1 | Py 1.1 | LiCl 1.1 | $CHCl_3$ 4.5 | room temp. | 24 | 35 | 90 |
| 4-12 | MsCl 1.1 | $K_2CO_3$ 1.1 | LiCl 1.1 | DMF 4.5 | room temp. | 24 | 44 | 87 |
| 4-13 | $SOCl_2$ 1.1 | Py 1.1 | LiCl 1.1 | DMF 4.5 | room temp. | 3.5 | 46 | 41 |
| 4-14 | MsCl 1.1 | 4-picoline 1.1 | - | DMF 4.5 | room temp. | 24 | 85 | 80 |
| 4-15 | $SOCl_2$ 1.1 | Py 1.1 | - | DMF 0.1<br>t-BuOMe 4.9 | room temp. | 8 | 87 | 74 |
| 4-16 | $SOCl_2$ 1.1 | 4-picoline 1.1 | - | DMF 0.1<br>n-heptane 4.9 | 0 | 3 | 83 | 77 |

*1 Equivalents based on (R)-1-(3-trifluoromethylphenyl)ethanol.

*2 Reaction temperature after addition of the halogenating agent.

MsCl = methanesulfonyl chloride

TsCl = p-toluenesulfonyl chloride

Py = pyridine

DMF = N,N-dimethylformamide

t-BuOMe = t-butyl methyl ether

THF = tetrahydrofuran

The above results suggested that pyridine, picoline and triethylamine were desirable as the base, and pyridine and 4-picoline were particularly effective in proceeding the reaction even in the absence of the hydrohalogenate. As the halogenating agent, methanesulfonyl chloride was particularly excellent, but the reaction also proceeded efficiently even by the use of thionyl chloride when a mixed solvent containing N,N-dimethylformamide was used.

Next, the reaction-acceleration effect of mixed solvents was examined in a case in which methanesulfonyl chloride was used as the halogenating agent, and pyridine as the base.

Example 4-17

A 1.0 g portion of (S)-1-(3-trifluoromethylphenyl)ethanol (100% ee), 0.478 g of pyridine, 1.5 ml of N,N-dimethylformamide and 3.5 ml of t-butyl methyl ether were put into a flask and cooled to 0°C. Then, 0.663 g of methanesulfonyl chloride was added dropwise. After 30 minutes of stirring at 0°C, 12 hours of reaction was carried out at room temperature. When the reaction solution was analyzed by a gas chromatography, it was found that (R)-1-(3-trifluoromethylphenyl)-1-ethyl chloride was formed with a yield of 97%. The optical purity was 95.6% ee when measured by a high performance liquid chromatography.

Examples 4-18 to 4-20

The procedure of Example 4-17 was repeated, except that amounts of N,N-dimethylformamide and t-butyl methyl ether were changed. The results are shown in the following Table 10.

Example 4-21

The procedure of Example 4-17 was repeated, except that t-butyl methyl ether was changed to diisopropyl ether (i-$Pr_2O$) and the reaction was carried out for 3 hours. The results are shown in the following Table 10.

Examples 4-22 to 4-24

The procedures of Examples 4-18 to 4-20 were repeated, except that t-butyl methyl ether was changed to n-heptane and the reaction was carried out for 3 hours. The results are shown in the following Table 10.

Examples 4-25 and 4-26

The procedures of Examples 23 and 24 were repeated, except that 2.0 g of (S)-1-(3-trifluoromethylphenyl)ethanol and 0.956 g of pyridine were used. The results are shown in the following Table 11.

Table 11

| Example | (S)BA (g) | Solvent | Reaction time (hr) | (S)BA (%) | (R)BC (%) | (R)BC (ee%) |
|---|---|---|---|---|---|---|
| 4-17 | 1.0 | DMF 1.5 ml<br>t-BuOMe 3.5 ml | 12.0 | 0 | 97 | 95.6 |
| 4-18 | 1.0 | DMF 1.0 ml<br>t-BuOMe 4.0 ml | 8.0 | 25 | 71 | 95.3 |
| 4-19 | 1.0 | DMF 0.5 ml<br>t-BuOMe 4.5 ml | 8.0 | 54 | 43 | 95.5 |
| 4-20 | 1.0 | DMF 0.1 ml<br>t-BuOMe 4.9 ml | 8.0 | 90 | 70 | - |
| 4-21 | 1.0 | DMF 1.5 ml<br>i-$Pr_2O$ 3.5 ml | 3.0 | 26 | 72 | 95.4 |
| 4-22 | 1.0 | DMF 1.5 ml<br>n-Heptane 3.5 ml | 3.0 | 0.4 | 95 | 95.5 |
| 4-23 | 1.0 | DMF 1.0 ml<br>n-Heptane 4.0 ml | 3.0 | 0.4 | 96 | 95.5 |
| 4-24 | 1.0 | DMF 0.5 ml<br>n-Heptane 4.5 ml | 3.0 | 0 | 97 | 95.6 |
| 4-25 | 2.0 | DMF 1.0 ml<br>n-Heptane 4.0 ml | 3.0 | 0 | 97 | 95.9 |
| 4-26 | 2.0 | DMF 0.5 ml<br>n-Heptane 4.5 ml | 3.0 | 0 | 97 | 96.0 |
| (S)BA = (S)-1-(3-trifluoromethyl)ethanol<br>(R)BC = (R)-1-(3-trifluoromethyl)ethyl chloride | | | | | | |

The following describes a method in which the optically active 1-arylethylalcohol derivative (XI) is obtained by subjecting the acetophenone derivative (XIII) to asymmetric reduction. In the following production examples, S/C indicates molar ratio of the starting material to a catalyst (Ru).

Example 5-1

Synthesis of (R)-1-(3-trifluoromethylphenyl)ethanol

The atmosphere in a Schlenk tube was replaced by argon, and 1.0 ml of 2-propanol, 6.2 mg of $[RuCl_2(p\text{-}cymene)]_2$ and 14.7 mg of (1R,2R)-N-(p-toluenesulfonyl)-1,2-diphenylethylenediamine were put into the tube. After 1 hour of heating under reflux, 38 ml of 2-propanol, 0.753 g of m-trifluoromethylacetophenone and 1.0 ml of 0.1 M potassium hydroxide 2-propanol solution were added, and the mixture was stirred at 0°C for 24 hours (S/C = 200, reaction substrate concentration = 0.1 mol/l). Analysis of the reaction solution by a high performance liquid chromatography revealed that the yield was 98.7%, the optical purity was 93.0% ee, and the R isomer was obtained in an excess amount.

Examples 5-2 to 5-4

Synthesis of (R)-1-(3-trifluoromethylphenyl)ethanol

In these examples, the S/C ratio and the reaction substrate concentration were changed, by fixing the reaction

temperature to room temperature and the reaction time to 5 hours, with the results shown in Table 12.

Table 12

| Example | S/C | Reaction substrate conc. (mol/l) | Reaction temp. (°C) | Reaction time (hr) | Yield (%) | Optical purity (%ee) |
|---|---|---|---|---|---|---|
| 5-2 | 200 | 0.1 | room temp. | 5 | 97.4 | 90.6 |
| 5-3 | 200 | 1.0 | room temp. | 5 | 93.7 | 88.3 |
| 5-4 | 1000 | 1.0 | room temp. | 5 | 80.7 | 89.7 |

Example 5-5

Synthesis of (R)-1-(3-trifluoromethylphenyl)ethanol

The atmosphere in a Schlenk tube was replaced by argon, and 1.0 ml of 2-propanol, 7.7 mg of $[RuCl_2(benzene)]_2$, 9.2 mg of (1R,2R)-N-(p-toluenesulfonyl)-1,2-diphenylethylenediamine and 7.0 μl of triethylamine were put into the tube. After 1 hour of heating under reflux, 2-propanol was evaporated. Then, 2.5 ml of an azeotropic mixture of formic acid and triethylamine, which was prepared independently, and 0.762 ml of m-trifluoromethylacetophenone were added, and the mixture was stirred at room temperature (S/C = 200, reaction substrate concentration = 2.0 mol/l). Analysis of the reaction solution 24 hours thereafter by a high performance liquid chromatography revealed that the yield was 99%, the optical purity was 93.5% ee, and the R isomer was obtained in an excess amount.

Examples 5-6

Synthesis of (R)-1-phenylethanol

The reaction of Example 5-3 was repeated, except that acetophenone was used as the starting material. When the reaction was carried out at room temperature for 6 hours, 1-phenylethanol was obtained with a yield of 65.4% and an optical purity of 95.7% ee.
Periodical changes in these values were compared with the case of m-trifluoromethyl derivative, with the results shown in Fig. 1 and Fig. 2.

Example 5-7

Synthesis of (R)-1-(3-trifluoromethylphenyl)ethanol

A 25 ml capacity three neck flask equipped with a dropping funnel, a thermometer and a distillation column having a solvent receiver on its top was charged with 13.4 mg (21.8 μM) of $[RuCl_2(p\text{-cymene})]_2$, 34.0 mg (92.8 μM) of (1R,2R)-N-(p-toluenesulfonyl)-1,2-diphenylethylenediamine and 1.1 ml of 2-propanol, and the mixture was heated under reflux for 1 hour. After cooling to room temperature, 1.75 g (9.3 mM) of m-trifluoromethylacetophenone and 16.9 ml of 2-propanol were added, and then 2.3 ml of 0.1 mol/L 2-propanol solution of potassium hydroxide (230 μM) was added. The reactor was arranged in a constant temperature water bath, and the reaction was started under an initial pressure of 100 mmHg and a water bath temperature of 25°C. The thus formed acetone was evaporated by gradually reducing the pressure to 40 mmHg spending 4 hours. In the course of the reaction, 6 ml of 2-propanol was added. The reaction after 4 hours was terminated by adding 2.3 ml of 0.1 N hydrochloric acid at ordinary temperature under atmospheric pressure. This was extracted with ethyl acetate and analyzed by a high performance liquid chromatography. The reaction yield was 99.5%, the optical purity was 90.3% ee, and the R isomer was obtained in an excess amount.
In this case, a relationship between reaction time and yield is shown in Fig. 3, and a relationship between reaction time and optical purity is shown in Fig. 4.

Example 5-8

The reaction of Example 5-7 was repeated, except that the reaction was carried out under atmospheric pressure, thereby obtaining a reaction yield of 92.8% and an optical purity of 88.4% ee after 4 hours of the reaction.
In this case, a relationship between reaction time and yield is shown in Fig. 3, and a relationship between reaction time and optical purity is shown in Fig. 4.

Example 5-9

Synthesis of (S)-1-(3-trifluoromethylphenyl)ethanol

The title compound was obtained in the same manner as described in Example 5-7, except that (1S,2S)-N-(p-toluenesulfonyl)-1,2-diphenylethylenediamine was used as the optically active nitrogen-containing compound. The reaction yield was 99.3%, and the optical purity was 90.6% ee.

The following describes a method in which the acetophenone derivative (XIII) is obtained by effecting diazotation of the aniline derivative (XIV), with reference to compounds of the general formulae (XIV) and (XIII) in which Y is trifluoromethyl group and n is 1 or 2.

Example 6-1

A 20 g (124 mmol) portion of m-aminobenzotrifluoride was added dropwise to dilute hydrochloric acid which has been prepared from 32 g (307 mmol) of concentrated hydrochloric acid and 60 ml of water. At -5°C, 15 ml of aqueous solution containing 9.4 g (136 mmol) of sodium nitrite was added dropwise, spending 10 minutes, followed by 20 minutes of stirring. By adding thereto 0.5 g of sulfamic acid, a diazonium hydrochloric acid solution was prepared.

A 6.6 g (41 mmol) portion of copper sulfate and 90 g (662 mmol) of sodium acetate trihydrate were added to 40 ml of aqueous solution containing 14.8 g (250 mmol) of acetaldoxime. Then, the previously prepared diazonium hydrochloric acid solution was subsequently added dropwise at 0 to 10°C. After 1.5 hours of stirring at 10 to 15°C, 55 ml of concentrated hydrochloric acid was added and the mixture was heated under reflux for 2 hours. After cooling, this was extracted with n-hexane, and the resulting organic layer was washed with water and saturated brine in that order. After drying with anhydrous sodium sulfate, the organic layer was distilled under a reduced pressure to obtain 14.5 g of 3-trifluoromethylacetophenone (to be referred to as MTFA hereinafter) (yield = 62%, b.p. 95 - 99°C/23 mmHg).

Example 6-2

A 20 g (124 mmol) portion of m-aminobenzotrifluoride was added dropwise to dilute hydrochloric acid which has been prepared from 32 g (307 mmol) of concentrated hydrochloric acid and 60 ml of water. At -5°C, 15 ml of aqueous solution containing 9.4 g (136 mmol) of sodium nitrite was added dropwise, spending 10 minutes, followed by 20 minutes of stirring. By adding 0.5 g of sulfamic acid, a diazonium hydrochloric acid solution was prepared.

A 10.2 g (41 mmol) portion of copper sulfate pentahydrate and 90 g (662 mmol) of sodium acetate trihydrate were added to 40 ml of aqueous solution containing 14.8 g (250 mmol) of acetaldoxime. Then, the previously prepared diazonium hydrochloric acid solution was subsequently added dropwise at 0 to 10°C. After 1.5 hours of stirring at 10 to 15°C, this was extracted with toluene. The resulting organic layer was washed with water and saturated brine in that order and then dried with anhydrous sodium sulfate. After concentration under a reduced pressure, the resulting residue was purified by a silica gel column chromatography (n-Hex:AcOEt = 2:1 → 1:1) to obtain 15.1 g of 3-trifluoromethylacetophenone hydroxime (yield = 60%).

$^1$H-NMR δ ($CDCl_3$); 2.31 (3 H, s), 7.51 (1 H, t), 7.63 (1 H, d), 7.81 (1 H, d), 7.89 (1 H, s), 8.19 (1 H, s)

A 30 ml portion of 6 N hydrochloric acid was added to 30 ml of toluene solution containing 15.1 g (74.4 mmol) of 3-trifluoromethylacetophenone hydroxime, and the mixture was heated under reflux for 1.5 hours. After cooling, this was separated, and the resulting organic layer was washed with water and saturated brine in that order. After drying with anhydrous sodium sulfate, the organic layer was distilled under a reduced pressure to obtain 13.6 g of MTFA (yield = 58%).

Example 6-3

A 14 g (62 mmol) portion of 3,5-bis(trifluoromethyl)aniline was added dropwise to 80 ml of water containing 9.3 g (95 mmol) of concentrated sulfuric acid. At -5°C, 10 ml of aqueous solution containing 5.5 g (80 mmol) of sodium nitrite was added dropwise, spending 10 minutes, followed by 20 minutes of stirring. By adding thereto 0.5 g of sulfamic acid, a diazonium hydrochloric acid solution was prepared.

A 3.3 g (20.5 mmol) portion of copper sulfate and 62 g (756 mmol) of sodium acetate were added to 40 ml of aqueous solution containing 7.4 g (250 mmol) of acetaldoxime. Then, the previously prepared diazonium hydrochloric acid solution was subsequently added dropwise at -5 to 0°C. After 1.5 hours of stirring at 10 to 15°C, 50 ml of concentrated hydrochloric acid was added and the mixture was heated under reflux for 2 hours. After cooling, this was extracted with n-hexane, and the resulting organic layer was washed with water and saturated brine in that order. After

drying with anhydrous sodium sulfate, the organic layer was concentrated under a reduced pressure, and the resulting residue was purified by a silica gel column chromatography (n-Hex:AcOEt = 10:1) to obtain 8.1 g of 3,5-bis(trifluoromethyl)acetophenone (yield = 51%, $n_D^{21}$; 1.422).

Example 6-4

A 20 g (124 mmol) portion of m-aminobenzotrifluoride was added dropwise to dilute hydrochloric acid which has been prepared from 36 g (345 mmol) of concentrated hydrochloric acid and 60 ml of water. At -5°C, 15 ml of aqueous solution containing 9.4 g (136 mmol) of sodium nitrite was added dropwise, spending 10 minutes, followed by 20 minutes of stirring. Subsequently, 11 g (81 mmol) of sodium acetate trihydrate was added to prepare a diazonium hydrochloric acid solution.

A 6.2 g (24.8 mmol) portion of copper sulfate pentahydrate, 70 g (513 mmol) of sodium acetate trihydrate and 0.5 g (4 mmol) of sodium sulfite were added to 40 ml of aqueous solution containing 15 g (254 mmol) of acetaldoxime. The previously prepared diazonium hydrochloric acid solution was subsequently added dropwise at -5 to 0°C. When the pH value reached 5.5 during the dropwise addition, 140 g (103 mmol) of sodium acetate trihydrate was added. After 1.5 hours of stirring at 10 to 15°C, this was extracted with toluene. The resulting organic layer was washed with a hydrochloric acid aqueous solution and saturated brine in that order.

At 75°C, the hydroxime/toluene solution prepared above was added dropwise to a hydrochloric acid aqueous solution which has been prepared from 30 ml of concentrated hydrochloric acid and 30 ml of water. After 2 hours of stirring at 75°C, this was cooled and separated. The organic layer was washed with water and saturated brine in that order and then distilled under a reduced pressure to obtain 15.3 g of MTFA (yield = 65.5%).

Example 6-5

MTFA was produced in the same manner as described in Example 6-4, except that, in carrying out the coupling reaction by adding the diazonium hydrochloric acid solution to the acetaldoxime aqueous solution, the former solution was added dropwise at 0 to 5°C. The yield was 63%.

Comparative Example 6-1

MTFA was produced in the same manner as described in Example 6-4, except that, in carrying out the coupling reaction by adding the diazonium hydrochloric acid solution to the acetaldoxime aqueous solution, the former solution was added dropwise at 10 to 15°C. The yield was 45%.

Example 6-6

MTFA was produced in the same manner as described in Example 6-4, except that the charging amount of copper sulfate pentahydrate was changed from 6.2 g (24.8 mmol) to 4.7 g (18.6 mmol). The yield was 67%.

Example 6-7

MTFA was produced in the same manner as described in Example 6-4, except that the acetaldoxime aqueous solution was prepared by adding 11 g (250 mmol) of acetaldehyde to 40 ml of aqueous solution containing 21 g (128 mmol) of hydroxylamine sulfate and 35 g (257 mmol) of sodium acetate trihydrate and stirring the mixture at room temperature for 3 hours. The yield was 66%.

It was found from the results of Examples 6-4 and 6-5 and Comparative Example 6-1 that it is important to add the diazonium hydrochloric acid aqueous solution dropwise by keeping the temperature at 10°C or less in carrying out the coupling reaction. It was found also from the results of Examples 6-1, 6-4 and 6-6 that the reaction progresses smoothly when copper sulfate is used in an amount of from 0.1 to 0.35 equivalent, particularly from 0.15 to 0.2 equivalent, based on the aniline derivative. It was found from the results of Example 6-7 that the reaction progresses smoothly even if the acetaldoxime to be used is prepared by one pot reaction from hydroxylamine and acetaldehyde.

The following reveals usefulness of the thus obtained optically active methoxyiminoacetamide derivative (I) of the present invention as agricultural chemicals, with reference to its formulation examples and biological activity tests.

Formulation Example 1

A wettable powder was obtained by uniformly pulverizing and mixing 20% by weight of the compound of compound No. 2 shown in Table 1, 75% by weight of diatomaceous earth and 5% by weight of a surfactant mainly composed

of alkylbenzenesulfonic acid.

Formulation Example 2

An emulsifiable concentrate was obtained by mixing and dissolving 30% by weight of the compound of compound No. 3 shown in Table 1, 15% by weight of "Sorpol" 3005 X (trade name by Toho Chemical Industry, mixture of a nonionic surfactant and an anionic surfactant), 25% by weight of xylene and 30% by weight of dimethylformamide.
In the following test examples, compound numbers of racemates used as comparative compounds were defined as follows.

Racemic form of compound No. 1 and 2 = compound No. 2r
Racemic form of compound No. 3 = compound No. 3r
Racemic form of compound No. 4 = compound No. 4r
Racemic form of compound No. 5 = compound No. 5r
Racemic form of compound No. 6 = compound No. 6r
Racemic form of compound No. 7 = compound No. 7r

Test Example 1 Preventive activity on wheat powdery mildew

A wettable powder prepared in the same manner as described in Formulation Example 1 was diluted with water to a predetermined concentration and applied on wheat (variety: Nohrin No. 61) a 1 to 2 leaf stage grown in pots of 6 cm in diameter, by foliar application in an amount of 10 ml per pot. After air-drying the chemical solution, the plant bodies were inoculated with spores collected from wheat leaves infected with wheat powdery mildew fungus (*Erysiphe graminis*), by spraying the suspension, and then left as such in a greenhouse for 7 to 10 days.
The evaluation was made by measuring the diseased area ratio on each leaf and calculating a preventive value based on the following formula. The results are shown in Table 13.

Preventive value (%) = (average diseased area ratio in untreated plot - average diseased area ratio in treated plot)/(average diseased area ratio in untreated plot) x 100

Table 13

| Preventive value (%) | | |
|---|---|---|
| Compound No. | 4.0 ppm | 2.0 ppm |
| 1 | 51 | 6 |
| 2 | 100 | 97 |
| 2r | 96 | 64 |
| 3 | 73 | 5 |
| 3r | 12 | 0 |
| 4 | 99 | 99 |
| 4r | 99 | 73 |
| 5 | 98 | 98 |
| 5r | 97 | 45 |
| 6 | 100 | 94 |
| 6r | 99 | 57 |
| 7 | 99 | 98 |
| 7r | 99 | 69 |

Test Example 2 Preventive activity on wheat brown rust

A wettable powder prepared in the same manner as described in Formulation Example 1 was diluted with water to a concentration of 10 ppm and applied on wheat (variety, Nohrin No. 61) at 1 to 2 leaf stage grown in pots of 6 cm in diameter, by foliar application in an amount of 10 ml per pot. After air-drying the chemical solution, the plant bodies were inoculated with a suspension of spores collected by pulverizing wheat leaves infected with wheat brown rust fungus (*Puccinia recondita*), by spraying the suspension, kept for 15 hours in a moist chamber at 22°C and then allowed to stand in a greenhouse for 7 days.

The evaluation was made by measuring the diseased area ratio on each leaf and calculating a preventive value based on the following formula. The results are shown in Table 14.

Table 14

| Compound No. | Preventive value |
|---|---|
| 1 | 0 |
| 2 | 99 |
| 2r | 32 |
| 3 | 45 |
| 3r | 0 |
| 4 | 100 |
| 4r | 28 |
| 5 | 98 |
| 5r | 35 |
| 6 | 96 |
| 6r | 14 |
| 7 | 99 |
| 7r | 75 |

Next, an example is described on the toxicity test against a mammal which is not the controlling subject.

Test Example 3 Oral acute toxicity test using rats

Each of the compound No. 2 and its racemate No. 2r was suspended in tragacanth gum aqueous solution and applied to CD rats (female) by single oral administration in a dosage of 300, 600, 1,200, 2,500 or 5,000 mg/kg to obtain its $LC_{50}$ value. As the results, the compound No. 2 showed no mortality by these dosages, and its $LC_{50}$ value therefore was 5,000 mg/kg or more. In the case of the racemate No. 2r, on the other hand, no mortality was found with a dosage of 300 mg/kg, but 1/5 cases died with each of 600 and 1,200 mg/kg dosages, and 4/5 cases with each of 2,500 and 5,000 mg/kg dosages. Based on these results, the $LC_{50}$ value (95% confidence limit) of the racemic form No. 2r was calculated to be 1,784 mg/kg.

The following reveals examples of improvement on preventive activity by adding organosilicon surfactant in a formulation, with reference to an emulsifiable concentrate and a flowable of optically active methoxyiminoaceamide derivative (I) of the present invention.

Formulation Example 3

An emulsifiable concentrate was obtained by mixing and dissolving 10% by weight of the No.2 compound shown in Table 1, 10% by weight of organosilicon surfactant "Silwet"-77 (manufactured by Witok, trademark), 15% by weight of "Sorpol" 3005X (a mixture of a nonionic surfactant and an anionic surfactant, manufactured by Toho Chemical Industry, trademark), 55% by weight of aromatic solvent "Solvesso"200 (manufactured by EXXON, trademark), and 10% by weight of N-methylpyrrolidone.

Additionally for comparison, an emulsifiable concentrate (Comparative Example 1) was obtained by mixing and

dissolving 10% by weight of the No.2 compound shown in Table 1, 15% by weight of "Sorpol" 3005X (a mixture of a nonionic surfactant and an anionic surfactant, manufactured by Toho Chemical Industry, trademark), 65% by weight of aromatic solvent "Solvesso"200 (manufactured by EXXON, trademark), and 10% by weight of N-methylpyrrolidone.

Formulation Example 4

10% By weight of the No.2 compound shown in Table 1 was added to 2% by weight of "Sorpol" 7290P (an anionic surfactant, manufactured by Toho Chemical Industry, trademark), 3% by weight of "Runox" 1000C (an anionic surfactant, manufactured by Toho Chemical Industry, trademark) and 55% by weight of water, and the mixture was ground by wetting method using sandmill. A flowable was obtained by mixing and stirring thereto 20% by weight of 1% "Xanthan gum" aqueous solution and 10% by weight of "Silwet"408 (organosilicon surfactant manufactured by Witok, trademark).

Additionally for comparison, 10% by weight of the No.2 compound shown in Table 1 was added to 2% by weight of "Sorpol" 7290P (anionic surfactant manufactured by Toho Chemical Industry, trademark), 3% by weight of "Runox" 1000C (anionic surfactant manufactured by Toho Chemical Industry, trademark) and 65% by weight of water, and the mixture was ground by wetting method using sandmill. A flowable (Comparative Example 2) was obtained by mixing and stirring 20% by weight of 1% "Xanthan gum" aqueous solution thereto.

Test Example 4 Residual activity on wheat powdery mildew

An emulsifiable concentrate and a flowable, obtained by the method mentioned in Formulation Example 3 to 4 and Comparative Example 1 to 2, was diluted with water to a predetermined concentration and applied by foliar application on wheat plants in the same way as described in Test Example l. After air-drying the chemical solution, the plants was kept outdoor for 3, 7 and 14 days, and inoculated with spores of Erysiphe graminis in the same way as described in Test Example 1. Then the plants were allowed to stand in a greenhouse for 7 to 10 days.

The evaluation was made by measuring the diseased area ratio on each leaf and calculating a preventive value by the same way as described in Test Example 1. The results are shown in Table 15.

Table 15

| Residual activity on wheat powdery mildew | | | | | |
|---|---|---|---|---|---|
| Chemicals | Formulation | Concentration (ppm) | Preventive value (%) | | |
| | | | 3 DAY | 7 DAY | 14 DAY |
| Formulation Example 3 | Emulsifiable concentrate | 10 | 100 | 100 | 97 |
| Comparative Example 1 | Emulsifiable concentrate | 10 | 100 | 76 | 42 |
| Formulation Example 4 | Flowable | 10 | 100 | 98 | 89 |
| Comparative Example 2 | Flowable | 10 | 98 | 45 | 18 |
| Untreated plot (diseased area ratio: %) | | - | (92) | (83) | (89) |
| DAT means days after treatment. | | | | | |

Next, effects of mixed formulations of the optically active methoxyiminoacetamide derivative (I) of the present invention and known compounds are described (tests on the effect of combined application).

Formulation Example 5

Respective emulsifiable concentrate was obtained by uniformly mixing and dissolving 10% by weight of the compound No. 2, 6 or 7 shown in Table 1, 10% by weight of a known compound a or b as a known agricultural chemical component, 35% by weight of xylene, 30% by weight of N,N-dimethylformamide and 15% by weight of "Sorpol" 3005 X (a polyoxyethylene based surfactant; trade name by Toho Chemical Industry).

The known compound a in this case is (1RS,5RS;1RS,5SR)-5-(4-chlorobenzyl)-2,2-dimethyl-1-(1H-1,2,4-triazol-1-ylmethyl)cyclopentanol, and the known compound b is (RS)-1-p-chlorophenyl-4,4-dimethyl-3-(1H-1,2,4-triazol-1-ylmethyl)pentan-3-ol.

Formulation Example 6

Respective emulsifiable concentrate was obtained by uniformly mixing and dissolving 10% by weight of the compound No. 2, 6 or 7 shown in Table 1, 30% by weight of a known compound c or d as a known agricultural chemical Component, 30% by weight of xylene, 20% by weight of N,N-dimethylformamide and 10% by weight of "Sorpol" 3005 X (a polyoxyethylene based surfactant; trade name by Toho Chemical Industry).
The known Compound c in this case is (±)-cis-4-[3-(4-tert-butylphenyl)-2-methylpropyl]-2,6-dimethylmorpholine, and the known compound d is N-propyl-N-[2-(2,4,6-trichlorophenoxy)ethyl]imidazole-1-carboxamide.

Formulation Example 7

Respective wettable powder was obtained by uniformly mixing, using a jet air mil, 10% by weight of the compound No. 2, 6 or 7 shown in Table 1, 5% by weight of a known compound e or f as a known agricultural chemical component, 10% by weight of CARPLEX #80 (white carbon; trade name by Shionogi), 67% by weight of Seek lite SP (a mixture of kaolinite and sericite; trade name by Zeek lite Kogyo) and 8% by weight of calcium lignosulfonate.
The known compound e in this case is methyl-N-(2-methoxyacetyl)-N-(2,6-xylyl)-DL-alaninate, and the known compound f is 3-chloro-N-(3-chloro-5-trifluoromethyl-2-pyridyl-α,α,α-trifluoro-2,6-dinitro-p-toluidine.

Formulation Example 8

Respective wettable powder was obtained by uniformly mixing, using a jet air mil, 10% by weight of the compound No. 2 shown in Table 1, 10% by weight of a known compound g, h, i, j, k, l, m, n, o or p as a known insecticide or acaricide, 10% by weight of CARPLEX #80 (white carbon; trade name by Shionogi), 62% by weight of Zeek lite SP (a mixture of kaolinite and sericite; trade name by Zeek lite Kogyo) and 8% by weight of calcium lignosulfonate.
The known compound g in this case is 1-(3,5-dichloro-2,4-difluorophenyl)-3-(2,6-difluorobenzoyl)urea, the known compound h is N-tert-butyl-N'-(4-ethylbenzoyl)-3,5-dimethylbenzohydrazide, the known compound i is O,O-dimethyl O-4-nitro-m-toluyl phosphorothioate, the known compound j is (RS)-α-cyano-3-phenoxybenzyl N-(2-chloro-α,α,α-trifluoro-p-toluyl)-D-Barinate, the known compound k is N-(4-tert-butylbenzyl)-4-chloro-3-ethyl-1-methylpyrazole-5-carboxamide, the known compound l is 1-(6-chloro-3-pyridylmethyl)-N-nitroimidazolidin-2-ylideneamine, the known compound m is 4-bromo-2-(4-chlorophenyl)-1-ethoxymethyl-5-trifluoromethylpyrrole-3-carbonitrile, the known compound n is 2,2,2-trichloro-1,1-bis(4-chlorophenyl)ethanol, the known compound o is bis[tris(2-methyl-2-phenylpropyl)tin]oxide and the known compound p is 6-methylquinoxaline-2,3-dithiocarbonate.
Next, test examples with the thus formulated admixtures are described.

Test Example 5 Test on the effect of combined application for controlling wheat powdery mildew

Each of the agricultural compositions and single preparations obtained in Formulation Examples 5 to 8 was dispersed in water containing 100 ppm of a spreader (Nonipol Soft SS-90; manufactured by Sanyo Chemical Industries) and diluted to a predetermined concentration and applied on wheat (variety: Nohrin No. 61) at 1 to 2 leaf stage grown in a pot of 6 cm in diameter, by foliar application in an amount of 10 ml per pot. After air-drying the chemical solution, the plant bodies were inoculated with spores collected from wheat leaves infected with wheat powdery mildew fungus (*Erysiphe graminis* f. sp. *tritici*) and then left as such in a greenhouse for 7 to 10 days.
The evaluation was made by measuring diseased area ratio on each leaf and calculating a preventive value in the same way as described in Test Example 1.
In addition, Limpel's formula (p.312 in *Pesticide Science* (1987), 19, 309 - 315) shown below was used in order to judge the degree of synergism.

$$E = Y + Y - XY/100$$

In the above formula, E, X and Y are defined as follows.
In the case of the combined application of a preparation A (a ppm) and a preparation B (b ppm), E is a theoretically expected preventive value (%) when treated with a + b ppm, X is an actual preventive value (%) when the preparation A is treated by a ppm, and Y is an actual preventive value (%) when the preparation B is treated by b ppm. That is, combined use of the preparation A with the preparation B is judged that it has a synergistic effect when the preventive value (%) obtained by the combined use of the preparation A with the preparation B is larger than the value of E calculated by the aforementioned Limpel's formula.
The preventive values and degree of synergistic effect calculated by these formulae are shown in Table 16.

Test Example 6 Test on the effect of combined application for controlling wheat brown rust

Each chemical diluted to a predetermined concentration prepared in the same manner as described in Test Example 5 was applied on wheat (variety: Nohrin No. 61) at 1 to 2 leaf stage grown in a pot of 6 cm in diameter, by foliar application in an amount of 10 ml per pot. After air-drying the chemical solution, the plant bodies were inoculated with a suspension of spores collected from wheat leaves infected with wheat brown rust fungus (*Puccinia recondita*), by spraying the suspension, kept in a moist chamber for 24 hours at 22°C and then left as such on a water bath in a greenhouse for 7 days.

Evaluation was made by measuring diseased area ratio on each leaf and calculating the preventive value and judging the degree of synergistic effect by the same method described in Test Example 1. The results are shown in Table 17.

Table 16

| Effect of combined application against wheat powdery mildew | | | | |
|---|---|---|---|---|
| Chemical | Treating conc. (ppm) | | Preventive value (%) | E value * (%) |
| | Invention derivative | Known compound | | |
| Compound 2 | 1 | - | 87 | - |
| | 0.2 | - | 25 | - |
| Compound 6 | 1 | - | 76 | - |
| | 0.2 | - | 18 | - |
| Compound 7 | 1 | - | 52 | - |
| | 0.2 | - | 10 | - |
| Known compound a | - | 1 | 80 | - |
| | - | 0.2 | 35 | - |
| Known compound b | - | 1 | 65 | - |
| | - | 0.2 | 23 | - |
| Known compound c | - | 12 | 50 | - |
| | - | 2.4 | 18 | - |
| Compound 2 + Known compound a | 1 | 1 | 100 | 97 |
| | 0.2 | 0.2 | 95 | 51 |
| Compound 6 + Known compound a | 1 | 1 | 100 | 90 |
| | 0.2 | 0.2 | 91 | 41 |
| Compound 7 + Known compound a | 1 | 1 | 98 | 95 |
| | 0.2 | 0.2 | 90 | 47 |
| Compound 2 + Known compound b | 1 | 1 | 99 | 95 |
| | 0.2 | 0.2 | 91 | 42 |
| Compound 2 + Known compound c | 1 | 12 | 99 | 94 |
| | 0.2 | 2.4 | 85 | 39 |
| Compound 6 + Known compound c | 1 | 12 | 98 | 88 |
| | 0.2 | 2.4 | 65 | 33 |
| Compound 7 + Known compound b | 1 | 1 | 96 | 83 |
| | 0.2 | 0.2 | 79 | 30 |

*; theoretical preventive value by combined application

Table 17

| Effect of combined application against wheat brown rust | | | | |
|---|---|---|---|---|
| Chemicals | Invention derivative (ppm) | Known compound (ppm) | Preventive value (%) | E value * (%) |
| Compound 2 | 1<br>0.2 | - | 48<br>2 | -<br>- |
| Compound 6 | 1<br>0.2 | - | 40<br>0 | -<br>- |
| Compound 7 | 1<br>0.2 | - | 30<br>0 | -<br>- |
| Known compound a | - | 1<br>0.2 | 81<br>25 | -<br>- |
| Known compound b | - | 1<br>0.2 | 75<br>22 | -<br>- |
| Known compound c | - | 12<br>2.4 | 0<br>0 | -<br>- |
| Known compound d | - | 12<br>2.4 | 19<br>0 | -<br>- |
| Compound 2 + Known compound a | 1<br>0.2 | 1<br>0.2 | 92<br>56 | 90<br>27 |
| Compound 6 + Known compound a | 1<br>0.2 | 1<br>0.2 | 94<br>45 | 89<br>25 |
| Compound 2 + Known compound b | 1<br>0.2 | 1<br>0.2 | 90<br>41 | 87<br>24 |
| Compound 2 + Known compound c | 1<br>0.2 | 12<br>2.4 | 88<br>25 | 48<br>2 |
| Compound 2 + Known compound d | 1<br>0.2 | 12<br>2.4 | 80<br>32 | 58<br>2 |
| Compound 6 + Known compound d | 1<br>0.2 | 12<br>2.4 | 81<br>26 | 51<br>0 |
| Compound 7 + Known compound c | 1<br>0.2 | 12<br>2.4 | 67<br>15 | 30<br>0 |

*; theoretical preventive value by combined application

Test Example 7 Test on the effect of combined application for controlling tomato late blight

Each chemical diluted to a predetermined concentration prepared in the same manner as described in Test Example 5 was applied on a tomato (variety: Red Cherry) at 3 to 5 leaf stage grown in a pot of 9 cm in diameter, by foliar application in an amount of 10 ml per pot. After air-drying the chemical solution, the plant bodies were inoculated with a suspension of zoosporangia collected from tomato leaves infected with tomato late blight fungus (*Phytophthora infestans*), by spraying the suspension, kept in a moist chamber for 24 hours at 22°C and then left as such on a water bath in a greenhouse for 7 days.

Evaluation was made by measuring diseased area ratio on each leaf and calculating the preventive value and judging the degree of synergistic effect by the same method described in Test Example 1. The results are shown in Table 18.

Table 18

| Effect of combined application against tomato late blight | | | | |
|---|---|---|---|---|
| Chemical | Treating conc. (ppm) | | Preventive value (%) | E value * (%) |
| | Invention derivative | Known compound | | |
| Compound 2 | 10 | - | 95 | - |
| | 2 | - | 73 | - |
| | 0.4 | - | 23 | - |
| Compound 6 | 10 | - | 95 | - |
| | 2 | - | 75 | - |
| | 0.4 | - | 35 | - |
| Compound 7 | 10 | - | 88 | - |
| | 2 | - | 46 | - |
| | 0.4 | - | 0 | - |
| Known compound d | - | 5 | 95 | - |
| | - | 1 | 79 | - |
| | - | 0.2 | 62 | - |
| Compound 2 + Known compound d | 10 | 5 | 100 | 99 |
| | 2 | 1 | 99 | 94 |
| | 0.4 | 0.2 | 87 | 71 |
| Compound 6 + Known compound d | 10 | 5 | 100 | 99 |
| | 2 | 1 | 100 | 95 |
| | 0.4 | 0.2 | 84 | 75 |
| Compound 7 + Known compound d | 10 | 5 | 99 | 99 |
| | 2 | 1 | 96 | 89 |
| | 0.4 | 0.2 | 78 | 62 |

*; theoretical preventive value by combined application

Test Example 8 Test on the effect of combined application for controlling cucumber gray mold

Each chemical diluted to a predetermined concentration prepared in the same manner as described in Test Example 5 was applied on a cucumber (variety: Yotsuba) at cotyledonous stage grown in a pot of 9 cm in diameter, by foliar application in an amount of 10 ml per pot. After air-drying the chemical solution, cucumber cotyledon was cut off at the petiole position and kept in a vat conditioned at high humidity. A circular piece of filter paper of 8 mm in diameter, containing a suspension of spores of cucumber gray mold fungus (*Botrytis cinerea*) which has been cultured on a potato broth agar medium supplemented with sucrose, was put on the center of the cotyledon to effect inoculation and then the small vat was left as such in moist chamber at 22°C for 5 days.

Evaluation was made by measuring diameter of lesion on each leaf and calculating the preventive value based on the following formula.

Preventive value (%) = (diameter of lesion in untreated plot - diameter of lesion in treated plot)/(diameter of lesion in untreated plot) x 100

The degree of synergistic effect was judged by the same method described in Test Example 5. The results are shown in Table 19.

Table 19

| Effect of combined application against cucumber gray mold | | | | |
|---|---|---|---|---|
| Chemical | Treating conc. (ppm) | | Preventive value (%) | E value * (%) |
| | Invention derivative | Known compound | | |
| Compound 2 | 10 | - | 94 | - |
| | 2 | - | 53 | - |
| | 0.4 | - | 13 | - |
| Compound 6 | 10 | - | 90 | - |
| | 2 | - | 61 | - |
| | 0.4 | - | 5 | - |
| Compound 7 | 10 | - | 87 | - |
| | 2 | - | 37 | - |
| | 0.4 | - | 0 | - |
| Known compound f | - | 5 | 98 | - |
| | - | 1 | 68 | - |
| | - | 0.2 | 52 | - |
| Compound 2 + Known compound f | 10 | 5 | 100 | 99 |
| | 2 | 1 | 100 | 85 |
| | 0.4 | 0.2 | 91 | 58 |
| Compound 2 + Known compound f | 10 | 5 | 100 | 99 |
| | 2 | 1 | 89 | 80 |
| | 0.4 | 0.2 | 71 | 52 |
| Compound 6 + Known compound f | 10 | 5 | 100 | 99 |
| | 2 | 1 | 100 | 88 |
| | 0.4 | 0.2 | 82 | 54 |

*; theoretical preventive value by combined application

Test Example 9 Test on the insecticidal effect against green peach aphid

A radish (variety: Tokinashi Daikon) at 3 to 5 leaf stage grown in a pot of 9 cm in diameter was cut at the petiole position to which was inoculated two adults of green peach aphid, and the petiole portion was put into a 5 ml capacity screw bottle. This was put into a glass cylinder of 3.5 cm in diameter and 18 cm in height, covered with a mesh cap and left as such for 7 days in a room to effect hatching. Each of the agricultural chemical composition of the present invention and single preparation controls was dispersed in water containing 200 ppm of a spreader (Sorpol 3005 X, manufactured by Toho Chemical Industry) and diluted to a predetermined concentration of the active component. Adults of green peach aphid were removed from the leaf keeping adults and larvae of the aphid to count the number of larvae which were subsequently soaked in the just described drug solution for 5 seconds, put into a glass cylinder in the same

manner as described above and then left as such for 4 days in a constant temperature room of 25 ± 1°C.

Evaluation was made by examining mortality of the larvae and calculating death rate of larvae based on the following formula.

Death rate (%) = {the number of dead larvae/(the number of dead larvae + the number of live larvae)} x 100

Also, the degree of synergistic effect was judged by the following method. That is, the $LC_{50}$ value of each chemical component which constitutes the agricultural chemical composition and the $LC_{50}$ value of the combined agricultural chemical composition were calculated from the death rate obtained by the above formula by the usually used probit analysis. Next, a synergistic factor was calculated using the following formula of Sun and Johnson (*J. Econ. Ent.* (1960), vol.53, p.887) which is generally used when the degree of synergistic effect is judged.

Synergistic factor = actual toxicity index of pesticide combination/theoretical toxicity index of pesticide combination x 100

In the above formula, when a mixture of a drug A and a drug B is used as a pesticide combination M,

actual toxicity index of the pesticide combination M = $(ALC_{50}/MLC_{50}) \times 100$,

theoretical toxicity index of the pesticide combination M = $(Y_A \times a + Y_B \times b) \times 100$,

$Y_B = (ALC_{50}/BLC_{50}) \times 100$ and
$Y_A = 100$.
$ALC_{50}$: $LC_{50}$ value of drug A, $BLC_{50}$: $LC_{50}$ value of drug B, $MLC_{50}$: $LC_{50}$ value of drug M,
$Y_A$: toxicity index of drug A, $Y_B$: toxicity index of drug B, and
a: ratio (%) of drug A in the pesticide combination M, b: ratio (%) of drug B in the pesticide combination M, wherein,

The value of synergistic factor obtained by this formula when larger than 100 means stronger synergism, the value when equal to 100 means the presence of an additive action and the value when smaller than 100 means the presence of an antagonism.

The $LC_{50}$ value of each drug component which constitutes the agricultural chemical composition, the $LC_{50}$ value of the combined agricultural chemical composition and the synergistic factor calculated using the formula of Sun and Johnson are shown in Table 20.

Table 20

| Synergistic effect of the agricultural chemical composition of the present invention against green peach aphid adults | | | |
|---|---|---|---|
| Chemicals | Mixing ratio (compound 2:other drug) | $LC_{50}$ value (ppm) | Synergistic factor |
| Compound 2 | - | 450 | - |
| Known compound i | - | 130 | - |
| Known compound j | - | 1.1 | - |
| Known compound k | - | 20.0 | - |
| Known compound l | - | 0.23 | - |
| Compound 2 + Known compound i | 1:1 | 60.0 | 406 |
| Compound 2 + Known compound j | 1:1 | 1.1 | 186 |
| Compound 2 + Known compound k | 1:1 | 21.0 | 189 |
| Compound 2 + Known compound 1 | 1:1 | 0.38 | 120 |

Test Example 10 Test on the acaricidal effect against two-spotted spider mite

A leaf disk of 2 cm in diameter was cut off using a leaf punch from the first leaf of a kidney bean (variety: Kintoki) raised in a pot of 15 cm in diameter, and each of 8 leaf disks thus prepared was arranged on wet absorbent cotton which was put on a plastic cup of 8 cm in diameter. A total of 5 female adults of two-spotted spider mite were inoculated on each leaf disk. After the inoculation, each of the agricultural chemical composition of the present invention and single compound controls was diluted to a predetermined concentration in the same manner as described in Test Example 9 and sprayed on the disks using a rotary spray tower (manufactured by Mizuho Rika) in an amount of 5 ml per one plastic cup which was subsequently left as such for 2 days in a constant temperature room of 25 ± 1°C.
The evaluation was made by examining the number of live and dead adults of two-spotted spider mite on each leaf disk and calculating their death rate based on the following formula.

Death rate (%) = {the number of dead mite/(the number of dead mite + the number of live mite)} x 100

Also, the degree of synergistic effect was judged by the same method described in Test Example 9. The results are shown in Table 21.

Table 21

| Synergistic effect of the agricultural chemical composition of the present invention against two-spotted spider mite adults | | | |
|---|---|---|---|
| Chemicals | Mixing ratio (compound 2:other drug) | $LC_{50}$ value (ppm) | Synergistic factor |
| Compound 2 | - | 1900 | - |
| Known compound m | - | 3.0 | - |
| Known compound n | - | 19.0 | - |
| Known compound o | - | 25.0 | - |
| Known compound p | - | 89.0 | - |
| Compound 2 + Known compound m | 1:1 | 5.1 | 117 |
| Compound 2 + Known compound n | 1:1 | 29.0 | 130 |
| Compound 2 + Known compound o | 1:1 | 19.0 | 260 |
| Compound 2 + Known compound p | 1:1 | 110.0 | 155 |

Test Example 11 Test on the insecticidal effect against diamond back moth

A cabbage leaf was cut to a size of 6 cm in diameter and soaked for about 60 seconds in a chemical solution obtained in the same manner as described in Test Example 9 by diluting each of the agricultural chemical composition of the present invention and single compound controls to a predetermined concentration. After thoroughly air-drying the chemical solution on the leaf surface, the leaf was put into a round cup (8 cm in diameter) (one leaf piece/round cup) together with five second instar larvae of diamond back moth and then kept in a constant temperature room of 25°C.
The evaluation was made by examining the number of live larvae, larvae in agony and dead larvae after 4 days of the treatment and calculating their death rate in the same manner as described in Test Example 9.
Also, the degree of synergistic effect was judged by the same method described in Test Example 9. The results are shown in Table 22.

Table 22

| Synergistic effect of the agricultural chemical composition of the present invention against second instar larvae of diamond back moth | | | |
|---|---|---|---|
| Chemicals | Mixing ratio (compound 2:other drug) | $LC_{50}$ value (ppm) | Synergistic factor |
| Compound 2 | - | 260 | - |
| Known compound g | - | 0.29 | - |
| Known compound h | - | 23.0 | - |
| Compound 2 + Known compound g | 1:1 | 0.47 | 123 |
| Compound 2 + Known compound h | 1:1 | 25.0 | 182 |

INDUSTRIAL APPLICABILITY

All of the optically active methoxyiminoacetamide derivatives of the present invention are novel compounds and have markedly excellent activities as agricultural chemicals. Also, according to the present invention, said compounds can be produced efficiently with low cost.

**Claims**

1. An optically active methoxyiminoacetamide derivative represented by the following general formula (I)

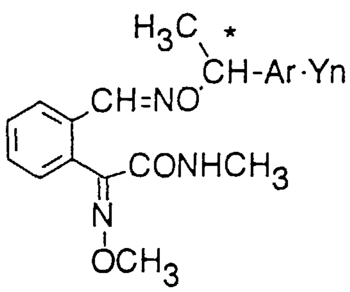

(I)

(in the above general formula, Ar represents an aryl group or a heteroaryl group, Y represents a hydrogen atom, a halogen atom, a $C_1$-$C_4$ alkyl group, a $C_1$-$C_4$ haloalkyl group, a $C_1$-$C_4$ alkoxy group, a $C_1$-$C_4$ haloalkoxy group, a $C_1$-$C_4$ alkylthio group, a $C_2$-$C_6$ alkynyloxy group, a cyano group, a nitro group or an aryl group and n is an integer of 1 to 3), wherein said derivative has a negative value of the angle of rotation in chloroform.

2. The optically active methoxyiminoacetamide derivative according to claim 1, wherein Ar is an aryl group, Y is a hydrogen atom, a halogen atom, a $C_1$-$C_4$ alkyl group, a $C_1$-$C_4$ haloalkyl group, a $C_1$-$C_4$ alkoxy group or a $C_1$-$C_4$ haloalkoxy group and n is an integer of 1 to 3.

3. The optically active methoxyiminoacetamide derivative according to claim 1, wherein its optical purity is 50% ee or more.

4. A method for producing an optically active methoxyiminoacetamide derivative represented by the aforementioned general formula (I), which comprises allowing an optically active 1-arylethoxyamine derivative represented by the following general formula (II)

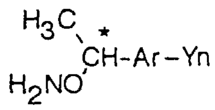

(II)

(in the above general formula, Ar, Y and n are as defined in the foregoing) or a salt thereof to react with a compound represented by the following general formula (III) or the following general formula (V)

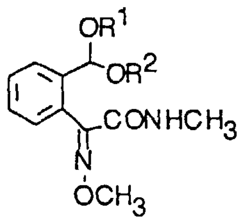

(III)

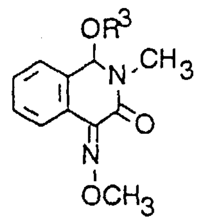

(V)

(in the above general formulae (III) and (V), each of $R^1$ and $R^2$ independently represents a $C_1$-$C_4$ alkyl group, or $R^1$ and $R^2$ together form a $C_2$-$C_4$ alkylene group and $R^3$ represents a hydrogen atom or a $C_1$-$C_4$ alkyl group).

**5.** The method for producing the optically active methoxyiminoacetamide derivative (I) according to claim 4, wherein the salt of optically active 1-arylethoxyamine derivative is tartrate.

**6.** A method for producing the acetal derivative of the aforementioned general formula (III), which comprises allowing a phenylglyoxylic acid ester derivative represented by the following general formula (IV)

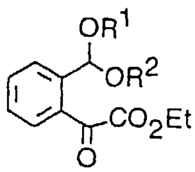

(IV)

to react with methoxyamine or a salt thereof at pH 4 to 6 and then to react with methylamine.

**7.** The method for producing the acetal derivative (III) according to claim 6, which comprises allowing to react with methoxyamine acid salt in the presence of organic bases having a conjugate acid pKa value of from 5.5 to 7 and then to react with methylamine.

**8.** The method for producing the acetal derivative (III) according to claim 6, wherein $R^1$ and $R^2$ together form an ethylene group.

**9.** A method for producing an optically active methoxyiminoacetamide derivative represented by the aforementioned general formula (I), which comprises allowing an optically active 1-arylethoxyamine derivative represented by the aforementioned general formula (II) or a salt thereof to react with an aldehyde derivative represented by the following general formula (VI)

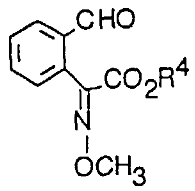

(VI)

(in the above formula, $R^4$ represents a $C_1$-$C_6$ alkyl group), to form a methoxyimonoacetic acid ester derivative represented by the following general formula (VII)

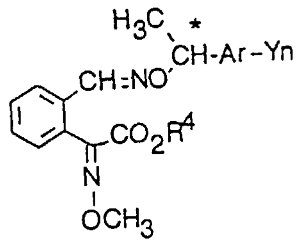

(VII)

(in the above formula, $R^4$, Ar, Y and n are as defined in the foregoing), and subsequently allowing the ester derivative to react with methylamine.

**10.** An optically active methoxyiminoacetic acid ester derivative represented by the aforementioned general formula (VII).

**11.** An optically active 1-arylethoxyamine derivative represented by the aforementioned general formula (II).

**12.** The optically active 1-arylethoxyamine derivative according to claim 11, wherein its optical purity is 50% ee or more.

**13.** A method for producing an optically active 1-arylethoxyamine derivative represented by the aforementioned general formula (II), which comprises carrying out optical resolution of a 1-arylethoxyamine derivative represented by the following general formula (II)'

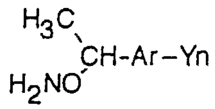

(II)'

(in the above formula, Ar, Y and n are as defined in the foregoing) using optically active tartaric acid.

**14.** A method for producing a 1-arylethoxyamine derivative represented by the aforementioned general formula (II)', which comprises allowing a 1-arylethyl halide derivative represented by the following general formula (VIII)

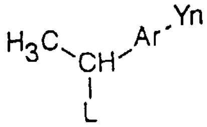

(VIII)

(in the above formula, Ar, Y and n are as defined in the foregoing, and L represents a halogen atom) to react with a hydroxamic acid represented by the following general formula (IX)

$R^5CONHOH$ (IX)

(in this formula, $R^5$ represents a $C_1$-$C_6$ alkyl group, a $C_1$-$C_6$ alkoxy group, a benzyloxy group, a phenoxy group or a phenyl group), thereby obtaining a hydroxamic acid derivative represented by the following general formula (X)

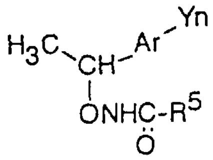

(X)

(in the above formula, $R^5$, Ar, Y and n are as defined in the foregoing), and subsequently hydrolyzing the derivative (X).

**15.** A hydroxamic acid derivative represented by the general formula (X).

**16.** The hydroxamic acid derivative according to claim 15, wherein $R^5$ is a $C_1$-$C_6$ alkyl group, a $C_1$-$C_6$ alkoxy group or a phenyl group.

**17.** The hydroxamic acid derivative according to claim 15, wherein Ar is an aryl group, Y is a $C_1$-$C_4$ haloalkyl group and n is 1.

**18.** A method for producing an optically active 1-arylethyl halide derivative represented by the general formula (VIII), which comprises treating an optically active 1-arylethylalcohol derivative represented by the following general formula (XI)

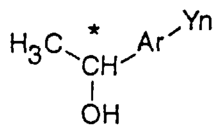

(XI)

(in the above formula, Ar, Y and n are as defined in the foregoing) with a halogenating agent in the presence of a base, thereby effecting its halogenation by which the configuration is inverted.

**19.** The method for producing an optically active 1-arylethyl halide derivative (VIII) according to claim 18, wherein a $C_1$-$C_6$ alkylsulfonyl halide or an arylsulfonyl halide which may be substituted with a substituent selected from $C_1$-$C_4$ alkyl groups and halogen atoms is used as the halogenating agent.

**20.** The method for producing an optically active 1-arylethyl halide derivative (VIII) according to claim 18, wherein pyridine which may be substituted with a halogen atom or a $C_1$-$C_4$ alkyl group is used as the base.

**21.** The method for producing an optically active 1-arylethyl halide derivative (VIII) according to claim 18, wherein a reaction solvent containing N,N-dimethylformamide is used.

**22.** A method for producing an optically active 1-arylethyl halide derivative represented by the general formula (VIII), which comprises treating an optically active sulfonic acid ester derivative represented by the following general formula (XII)

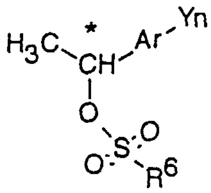

(XII)

(in the above formula, Ar, Y and n are as defined in the foregoing, and $R^6$ represents a $C_1$-$C_6$ alkyl group; a phenyl group which may be substituted with a $C_1$-$C_4$ alkyl group or a halogen atom; or a benzyl group or a naphthyl group) with a hydrohalogenic acid salt, thereby effecting its halogenation by which the configuration is inverted.

**23.** An optically active sulfonic acid ester derivative represented by the aforementioned general formula (XII).

**24.** The optically active sulfonic acid ester derivative (XII) according to claim 23, wherein Ar is an aryl group, Y is a $C_1$-$C_4$ haloalkyl group and n is 1.

**25.** A method for producing an optically active 1-arylethylalcohol derivative represented by the general formula (XI), which comprises effecting hydrogen transferring asymmetric reduction of an acetophenone derivative represented by the following general formula (XIII)

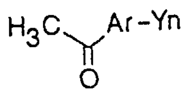

(XIII)

(in the above formula, Ar, Y and n are as defined in the foregoing) in the presence of a transition metal complex, a base, an optically active nitrogen-containing compound and a hydrogen donor.

**26.** The method for producing the optically active 1-arylethylalcohol derivative (XI) according to claim 25, wherein Ar is an aryl group, Y is a $C_1$-$C_4$ haloalkyl group and n is 1.

**27.** The method for producing the optically active 1-arylethylalcohol derivative (XI) according to claim 25, wherein the transition metal complex is $[RuCl_2(arene)]$ (wherein arene represents a benzene derivative which may have 1 to 6 alkyl groups).

**28.** The method for producing the optically active 1-arylethylalcohol derivative (XI) according to claim 25, wherein the optically active nitrogen-containing compound is a 1,2-diphenylethylenediamine monosulfonamide derivative having an aromatic sulfonyl group.

**29.** The method for producing the optically active 1-arylethylalcohol derivative (XI) according to claim 25, wherein $C_1$-$C_4$ alcohols or formic acid is used as the hydrogen donor.

**30.** A method for producing a hydroxime derivative represented by the following general formula (XV)

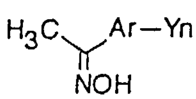

(XV)

(wherein Ar, Y and n are as defined in the foregoing), which comprises allowing a diazonium salt derived from an aniline derivative represented by the following general formula (XIV)

$$H_2N\text{-}Ar-Yn \qquad (XIV)$$

(in the above formula, Ar, Y and n are as defined in the foregoing) to react with acetaldoxime.

**31.** The method for producing the hydroxime derivative (XV) according to claim 30, wherein Ar is an aryl group, Y is a $C_1$-$C_4$ haloalkyl group and n is 1.

**32.** A method for producing an acetophenone derivative represented the general formula (XIII), which comprises allowing a diazonium salt derived from an aniline derivative of the general formula (XIV) to react with acetaldoxime to obtain a hydroxime derivative (XV) and subsequently hydrolyzing the thus obtained derivative.

**33.** The method for producing the acetophenone derivative (XIII) according to claim 32, wherein a diazonium salt derived from an aniline derivative of the general formula (XIV) is allowed to react with acetaldoxime at 10°C or less.

**34.** A method for producing an optically active methoxyiminoacetamide derivative represented by the general formula (I), which comprises the steps of carrying out a configuration-inverting etherification through reaction of an optically active 1-arylethyl halide derivative represented by the general formula (VIII) with a hydroxamic acid (IX), thereby obtaining a hydroxamic acid derivative of the general formula (X) which is subsequently hydrolyzed while keeping its configuration, and then carrying out condensation of the thus obtained optically active 1-arylethoxyamine derivative (II) or a salt thereof with a compound represented by the general formula (III), (V) or (VI) while keeping the configuration.

**35.** The method for producing an optically active methoxyiminoacetamide derivative (I) according to claim 34, wherein the optically active 1-arylethyl halide derivative (VIII) is obtained by subjecting an optically active 1-arylethylalcohol derivative represented by the general formula (XI) to halogenation which accompanies inversion of the configuration.

**36.** The method for producing the optically active methoxyiminoacetamide derivative (I) according to claim 34, wherein the optically active 1-arylethyl halide derivative (VIII) is obtained by subjecting an acetophenone derivative represented by the general formula (XIII) to hydrogen transfer type asymmetric reduction thereby obtaining an optically active 1-arylethylalcohol (XI) and subsequently subjecting the thus obtained alcohol to halogenation which accompanies inversion of the configuration.

**37.** The method for producing the optically active methoxyiminoacetamide (I) according to claim 34, wherein the optically active 1-arylethyl halide derivative (VIII) is obtained by allowing a diazonium salt derived from an aniline derivative represented by the general formula (XIV) to react with acetaldoxime, thereby obtaining an acetophenone derivative (XIII) which is subsequently subjected to hydrogen transfer type asymmetric reduction to obtain an optically active 1-arylethylalcohol (XI), and then subjecting the thus obtained alcohol to halogenation which accompanies inversion of the configuration.

**38.** An agricultural chemical preparation which comprises the optically active methoxyiminoacetamide derivative of claim 1 as its active ingredient.

**39.** A method for controlling fungi, which comprises applying the optically active methoxyiminoacetamide derivative of claim 1 as an active ingredient to plants or seeds.

FIG. 1

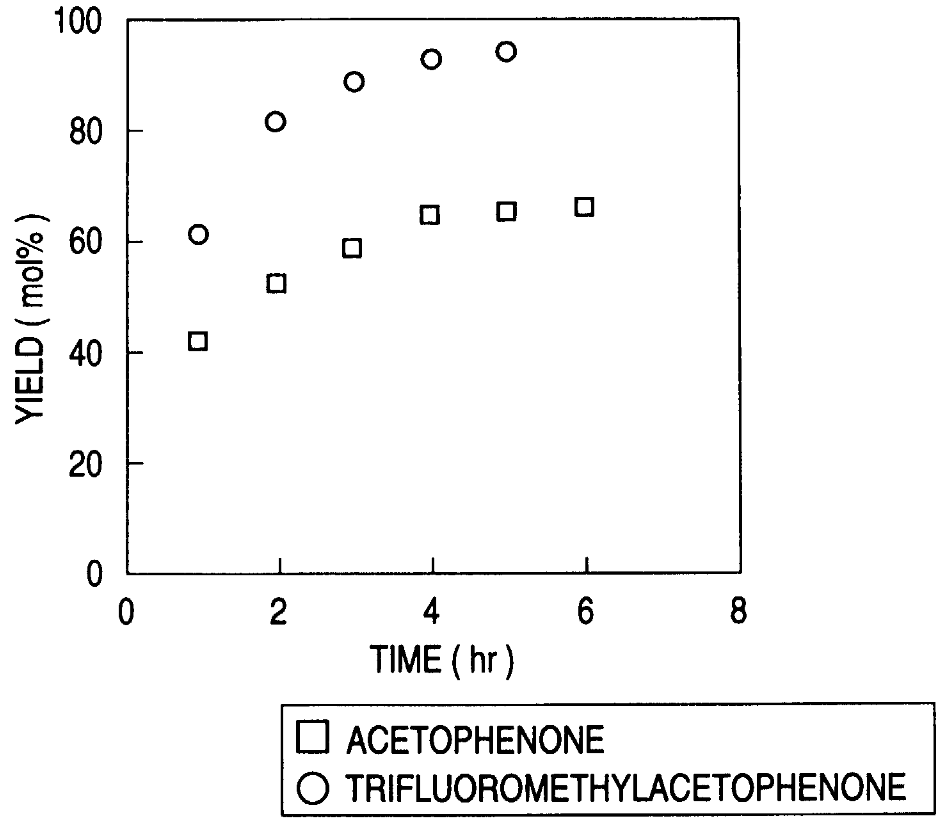
100
80
60
40
20
0
YIELD ( mol% )
0
2
4
6
8
TIME ( hr )
ACETOPHENONE
TRIFLUOROMETHYLACETOPHENONE

FIG. 2

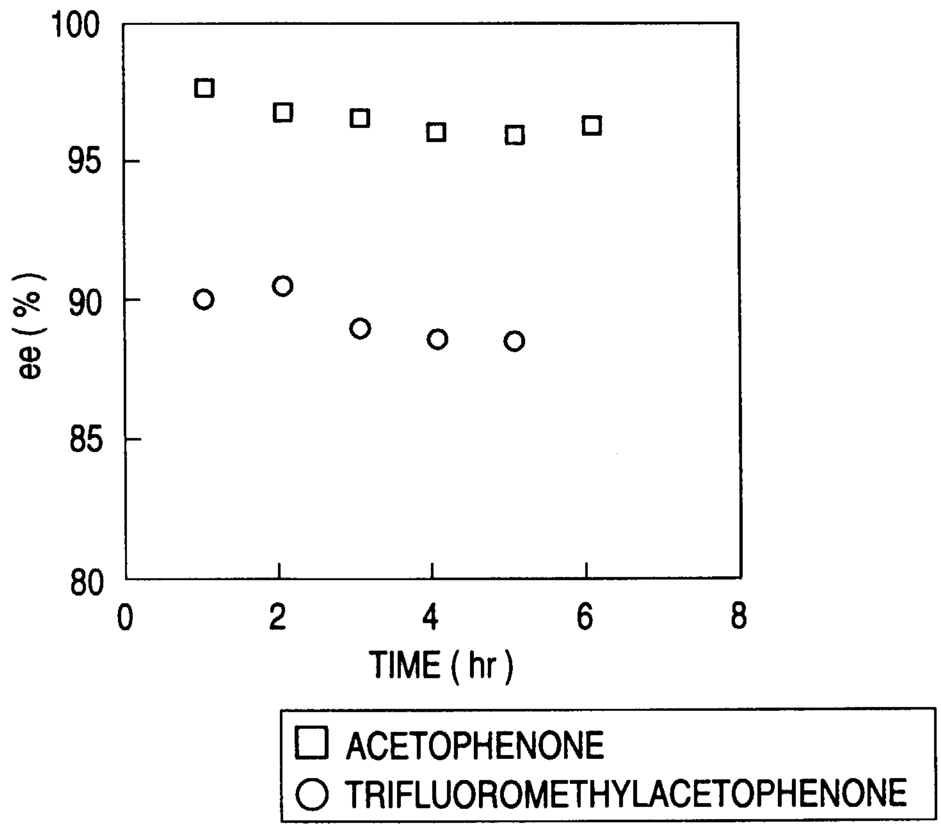
100
95
90
85
80
ee ( % )
0
2
4
6
8
TIME ( hr )
ACETOPHENONE
TRIFLUOROMETHYLACETOPHENONE

FIG. 3

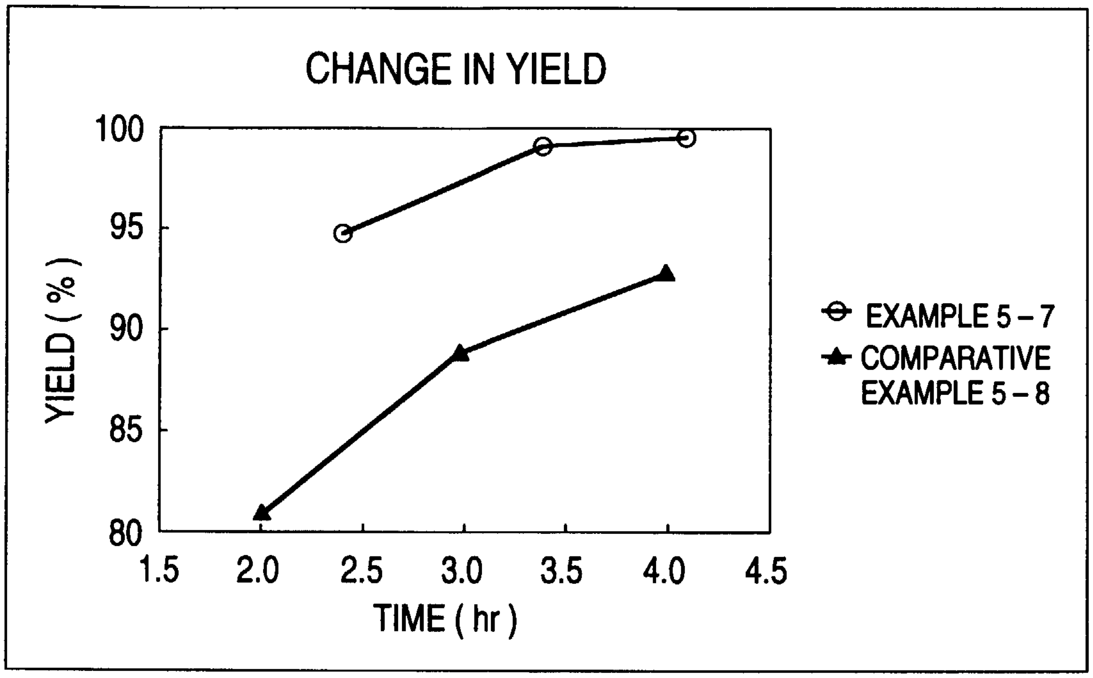
CHANGE IN YIELD
YIELD ( % )
100
95
90
85
80
1.5
2.0
2.5
3.0
3.5
4.0
4.5
TIME ( hr )
EXAMPLE 5 – 7
COMPARATIVE EXAMPLE 5 – 8

FIG. 4

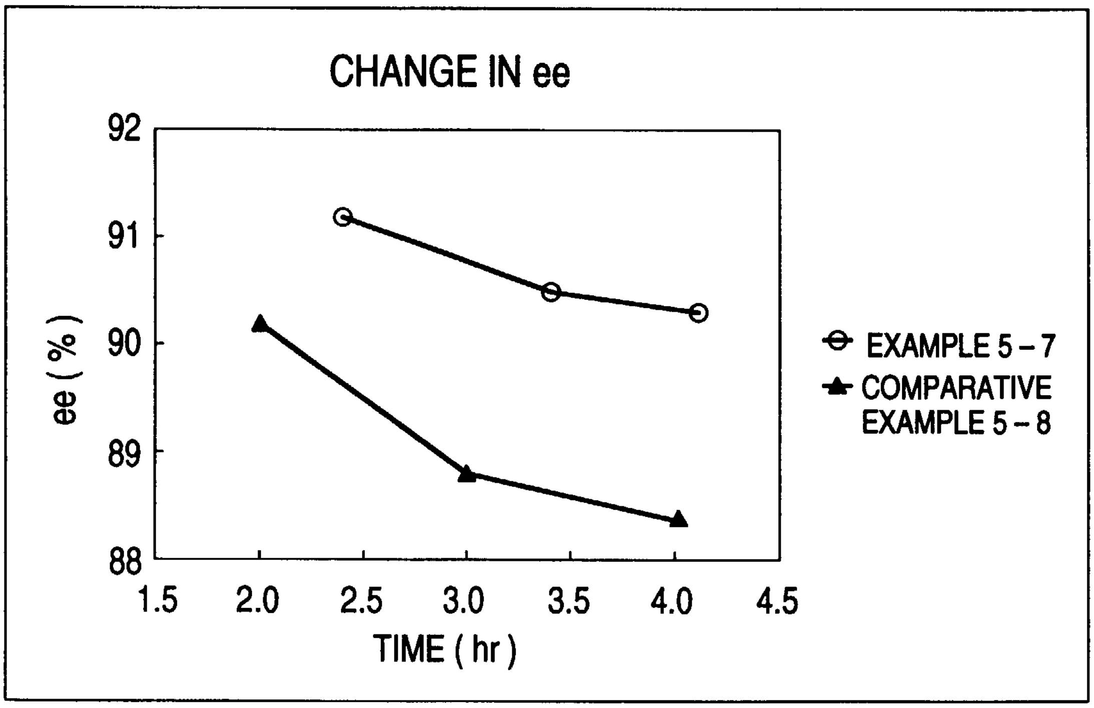
CHANGE IN ee
ee ( % )
92
91
90
89
88
1.5
2.0
2.5
3.0
3.5
4.0
4.5
TIME ( hr )
EXAMPLE 5 – 7
COMPARATIVE EXAMPLE 5 – 8

## INTERNATIONAL SEARCH REPORT

International application No. PCT/JP97/04291

A. CLASSIFICATION OF SUBJECT MATTER
Int.Cl$^6$ C07C251/50, 249/04, 249/08, 239/20, 259/04, 271/06, 309/63, 33/22, 29/136, 45/42, 49/78, C07B53/00, 57/00, C07D333/28,
According to International Patent Classification (IPC) or to both national classification and IPC

B. FIELDS SEARCHED
Minimum documentation searched (classification system followed by classification symbols)
Int.Cl$^6$ C07C251/50, 249/04, 249/08, 239/20, 259/04, 271/06, 309/63, 33/22, 29/136, 45/42, 49/78, C07B53/00, 57/00, C07D333/28,

Documentation searched other than minimum documentation to the extent that such documents are included in the fields searched

Electronic data base consulted during the international search (name of data base and, where practicable, search terms used)
CA (STN), REGISTRY (STN)

C. DOCUMENTS CONSIDERED TO BE RELEVANT

| Category* | Citation of document, with indication, where appropriate, of the relevant passages | Relevant to claim No. |
|---|---|---|
| PX,PY<br>PA | WO, 96/38408, A1 (Mitsubishi Chemical Corp.), December 5, 1996 (05. 12. 96), Refer to Claims ; Reaction Formulae 1, 2 and 4 ; Table-1 (Family: none) | 1-5, 11-21, 34-39<br>6-10, 30-31 |
| PX,PY | WO, 96/37463, A1 (Mitsubishi Chemical Corp.), November 28, 1996 (28. 11. 96), Refer to Claims ; Table-1 & JP, 8-319265, A | 1-5, 11-12, 38-39 |
| X,Y | JP, 8-283223, A (Mitsubishi Chemical Corp.), October 29, 1996 (29. 10. 96), Refer to Claims ; Scheme ; Table-1 (Family: none) | 1-5, 11-13, 18-21, 25-29, 38-39 |
| X,Y<br>A | JP, 7-76564, A (Mitsubishi Chemical Corp.), March 20, 1995 (20. 03. 95), Refer to Claims ; Reaction Formula ; Table-1 & EP, 596254, B1 & US, 5407902, A & DE, 69311257, A1 | 1-3, 10, 38-39<br>9 |

[X] Further documents are listed in the continuation of Box C. [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>February 17, 1998 (17. 02. 98) | Date of mailing of the international search report<br>March 3, 1998 (03. 03. 98) |
| Name and mailing address of the ISA/<br>Japanese Patent Office<br>Facsimile No. | Authorized officer<br>Telephone No. |

Form PCT/ISA/210 (second sheet) (July 1992)

INTERNATIONAL SEARCH REPORT

International application No.
PCT/JP97/04291

C (Continuation). DOCUMENTS CONSIDERED TO BE RELEVANT

| Category* | Citation of document, with indication, where appropriate, of the relevant passages | Relevant to claim No. |
|---|---|---|
| X,Y<br>A | JP, 6-25132, A (BASF AG.),<br>February 1, 1994 (01. 02. 94),<br>Refer to Claims ; Reaction Formula 1<br>& EP, 499823, B1 & DE, 59206443, B1<br>& US, 5254717, A | 10<br>9 |
| Y | JP, 4-178356, A (Hokuriku Seiyaku Co., Ltd.),<br>June 25, 1992 (25. 06. 92),<br>Refer to Examples<br>& EP, 420120, B1 & DE, 69012031, A1<br>& KR, 9500778, B1 | 13 |
| A | JP, 61-286349, A (Yashima Chemical Industrial Co., Ltd.),<br>December 16, 1986 (16. 12. 86),<br>Refer to Claims ; Examples (Family: none) | 14-17 |
| X | Fritz-Langhals, Elke, "Tetrahedron Lett."<br>35(12)(1994), p.1851-1854,<br>Refer to Scheme 1, Table 1 | 22-24 |
| X | JP, 2-72149, A (Japan Energy Corp.),<br>March 12, 1990 (12. 03. 90),<br>Refer to Claims<br>& EP, 337820, B1 & US, 5041641, A<br>& DE, 68907771, A1 | 23-24 |
| PY | JP, 9-157196, A (Japan Science and Technology Corp.),<br>June 17, 1997 (17. 06. 97),<br>Refer to Claims ; Examples<br>& JP, 9-157228, A | 25-29 |
| A | JP, 1-117839, A (Seitetsu Kagaku Kogyo Co., Ltd.),<br>May 10, 1989 (10. 05. 89),<br>Refer to Claims (Family: none) | 32-33 |

Form PCT/ISA/210 (continuation of second sheet) (July 1992)

INTERNATIONAL SEARCH REPORT

International application No.
PCT/JP97/04291

A. (Continuation) CLASSIFICATION OF SUBJECT MATTER

317/18, 319/06, 317/54, 319/18, A01N35/10

B. (Continuation) FIELDS SEARCHED

317/18, 319/06, 317/54, 319/18, A01N35/10

Form PCT/ISA/210 (extra sheet) (July 1992)

INTERNATIONAL SEARCH REPORT

International application No.
PCT/JP97/04291

**Box I Observations where certain claims were found unsearchable (Continuation of item 1 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box II Observations where unity of invention is lacking (Continuation of item 2 of first sheet)**

This International Searching Authority found multiple inventions in this international application, as follows:

Claims 1 to 39 are divided into the following nine groups of inventions.

I. A group of inventions of claims 1 to 5, 9, and 34 to 39 relates to methoxyiminoacetamide derivatives represented by the general formula (I), processes for the preparation of them, and uses thereof, and a group of inventions of claims 11 to 13 relates to 1-arylethoxyamine derivatives represented by the general formula (II) and processes for the preparation thereof, while an invention of claim 10 relates to optically active methoxyiminoacetamide derivatives represented by the general formula (VII). The 1-

1. ☒ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** ☐ The additional search fees were accompanied by the applicant's protest.
☒ No protest accompanied the payment of additional search fees.

Form PCT/ISA/210 (continuation of first sheet (1)) (July 1992)

INTERNATIONAL SEARCH REPORT

International application No.
PCT/JP97/04291

Continuation of Box No. II of continuation of first sheet (1)

arylethoxyamine derivatives represented by the general formula (II) and the optically active methoxyiminoacetamide derivatives represented by the general formula (VII) each have one and the same main structural element as that of the methoxyiminoacetamide derivatives represented by the general formula (I), and technical relevancy is present between the chemical structures of both substances. Accordingly, the group of inventions of claims 1 to 5, that of claims 9 to 13, and that of claims 34 to 39 are considered as relating to a group of inventions so linked as to form a single general inventive concept.

II. A group of inventions of claims 6 to 8 relates to processes for the preparation of acetal derivatives represented by the general formula (III) and not containing one and the same main structural element as that of compounds of other independent claims. Therefore, the group of inventions of claims 6 to 8 does not fall under the category of processes for the preparation of the compounds.

III. An invention of claim 14 relates to a process for the preparation of 1-arylethoxyamine derivatives represented by the general formula (II') and not containing one and the same main structural element as that of compounds of other independent claims. Therefore, the invention of claim 14 do not fall under the category of processes for the preparation of the compounds.

IV. A group of inventions of claims 15 to 17 relates to hydroxamic acid derivatives represented by the general formula (X). Since the 1-arylethoxyamine derivatives represented by the general formula (II') are known substances, however, other intermediates lacking in novelty are present in the steps leading to the final product. Such being the case, the invention of claim 1 and the group of inventions of claims 15 to 17 are not considered as relating to a group of inventions so linked as to form a single general inventive concept.

V. A group of inventions of claims 18 to 22 relates to processes for the preparation of 1-arylethoxyamine derivatives represented by the general formula (II') and not containing one and the same main structural element as that of compounds of other independent claims. Therefore, the group of inventions of claims 18 to 22 does not fall under the category of processes for the preparation of the compounds.

VI. A group of inventions of claims 23 to 24 relates to optically active sulfonic acid ester derivatives represented by the general formula (XII). Since the 1-arylethoxyamine derivatives represented by the general formula (II') are known substances, however, other intermediates lacking in novelty are present in the steps leading to the final product. Such being the case, the invention of claim 1 and the group of inventions of claims 23 to 24 are not considered as relating to a group of inventions so linked as to form a single general inventive concept.

VII. A group of inventions of claims 25 to 29 relates to processes

Form PCT/ISA/210 (extra sheet) (July 1992)

INTERNATIONAL SEARCH REPORT

International application No.
PCT/JP97/04291

for the preparation of 1-arylethyl alcohol derivatives represented by the general formula (XI) and not containing one and the same main structural element as that of compounds of other independent claims. Therefore, the group of inventions of claims 25 to 29 does not fall under the category of processes for the preparation of the compounds.

VIII. A group of inventions of claims 30 to 31 relates to processes for the preparation of hydroxime derivatives represented by the general formula (XV) and not containing one and the same main structural element as that of compounds of other independent claims. Therefore, the group of ivnentions of claims 30 to 31 does not fall under the category of processes for the preparation of the compounds.

IX. A group of inventions of claims 32 to 33 relates to processes for the preparation of acetophenone derivatives represented by the general formula (XIII) and not containing one and the same main structural element as that of compounds of other independent claims. Therefore, the group of inventions of claims 32 to 33 does not fall under the category of processes for the preparation of the compounds.

Form PCT/ISA/210 (extra sheet) (July 1992)